# EUROPEAN PATENT APPLICATION

(11) **EP 4 549 441 A1**
(43) Date of publication of application: **07.05.2025**
(21) Application number: 23831885.1
(22) Date of filing: 28.06.2023
(51) Int. Cl.: C07D 491/107, A61K 31/529, A61K 31/5377, A61P 25/28, A61P 29/00, A61P 43/00

(54) **NOVEL COMPOUND AS NADPH OXIDASE 2 INHIBITOR AND PHARMACEUTICAL COMPOSITION CONTAINING SAME**

(30) Priority: 28.06.2022 KR 20220079153
(71) Applicant: Dongguk University Industry-Academic Cooperation Foundation, Seoul 04620 (KR)
(72) Inventor: LEE, Moo-Yeol, Seoul 06213 (KR); LEE, Kyeong, Goyang-si Gyeonggi-do 10371 (KR)
(74) Representative: Mewburn Ellis LLP
(86) International application number: PCT/KR2023/008993
(87) International publication number: WO 2024/005526

(57) **Abstract**

The present invention relates to a novel compound as an NADPH oxidase 2 inhibitor and a pharmaceutical composition containing same. More specifically, the compound according to the present invention exhibits selective and excellent inhibitory activity against NADPH oxidase 2, which is known to date, and thus can be advantageously used as a therapeutic for various diseases associated with NADPH oxidase 2, such as inflammatory diseases, fibrotic diseases, and degenerative neural diseases.

## Description

### Technical Field

The present disclosure relates to a novel compound as an NADPH oxidase 2 inhibitor and a pharmaceutical composition including the same.

### Background Art

NADPH oxidase (NOX) is a protein that transfers electrons across biological membranes. In general, an electron acceptor is oxygen, and the product of an electron transfer reaction is superoxide. Thus, the biological function of NOX enzymes is to produce reactive oxygen species (ROS) from oxygen. Reactive oxygen species (ROS) are oxygen-derived small molecules that include oxygen radicals and/or specific non-radicals that are oxidizers and/or readily converted to radicals. Nitrogen-containing oxidizers, such as nitric oxide, are also called reactive nitrogen species (RNS). ROS generation is a series of reactions that usually begin with the production of superoxide. Superoxide is quickly dismutated to hydrogen peroxide spontaneously, especially at low pH or catalyzed by superoxide dismutase. Other factors in the series of ROS generation include the reaction of superoxide and nitric oxide resulting in peroxynitrite, the formation of hypochlorous acid from hydrogen peroxide facilitated by peroxidase, and the Fenton reaction by iron catalysts leading to the production of hydroxyl radicals.

Of NADPH oxidases, NADPH oxidase 2 (NOX2) is known to mediate the occurrence and progression of various diseases, such that attempts are being made to develop NOX2 inhibitors as new drugs. However, NOX2 inhibitors have not yet been developed as new drugs, and several types of inhibitors are in clinical trials. Therefore, NOX2 is attracting attention as a target for innovative first-in-class drugs.

Specifically, NOX2 inhibitors can be developed as a therapeutic agent for inflammatory diseases, and especially it is applicable for diseases accompanied with fibrosis such as non-alcoholic fatty liver diseases, chronic kidney diseases, and idiopathic pulmonary fibrosis. In addition, it is anticipated to have therapeutic effects even on neuroinflammatory diseases such as microglia-mediated neurotoxicity, Alzheimer's disease, and Parkinson's disease.

### Disclosure of the Invention

### Technical Goals

An object of the present disclosure is to provide a novel compound as an NADPH oxidase 2 (NOX2) inhibitor.

Another object of the present disclosure is to provide a pharmaceutical composition for treating or preventing an NADPH oxidase 2 (NOX2)-related disease, including the novel compound.

Still another object of the present disclosure is to provide a method of treating an NADPH oxidase 2 (NOX2)-related disease, including administering the novel compound to a subject.

### Technical Solutions

The present disclosure provides a compound selected from a compound represented by the following Chemical Formula 1, an isomer thereof, or a pharmaceutically acceptable salt thereof: 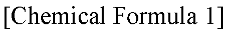 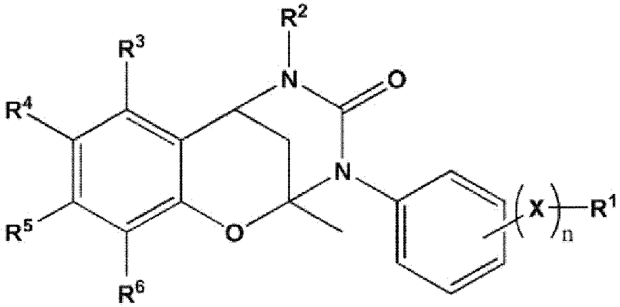
[11] wherein X is selected from carbonyl or methylene, n is an integer of 0 or 1,
[12] R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
[13] R² is hydrogen or (C1~C4)alkyl, and
[14] R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound,
[15] provided that the case where, in the compound, X is carbonyl, n is 1, R¹ is tetrahydroisoquinoline, and R² to R⁶ are all hydrogen is excluded.

In addition, the present disclosure provides a pharmaceutical composition for treating or preventing an NADPH oxidase 2 (NOX2)-related disease, including the compound represented by Chemical Formula 1.

In addition, the present disclosure provides a pharmaceutical composition for treating or preventing an inflammatory disease or a fibrotic disease, including a compound represented by the following Chemical Formula 1: 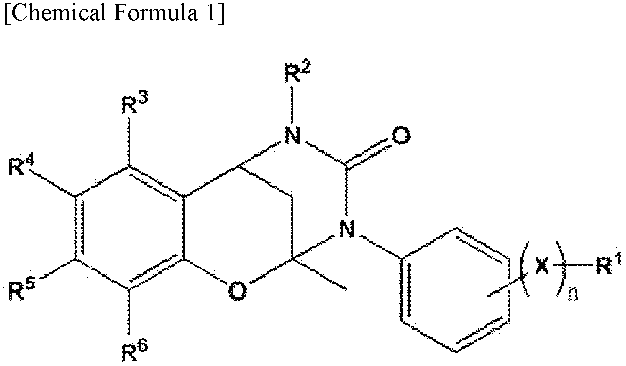
wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound.

### Advantageous Effects

The present disclosure relates to a novel compound as an NADPH oxidase 2 inhibitor and a pharmaceutical composition including the same, wherein, by providing the NADPH oxidase 2 inhibitor, it is possible to develop therapeutic agents for various diseases such as NADPH oxidase 2 (NOX2)-related diseases, e.g., inflammatory diseases, fibrotic diseases, and neurodegenerative diseases.

### Brief Description of Drawings

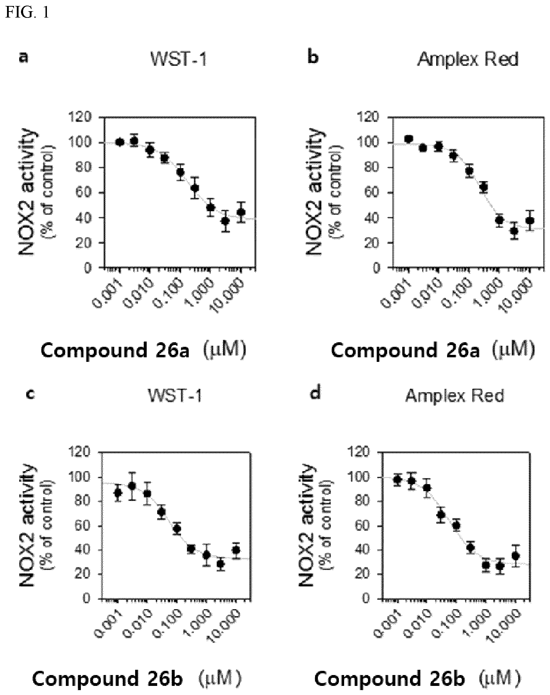

FIG. 1 shows graphs showing experimental results for NADPH oxidase 2 inhibitory ability according to the concentrations of a novel compound according to the present disclosure.

### Best Mode for Carrying Out the Invention

Hereinafter, the present disclosure will be described in more detail.

The present inventors completed the present disclosure by developing a leading candidate substance that is applicable to the human body by studying a structure-activity relationship of an effective substance, 2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one that is derived from previous research.

Hereby, the present disclosure provides a compound selected from a compound represented by the following Chemical Formula 1, an isomer thereof, or a pharmaceutically acceptable salt thereof:

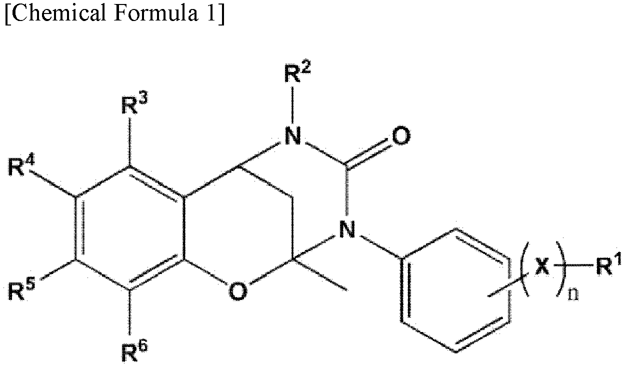

wherein X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound, provided that the case where, in the compound, X is carbonyl, n is 1, R¹ is tetrahydroisoquinoline, and R² to R⁶ are all hydrogen may be excluded.

Preferably, the 5-ring or 6-ring heterocyclic compound may be selected from the group consisting of thiophenyl, furanyl, morpholino, pyridinyl, pyranyl, oxazinyl, thiazinyl, pyrimidinyl, and piperazinyl.

More preferably, the compound may be a compound represented by the following Chemical Formula 1-1:

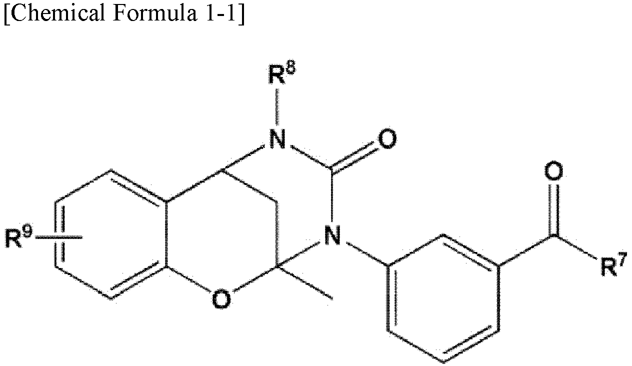

wherein, in the Chemical Formula 1-1,
R⁷ is tetrahydroisoquinoline that is substituted or unsubstituted with one or more substituents selected from halo, di(C1~C4)alkyl, difluoromethyl, or trifluoromethyl,
R⁸ is hydrogen or (C1~C2)alkyl, and
R⁹ may be the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

In one embodiment, the compound may be selected from the group consisting of 3-(2-methyl-4-oxo-5,6-dihydro-2H-2,6-methanobenzo[g][1,3,5]oxadiazocin-3(4H)-yl)benzoic acid (Compound 7); 3-(3-(6,7-dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 8b); 3-(2-methyl-4-oxo-5,6-dihydro-2H-2,6-methanobenzo[g][1,3,5]oxadiazocin-3(4H)-yl)-N-(4-(trifluoromethyl)benzyl)benzamide (Compound 8c); 3-(2-methyl-4-oxo-5,6-dihydro-2H-2,6-methanobenzo[g][1,3,5]oxadiazocin-3(4H)-yl)-N-(4-(trifluoromethyl)phenethyl)benzamide (Compound 8d); N-(2,3-dihydro-1H-inden-2-yl)-3-(2-methyl-4-oxo-5,6-dihydro-2H-2,6-methanobenzo[g][1,3,5]oxadiazocin-3(4H)-yl)benzamide (Compound 8e); 3-(2-methyl-4-oxo-5,6-dihydro-2H-2,6-methanobenzo[g][1,3,5]oxadiazocin-3(4H)-yl)-N-(pyridin-3-ylmethyl)benzamide (Compound 8f); N-(furan-2-ylmethyl)-3-(2-methyl-4-oxo-5,6-dihydro-2H-2,6-methanobenzo[g][1,3,5]oxadiazocin-3(4H)-yl)benzamide (Compound 8g); N-cyclopropyl-3-(2-methyl-4-oxo-5,6-dihydro-2H-2,6-methanobenzo[g][1,3,5]oxadiazocin-3(4H)-yl)benzamide (Compound 8h); N-benzyl-N-methyl-3-(2-methyl-4-oxo-5,6-dihydro-2H-2,6-methanobenzo[g][1,3,5]oxadiazocin-3(4H)-yl)benzamide (Compound 8i); 2,5-dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 9); N-(4-bromobenzyl)-3-((2R,6R)-2-methyl-4-oxo-5,6-dihydro-2H-2,6-methanobenzo[g][1,3,5]oxadiazocin-3(4H)-yl)benzamide (Compound 10b); N-(3,5-dibromobenzyl)-3-((2S,6S)-2-methyl-4-oxo-5,6-dihydro-2H-2,6-methanobenzo[g][1,3,5]oxadiazocin-3(4H)-yl)benzamide (Compound 10d); 3'-(2-methyl-4-oxo-5,6-dihydro-2H-2,6-methanobenzo[g][1,3,5]oxadiazocin-3(4H)-yl)-[1,1'-biphenyl]-3-carbonitrile (Compound 15a); 3-(3-(furan-2-yl)phenyl)-2-methyl-5,6-dihydro-2H-2,6-methanobenzo[g][1,3,5]oxadiazocin-4(3H)-one (Compound 15b); 3-(3-(furan-2-yl)phenyl)-2,5-dimethyl-5,6-dihydro-2H-2,6-methanobenzo[g][1,3,5]oxadiazocin-4(3H)-one (Compound 16); 2-methyl-3-(3-(((3-(trifluoromethyl)phenyl)amino)methyl)phenyl)-5,6-dihydro-2H-2,6-methanobenzo[g][1,3,5]oxadiazocin-4(3H)-one (Compound 19); 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a); 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25c); 8-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25d); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f); 10-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25g); 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h); 2-methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one (Compound 25i); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 9-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25k); 9-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25l); 7-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25m); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 2-methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26f); 2-methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one (Compound 26g); 10-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26h); 2-methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one (Compound 26i); 9-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26j); 8-bromo-3-(3-(7-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27a); 8-bromo-3-(3-(6-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27b); 8-bromo-3-(3-(8-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27c); 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27d); 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27e); 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27f); 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27g); 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27h); 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27i); 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27j); 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 27k); 3-(3-(7-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28a); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d); 8-(furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28e); 8-(furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28f); 8-(furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28g); 8-(furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28h); 3-(3-(4,4-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28i); 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28j); and 8-(furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 28k).

More specifically, the compound may be selected from the group consisting of 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); and 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d).

The compound according to the present disclosure may selectively inhibit NADPH oxidase 2 (NOX2).

In addition, among the compounds, S,S isomers may exhibit superior NADPH oxidase 2 (NOX2) inhibitory activity than pure R,R isomers.

The 'pharmaceutically acceptable salt' may be used in any one form of pharmaceutically acceptable basic salts or acidic salts. Basic salts may be used in any one form of organic or inorganic salts, such as sodium salts, potassium salts, calcium salts, lithium salts, magnesium salts, cesium salts, aminium salts, ammonium salts, triethylamine salts, and pyridinium salts, but are not limited thereto.

Also, for acidic salts, acid addition salts that are formed by free acids are useful. Inorganic acids and organic acids may be used as free acids, hydrochloric acid, bromic acid, sulfuric acid, sulfurous acid, and phosphoric acid may be used as the inorganic acid, and citric acid, acetic acid, maleic acid, fumaric acid, glucosan, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, and aspartic acid may be used as the organic acid. Preferably, hydrochloric acid may be used as the inorganic acid, and methanesulfonic acid as the organic acid.

In addition, the compound according to the present disclosure may include not only pharmaceutically acceptable salts, but also all salts, hydrates, and solvents that may be prepared by conventional methods.

The addition salt according to the present disclosure may be prepared by conventional methods, for example, by dissolving the compound in a water-miscible organic solvent, e.g., acetone, methanol, ethanol, or acetonitrile, and then precipitating or crystallizing after adding an excess of organic bases or an aqueous basic solution of inorganic bases. Alternatively, preparation may be carried out by obtaining the addition salt by evaporation of the solvent or excess base from the mixture followed by drying or suction filtration of the precipitated salts.

In addition, the present disclosure provides a pharmaceutical composition for treating or preventing an NADPH oxidase 2 (NOX2)-related disease, including a compound selected from a compound represented by the following Chemical Formula 1, an isomer thereof, or a pharmaceutically acceptable salt thereof:

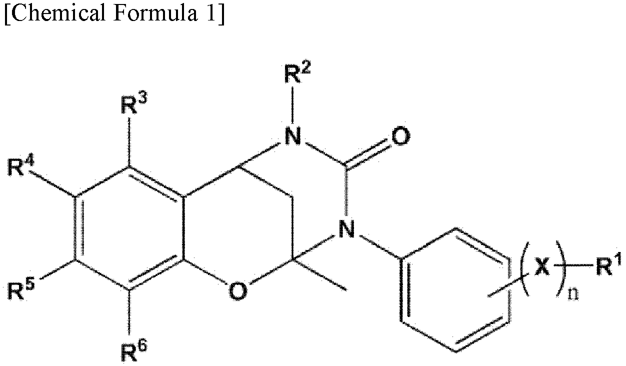

wherein X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound, but are not limited thereto.

Preferably, the 5-ring or 6-ring heterocyclic compound may be selected from the group consisting of thiophenyl, furanyl, morpholino, pyridinyl, pyranyl, oxazinyl, thiazinyl, pyrimidinyl, and piperazinyl, provided that the case where, in the compound, X is carbonyl, n is 1, R¹ is tetrahydroisoquinoline, and R² to R⁶ are all hydrogen may be excluded.

More preferably, the compound may be a compound represented by the following Chemical Formula 1-1:

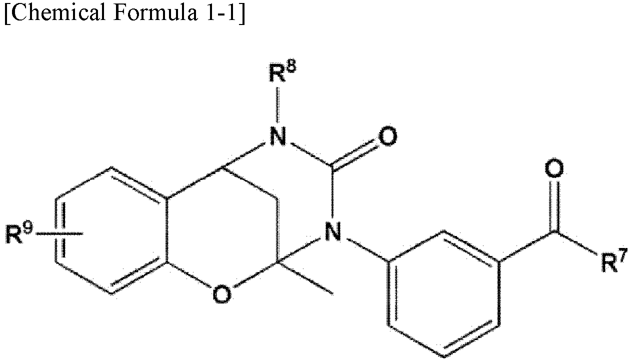

wherein, in the Chemical Formula 1-1,
R⁷ is tetrahydroisoquinolines that is substituted or unsubstituted with one or more substituents selected from halo, di(C1~C4)alkyl, difluoromethyl, or trifluoromethyl,
R⁸ is hydrogen or (C1~C2)alkyl, and
R⁹ may be the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

In one embodiment, the compound may be selected from the group consisting of 3-(2-methyl-4-oxo-5,6-dihydro-2H-2,6-methanobenzo[g][1,3,5]oxadiazocin-3(4H)-yl)benzoic acid (Compound 7); 3-(3-(6,7-dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 8b); 3-(2-methyl-4-oxo-5,6-dihydro-2H-2,6-methanobenzo[g][1,3,5]oxadiazocin-3(4H)-yl)-N-(4-(trifluoromethyl)benzyl)benzamide (Compound 8c); 3-(2-methyl-4-oxo-5,6-dihydro-2H-2,6-methanobenzo[g][1,3,5]oxadiazocin-3(4H)-yl)-N-(4-(trifluoromethyl)phenethyl)benzamide (Compound 8d); N-(2,3-dihydro-1H-inden-2-yl)-3-(2-methyl-4-oxo-5,6-dihydro-2H-2,6-methanobenzo[g][1,3,5]oxadiazocin-3(4H)-yl)benzamide (Compound 8e); 3-(2-methyl-4-oxo-5,6-dihydro-2H-2,6-methanobenzo[g][1,3,5]oxadiazocin-3(4H)-yl)-N-(pyridin-3-ylmethyl)benzamide (Compound 8f); N-(furan-2-ylmethyl)-3-(2-methyl-4-oxo-5,6-dihydro-2H-2,6-methanobenzo[g][1,3,5]oxadiazocin-3(4H)-yl)benzamide (Compound 8g); N-cyclopropyl-3-(2-methyl-4-oxo-5,6-dihydro-2H-2,6-methanobenzo[g][1,3,5]oxadiazocin-3(4H)-yl)benzamide (Compound 8h); N-benzyl-N-methyl-3-(2-methyl-4-oxo-5,6-dihydro-2H-2,6-methanobenzo[g][1,3,5]oxadiazocin-3(4H)-yl)benzamide (Compound 8i); 2,5-dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 9); N-(4-bromobenzyl)-3-((2R,6R)-2-methyl-4-oxo-5,6-dihydro-2H-2,6-methanobenzo[g][1,3,5]oxadiazocin-3(4H)-yl)benzamide (Compound 10b); N-(3,5-dibromobenzyl)-3-((2S,6S)-2-methyl-4-oxo-5,6-dihydro-2H-2,6-methanobenzo[g][1,3,5]oxadiazocin-3(4H)-yl)benzamide (Compound 10d); 3'-(2-methyl-4-oxo-5,6-dihydro-2H-2,6-methanobenzo[g][1,3,5]oxadiazocin-3(4H)-yl)-[1,1'-biphenyl]-3-carbonitrile (Compound 15a); 3-(3-(furan-2-yl)phenyl)-2-methyl-5,6-dihydro-2H-2,6-methanobenzo[g][1,3,5]oxadiazocin-4(3H)-one (Compound 15b); 3-(3-(furan-2-yl)phenyl)-2,5-dimethyl-5,6-dihydro-2H-2,6-methanobenzo[g][1,3,5]oxadiazocin-4(3H)-one (Compound 16); 2-methyl-3-(3-(((3-(trifluoromethyl)phenyl)amino)methyl)phenyl)-5,6-dihydro-2H-2,6-methanobenzo[g][1,3,5]oxadiazocin-4(3H)-one (Compound 19); 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a); 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25c); 8-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25d); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f); 10-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25g); 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h); 2-methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one (Compound 25i); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 9-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25k); 9-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25l); 7-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25m); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 2-methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26f); 2-methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one (Compound 26g); 10-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26h); 2-methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one (Compound 26i); 9-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26j); 8-bromo-3-(3-(7-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27a); 8-bromo-3-(3-(6-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27b); 8-bromo-3-(3-(8-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27c); 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27d); 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27e); 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27f); 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27g); 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27h); 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27i); 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27j); 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 27k); 3-(3-(7-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28a); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d); 8-(furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28e); 8-(furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28f); 8-(furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28g); 8-(furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28h); 3-(3-(4,4-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28i); 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28j); and 8-(furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 28k).

More specifically, the compound may be selected from the group consisting of 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); and 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d).

The NADPH oxidase 2 (NOX2)-related disease may be selected from the group consisting of neurodegenerative diseases.

The neurodegenerative disease may be one selected from the group consisting of microglia-mediated neuroinflammatory disease, stroke, apoplexy, memory loss, memory impairment, dementia, forgetfulness, Parkinson's disease, Alzheimer's disease, Pick disease, Creutzfeldt-Jakob disease, Huntington's disease, and Lou Gehrig's disease, but is not limited thereto.

The pharmaceutical composition may be provided in one or more formulations selected from the group consisting of gels, emulsions, injections, acids, granules, aerosols, pastes, transdermal absorbents, and patches in accordance with conventional methods, but is not limited thereto.

In another embodiment of the present disclosure, the pharmaceutical composition may further include one or more additives selected from the group consisting of appropriate carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are commonly used in the manufacture of pharmaceutical compositions.

Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil may be used as the carriers, excipients, and diluents, solid preparations for oral administration may include tablets, pills, acids, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, and various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used.

The pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes.

The preferred dosage of the compound may vary depending on the condition and weight of the subject, the type and degree of disease, the form of the drug, and the route and duration of administration and be appropriately selected by those skilled in the art. According to one embodiment of the present disclosure, the daily dose may be, but not limited to, 0.01 mg/kg to 200 mg/kg, preferably 0.1 mg/kg to 200 mg/kg, and more preferably 0.1 mg/kg to 100 mg/kg. The administration may be conducted once a day or divided into several doses, but the scope of the present disclosure is not limited thereby.

In the present disclosure, the 'subject' may be a mammal including human, but is not limited to these examples.

In addition, the present disclosure provides a method of treating an NADPH oxidase 2 (NOX2)-related disease, including administering a compound selected from the compound represented by Chemical Formula 1, an isomer thereof, or a pharmaceutically acceptable salt thereof to a subject suffering from the NADPH oxidase 2 (NOX2)-related disease:

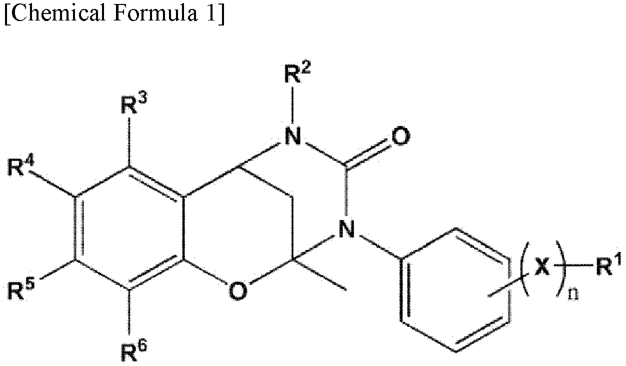

wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound, provided that the case where, in the compound, X is carbonyl, n is 1, R¹ is tetrahydroisoquinoline, and R² to R⁶ are all hydrogen may be excluded.

Corresponding features may be replaced with the above description.

In addition, provided is a pharmaceutical composition for treating or preventing an inflammatory disease or a fibrotic disease, including a compound selected from a compound represented by the following Chemical Formula 1, an isomer thereof, or a pharmaceutically acceptable salt thereof:

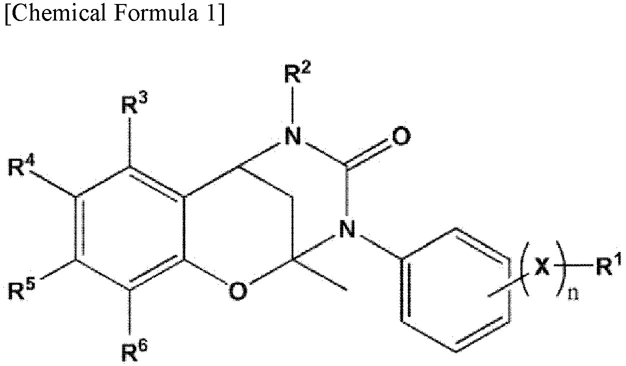

wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ may be the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound, but are not limited thereto.

Preferably, the 5-ring or 6-ring heterocyclic compound may be selected from the group consisting of thiophenyl, furanyl, morpholino, pyridinyl, pyranyl, oxazinyl, thiazinyl, pyrimidinyl, and piperazinyl.

More preferably, the compound may be a compound represented by the following Chemical Formula 1-1:

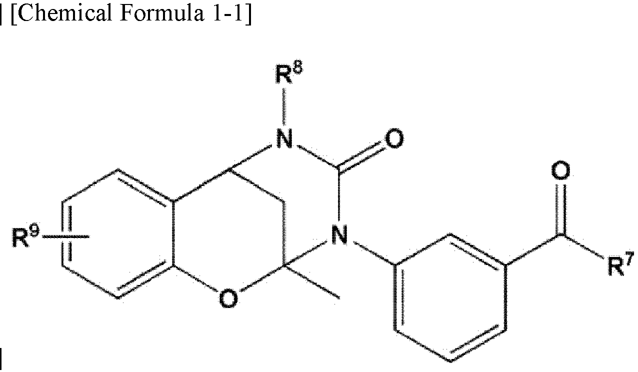

wherein, in the Chemical Formula 1-1,
R⁷ is tetrahydroisoquinoline that is substituted or unsubstituted with one or more substituents selected from halo, di(C1~C4)alkyl, difluoromethyl, or trifluoromethyl,
R⁸ is hydrogen or (C1~C2)alkyl, and
R⁹ may be the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

As an embodiment, the compound may be selected from the group consisting of 3-(2-methyl-4-oxo-5,6-dihydro-2H-2,6-methanobenzo[g][1,3,5]oxadiazocin-3(4H)-yl)benzoic acid (Compound 7); 2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 8a); 3-(3-(6,7-dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 8b); 3-(2-methyl-4-oxo-5,6-dihydro-2H-2,6-methanobenzo[g][1,3,5]oxadiazocin-3(4H)-yl)-N-(4-(trifluoromethyl)benzyl)benzamide (Compound 8c); 3-(2-methyl-4-oxo-5,6-dihydro-2H-2,6-methanobenzo[g][1,3,5]oxadiazocin-3(4H)-yl)-N-(4-(trifluoromethyl)phenethyl)benzamide (Compound 8d); N-(2,3-dihydro-1H-inden-2-yl)-3-(2-methyl-4-oxo-5,6-dihydro-2H-2,6-methanobenzo[g][1,3,5]oxadiazocin-3(4H)-yl)benzamide (Compound 8e); 3-(2-methyl-4-oxo-5,6-dihydro-2H-2,6-methanobenzo[g][1,3,5]oxadiazocin-3(4H)-yl)-N-(pyridin-3-ylmethyl)benzamide (Compound 8f); N-(furan-2-ylmethyl)-3-(2-methyl-4-oxo-5,6-dihydro-2H-2,6-methanobenzo[g][1,3,5]oxadiazocin-3(4H)-yl)benzamide (Compound 8g); N-cyclopropyl-3-(2-methyl-4-oxo-5,6-dihydro-2H-2,6-methanobenzo[g][1,3,5]oxadiazocin-3(4H)-yl)benzamide (Compound 8h); N-benzyl-N-methyl-3-(2-methyl-4-oxo-5,6-dihydro-2H-2,6-methanobenzo[g][1,3,5]oxadiazocin-3(4H)-yl)benzamide (Compound 8i); 2,5-dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 9); (2R,6R)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 10a); N-(4-bromobenzyl)-3-((2R,6R)-2-methyl-4-oxo-5,6-dihydro-2H-2,6-methanobenzo[g][1,3,5]oxadiazocin-3(4H)-yl)benzamide (Compound 10b); (2S,6S)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 10c); N-(3,5-dibromobenzyl)-3-((2S,6S)-2-methyl-4-oxo-5,6-dihydro-2H-2,6-methanobenzo[g][1,3,5]oxadiazocin-3(4H)-yl)benzamide (Compound 10d); 3'-(2-methyl-4-oxo-5,6-dihydro-2H-2,6-methanobenzo[g][1,3,5]oxadiazocin-3(4H)-yl)-[1,1'-biphenyl]-3-carbonitrile (Compound 15a); 3-(3-(furan-2-yl)phenyl)-2-methyl-5,6-dihydro-2H-2,6-methanobenzo[g][1,3,5]oxadiazocin-4(3H)-one (Compound 15b); 3-(3-(furan-2-yl)phenyl)-2,5-dimethyl-5,6-dihydro-2H-2,6-methanobenzo[g][1,3,5]oxadiazocin-4(3H)-one (Compound 16); 2-methyl-3-(3-(((3-(trifluoromethyl)phenyl)amino)methyl)phenyl)-5,6-dihydro-2H-2,6-methanobenzo[g][1,3,5]oxadiazocin-4(3H)-one (Compound 19); 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a); 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25c); 8-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25d); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f); 10-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25g); 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h); 2-methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one (Compound 25i); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 9-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25k); 9-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25l); 7-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25m); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 2-methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26f); 2-methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one (Compound 26g); 10-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26h); 2-methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one (Compound 26i); 9-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26j); 8-bromo-3-(3-(7-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27a); 8-bromo-3-(3-(6-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27b); 8-bromo-3-(3-(8-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27c); 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27d); 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27e); 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27f); 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27g); 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27h); 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27i); 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27j); 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 27k); 3-(3-(7-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28a); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d); 8-(furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28e); 8-(furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28f); 8-(furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28g); 8-(furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28h); 3-(3-(4,4-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28i); 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28j); and 8-(furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 28k).

More preferably, The compound may be selected from the group consisting of 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b); 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e); 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j); 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a); 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b); 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c); 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d); 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e); 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b); 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c); and 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d).

The inflammatory disease may be selected from the group consisting of, but is not limited to, osteoarthritis, rheumatoid arthritis, dermatitis, allergies, atopy, asthma, psoriasis, conjunctivitis, rhinitis, otitis media, pharyngolaryngitis, tonsillitis, pneumonia, gastric ulcer, gastritis, Crohn's disease, inflammatory bowel disease, lupus, hepatitis, cystitis, interstitial cystitis, nephritis, sjogren's syndrome, multiple sclerosis, Hashimoto thyroiditis, polymyositis, scleroderma, Addison disease, vitiligo, pernicious anemia, cystic fibrosis, graft-versus-host disease, transplant rejection disease, autoimmune diabetes, diabetic retinopathy, ischemia-reperfusion injury, post-angioplasty restenosis, chronic obstructive pulmonary disease (COPD), Graves' disease, and acute and chronic inflammatory diseases.

The fibrotic disease may be selected from the group consisting of liver fibrosis, pulmonary fibrosis, renal fibrosis, cardiac fibrosis, dermatofibrosis, and corneal fibrosis, but is not limited thereto.

The liver fibrosis may include, but is not limited to, non-alcoholic steatohepatitis (NASH) or non-alcoholic fatty liver disease (NAFLD).

In addition, the pulmonary fibrosis may be idiopathic pulmonary fibrosis, and the dermatofibrosis may be scleroderma, but are not limited thereto.

Corresponding features may be replaced with the above description.

### Modes for Carrying Out the Invention

Hereinafter, the present disclosure will be described in more detail through example embodiments. These example embodiments are merely intended to describe the present disclosure more specifically, and it will be apparent to those of ordinary skill in the art to which the present disclosure pertains that the scope of the present disclosure is not limited by the example embodiments according to the gist of the present disclosure.

### <Example> Synthesis of 2-methyl-5,6-dihydro-2H-2,6-methanobenzo[g][1,3,5]oxadiazocin-4(3H)-one derivatives

An NOX2 inhibitor was synthesized by a general process according to the following Scheme 1 or Scheme 2. The specific synthesis process for each compound will be described in detail below.

### A general process of converting amines to urea: A

KOCN (2.0 eq) was added at room temperature to a solution of AcOH: H₂O (1:1) containing appropriate amines (1.0 eq), and the mixture was stirred at ambient temperature for 4 hours. The reaction mixture was diluted with water until it became a solid precipitate, and the resulting precipitate was separated by filtration and dried under vacuum.

### A general process of acylation: B

At room temperature, acetic anhydride (1.2 eq) was added to a phenol (1.0 eq) solution in pyridine (1.2 eq). The resulting mixture was stirred at ambient temperature for 2 hours. The reaction mixture was quenched with 4N HCl (25 mL), and the aqueous phase was extracted with ethyl acetate (3 x 50 mL). The combined organic layer was dried with anhydrous sodium sulfate, filtered, and concentrated under reduced pressure. The residue was used in the next step without additional purification.

### A general process of the Biginelli reaction: C

Trimethylsilyl chloride (6.0 eq) was added dropwise to a stirred solution of aryl urea (1.0 eq), aldehyde (1.0 eq), and ethyl acetoacetate (1.5 eq) in DMF (25 mL). The resulting mixture was stirred at ambient temperature for 12 hours. The reaction mixture was quenched with ice-cold water (50 mL), and the aqueous phase was extracted with ethyl acetate (3 x 50 mL). The combined organic layer was dried with anhydrous sodium sulfate, filtered, and evaporated under reduced pressure. Residues were purified by silica gel column chromatography (15-45% EtOAc in hexane).

### A general one-pot process of deprotection, cyclization, and decarboxylation: D

Saturated NaHCO₃ solution (15 mL to 50 mL) was added to the stirred solution of dihydropyrimidinone (Biginelli product) (1.0 eq) in MeOH (20 mL). The resulting mixture was heated at 40°C for 12 hours. The reaction mixture was cooled to room temperature, diluted with water (25 mL), and the aqueous phase was extracted with ethyl acetate (3 x 20 mL). The combined organic layers were dried with anhydrous sodium sulfate, filtered, and evaporated under reduced pressure.

The residue was re-dissolved in THF:H₂O (8:2), and then LiOH (20 eq) was added, followed by heating at 40°C for 12 hours. The reaction mixture was cooled to room temperature and acidified by slowly adding 4M HCl to pH 1-2, and the resulting mixture was heated at 80°C for 8 hours. The reaction mixture was cooled to room temperature, and the resulting precipitate was isolated by filtration and dried under vacuum.

### A general process of acid-amine coupling: E

HATU (1.2 eq) was added to the stirred solution of benzoic acid (1.0 eq), amine (1.2 eq), and DIPEA (3.0 eq) in DMF (5 mL). The resulting mixture was stirred at ambient temperature for 2 hours. The reaction mixture was quenched with 25 mL of ice-cold water, and the aqueous phase was extracted with ethyl acetate (3 x 30 mL). The combined organic layer was dried with anhydrous sodium sulfate, filtered, and evaporated under reduced pressure. Residues were purified by silica gel column chromatography (5-10% MeOH in DCM).

### A general process of Suzuki coupling: F

### 1) Method A

Xphos-Pd G2 (0.01 eq) was added to a degassing solution in which bromo compound (1.0 eq), corresponding boronic acid (1.2 eq), and Cs₂CO₃ (3.0 eq) were dissolved in DMF:H₂O (9:1). The resulting mixture was heated in a sealed tube at 100°C for 2 hours. The reaction mixture was cooled to room temperature and diluted with water (20 mL), and the aqueous phase was extracted with ethyl acetate (3 x 30 mL). The combined organic layers were dried with anhydrous sodium sulfate, filtered, and evaporated under reduced pressure. The residue was purified by silica gel column chromatography (5-10% MeOH dissolved in DCM).

### 2) Method B

Pd(dppf)Cl₂.DCM (0.01 eq) was added to a degassing solution in which bromo compound (1.0 eq), corresponding boronic acid (1.2 eq), and Cs₂CO₃ (3.0 eq) were dissolved in 1,4 dioxane:H₂O (9:1). The resulting mixture was heated in a sealed tube at 100°C for 12 hours. The reaction mixture was cooled to room temperature and diluted with water (20 mL), and the aqueous phase was extracted with ethyl acetate (3 x 30 mL). The combined organic layers were dried with anhydrous sodium sulfate, filtered, and evaporated under reduced pressure. Residues were purified by silica gel column chromatography (25-30% EtOAc dissolved in hexane).

### Synthesis of 2-methyl-5,6-dihydro-2H-2,6-methanobenzo[g][1,3,5]oxadiazocin-4(3H)-one) derivatives

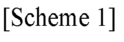

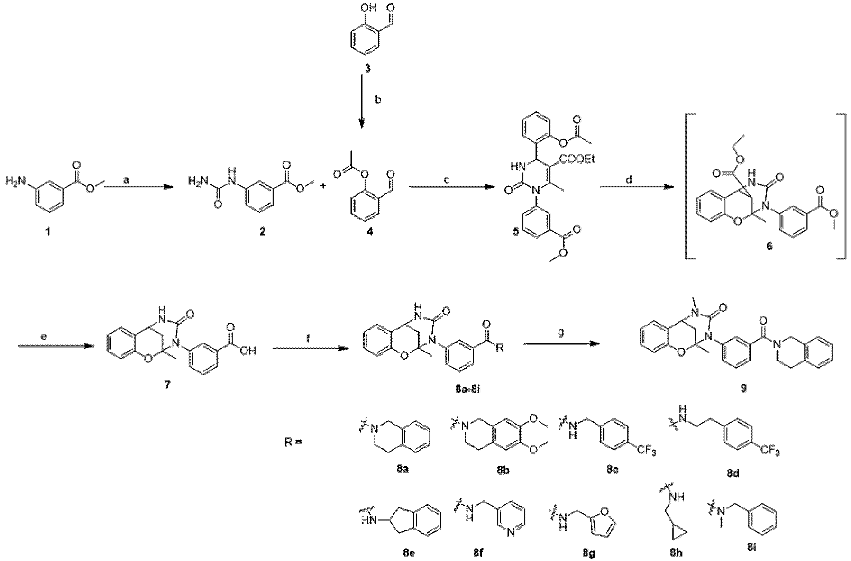

(a) KOCN, H₂O, AcOH, room temperature (rt), 12 hours;
(b) Acetic anhydride, pyridine, room temperature (rt), 12 hours;
(c) Ethyl acetoacetate, TMSCl, DMF, room temperature (rt), 12 hours;
(d) NaHCO₃, MeOH, H₂O, 40°C, 16 hours;
(e) LiOH, THF, H₂O, 40°C, 18 hours; 4 M HCl, pH 1-2, 80°C, 6 hours;
(f) Amines, HATU, DIPEA, DMF, at room temperature (rt), 2 hours;
(g) MeI, Cs₂CO₃, acetonitrile, room temperature (rt), 12 hours. 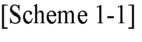 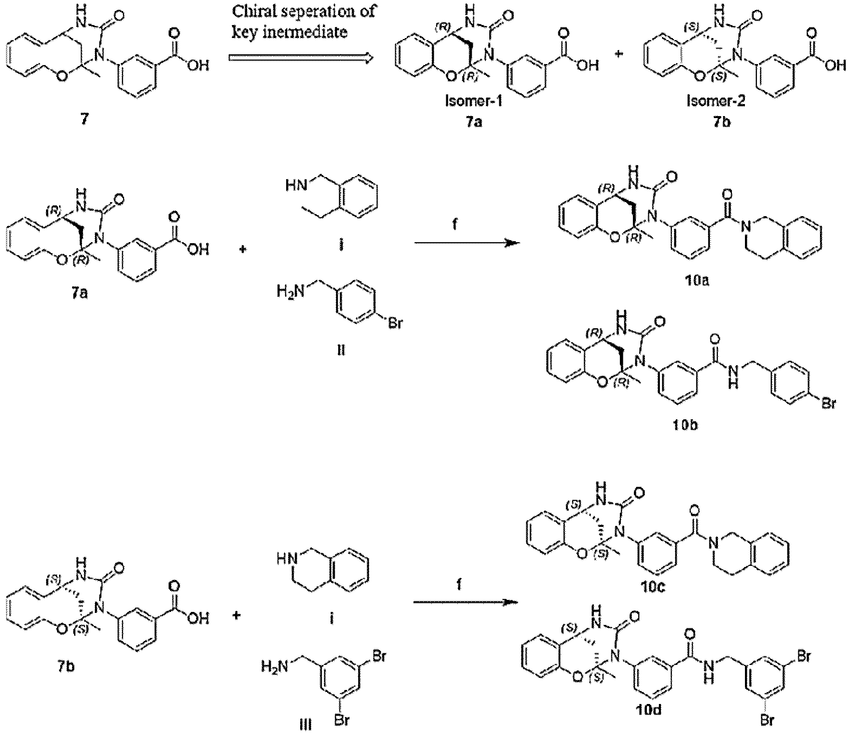
(f) Amines, HATU, DIPEA, DMF, at room temperature (rt), 2 hours.

The starting material methyl 3-aminobenzoate (1) and KOCN was reacted to form a urea structure. The resulting urea material (2) were reacted with ethyl acetoacetate, aldehyde, and trimethylsilyl chloride (TMSCl) to synthesize Compound 5 through the key reaction, the Biginelli reaction. Afterwards, NaHCO₃ was used for cyclization, and then Compound 7 was obtained through hydrolysis reaction. Through amide coupling in which the acid of Compound 7 is reacted with 11 types of amine and HATU, 11 types of compounds [8a-8l (racemic), 10a-10b (chirally pure R,R isomers), and 10c-10d (chirally pure S,S isomers) were derived. Finally, Compound 9 was synthesized via methylation using MeI to introduce a methyl group at the secondary amine site of Compound 8a (7a and 7b were separated using SFC purification).

### 1) Methyl 3-ureidobenzoate (Compound 2)

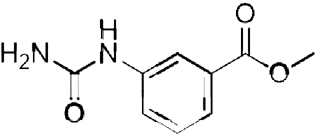

According to the general process of converting amines to urea (A), Compound 2 in white solid (4.70 g, 91.6%) was prepared from methyl 3-aminobenzoate (Compound 1) to be used in the next step without additional purification.

¹H NMR (400 MHz, DMSO-d₆) d 8.83 (s, 1H), 8.12 (s, 1H), 7.60 (d, J = 8.2 Hz, 1H), 7.49 (d, J = 7.7 Hz, 1H), 7.36 (t, J = 7.9 Hz, 1H), 5.95 (s, 2H), 3.84 (s, 3H).

### 2) 2-Formylphenyl acetate (Compound 4)

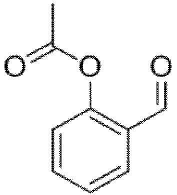

Compound 4 in colorless oil (1.30 g, 97%) was prepared from 2-hydroxybenzaldehyde (Compound 3) according to the general process of acylation (B).

¹H NMR (400 MHz, CDCl₃) δ 10.11 (s, 1H), 7.91 - 7.87 (m, 1H), 7.64 (ddd, J = 8.1, 7.5, 1.8 Hz, 1H), 7.44 - 7.38 (m, 1H), 7.19 (dd, J = 8.1, 1.0 Hz, 1H), 2.40 (s, 3H).

### 3) Ethyl 4-(2-acetoxyphenyl)-1-(3-(methoxycarbonyl)phenyl)-6-methyl-2-oxo-1,2,3,4-tetrahydropyrimidine-5-carboxylate (Compound 5)

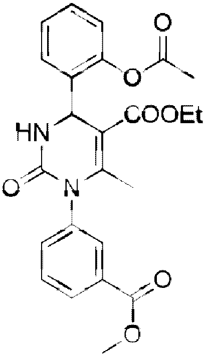

Compound 5 in white solid (1.60 g, 11.4%) was prepared from 2-formylphenyl acetate (Compound 4) according to the general Biginelli reaction process (C).

¹H NMR (400 MHz, CDCl₃) δ 8.10 - 8.06 (m, 1H), 7.90 (s, 1H), 7.54 (t, J = 7.8 Hz, 1H), 7.52 - 7.47 (m, 1H), 7.45 (s, 1H), 7.36 - 7.30 (m, 2H), 7.10 - 7.06 (m, 1H), 5.70 (s, 1H), 5.58 (d, J = 1.5 Hz, 1H), 4.07 - 3.99 (m, 2H), 3.94 (s, 3H), 2.39 (s, 3H), 2.18 (d, J = 0.7 Hz, 3H), 1.08 (t, J = 7.1 Hz, 3H).

### 4) 3-(2-Methyl-4-oxo-5,6-dihydro-2H-2,6-methanobenzo[g][1,3,5]oxadiazocin-3(4H)-yl)benzoic acid (Compound 7)

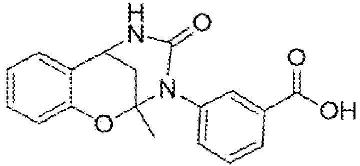

Compound 7 in pink solid (0.25 g, 76.0%) was prepared from ethyl 4-(2-acetoxyphenyl)-1-(3-(methoxycarbonyl)phenyl)-6-methyl-2-oxo-1,2,3,4-tetrahydropyrimidine-5-carboxylate (Compound 5) according to the general process (D) to be used in the next step without additional purification.

¹H NMR (400 MHz, DMSO-d₆) δ 13.04 (s, 1H), 7.88 (d, J = 7.9 Hz, 1H), 7.71 (d, J = 4.3 Hz, 1H), 7.66 (s, 1H), 7.50 (t, J = 7.8 Hz, 1H), 7.34 (d, J = 7.7 Hz, 1H), 7.27 (dd, J = 14.5, 7.4 Hz, 2H), 6.99 (t, J = 7.3 Hz, 1H), 6.93 (d, J = 8.0 Hz, 1H), 4.35 (s, 1H), 2.62 - 2.54 (m, 1H), 2.36 (d, J = 13.3 Hz, 1H), 1.37 (s, 3H).

### 5) 2-Methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 8a)

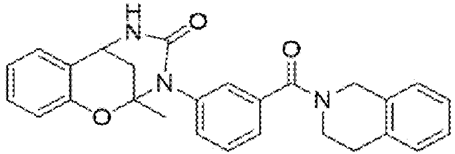

Compound 8a in off-white solid (0.04 g, 41.6%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

SOR: [α]²³_{D} -7.65° (c 0.1, MeOH); ¹H NMR (400 MHz, CD₃OD) δ 7.53 (t, J = 7.7 Hz, 1H), 7.46 (d, J = 8.2 Hz, 1H), 7.33 (d, J = 7.3 Hz, 1H), 7.30 - 7.07 (m, 6H), 7.01 - 6.79 (m, 3H), 4.63 (s, 1H), 4.43 (s, 1H), 3.96 (s, 1H), 3.67 (s, 1H), 2.91 (d, J = 50.6 Hz, 3H), 2.63 (d, J = 2.5 Hz, 1H), 2.43 (dd, J = 13.0, 2.9 Hz, 1H), 1.48 (s, 3H); Purity ≥98.1% (RP-HPLC, Rₜ = 18.2 min).

### 6) 3-(3-(6,7-Dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 8b)

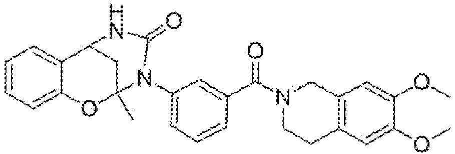

Compound 8b in off-white solid (0.04 g, 41.6%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 7.69 (d, J = 4.1 Hz, 1H), 7.48 (t, J = 7.7 Hz, 1H), 7.39 (d, J = 7.2 Hz, 1H), 7.28 - 7.06 (m, 4H), 7.01 - 6.81 (m, 3H), 6.76 (s, 1H), 4.67 (s, 1H), 4.35 (s, 1H), 4.03 (dd, J = 15.0, 7.1 Hz, 1H), 3.72 (s, 6H), 3.53 (s, 1H), 2.76 (s, 3H), 2.55 (d, J = 18.6 Hz, 1H), 2.35 (d, J = 14.4 Hz, 1H), 1.41 (s, 3H); Purity ≥99.6% (RP-HPLC, Rₜ = 16.4 min).

### 7) 3-(2-Methyl-4-oxo-5,6-dihydro-2H-2,6-methanobenzo[g][1,3,5]oxadiazocin-3(4H)-yl)-N-(4-(trifluoromethyl)benzyl)benzamide (Compound 8c)

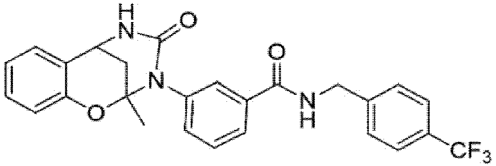

Compound 8c in off-white solid (0.02 g, 17.3%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 9.19 (t, J = 5.6 Hz, 1H), 7.86 (d, J = 5.0 Hz, 1H), 7.71 (d, J = 8.2 Hz, 2H), 7.63 (s, 1H), 7.54 (d, J = 7.2 Hz, 2H), 7.49 (t, J = 7.7 Hz, 1H), 7.30 - 7.20 (m, 2H), 7.01 - 6.90 (m, 2H), 4.60 - 4.51 (m, 2H), 4.35 (d, J = 5.1 Hz, 1H), 2.54 (dd, J = 14.5, 2.7 Hz, 1H), 2.37 (d, J = 11.3 Hz, 1H), 1.37 (s, 3H); Purity ≥99.4% (RP-HPLC, Rₜ = 19.7 min). (No interchangeable protons are observed).

### 8) 3-(2-Methyl-4-oxo-5,6-dihydro-2H-2,6-methanobenzo[g][1,3,5]oxadiazocin-3(4H)-yl)-N-(4-(trifluoromethyl)phenethyl)benzamide (Compound 8d)

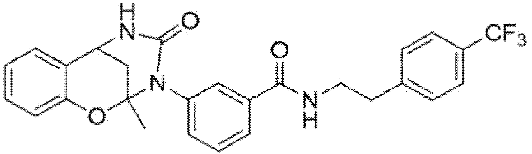

Compound 8d in off-white solid (0.03 g, 23.6%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 8.62 (t, J = 5.6 Hz, 1H), 7.74 (d, J = 7.9 Hz, 1H), 7.68 (dd, J = 13.5, 6.6 Hz, 3H), 7.57 (s, 1H), 7.46 (dd, J = 15.5, 7.7 Hz, 3H), 7.31 - 7.18 (m, 3H), 6.98 (t, J = 7.5 Hz, 1H), 6.93 (d, J = 8.1 Hz, 1H), 4.35 (d, J = 4.5 Hz, 1H), 3.58 - 3.46 (m, 2H), 2.95 (t, J = 6.9 Hz, 2H), 2.54 (d, J = 14.1 Hz, 1H), 2.37 (d, J = 11.3 Hz, 1H), 1.35 (s, 3H); Purity ≥99.7% (RP-HPLC, Rₜ = 20.2 min).

### 9) N-(2,3-Dihydro-1H-inden-2-yl)-3-(2-methyl-4-oxo-5,6-dihydro-2H-2,6-methanobenzo[g][1,3,5]oxadiazocin-3(4H)-yl)benzamide (Compound 8e)

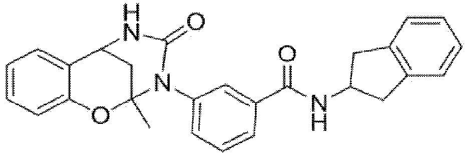

Compound 8e in off-white solid (0.04 g, 34.3%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 8.69 (d, J = 6.7 Hz, 1H), 7.82 (d, J = 7.4 Hz, 1H), 7.68 (d, J = 4.5 Hz, 1H), 7.63 (s, 1H), 7.45 (t, J = 7.8 Hz, 1H), 7.26 (dd, J = 15.4, 7.7 Hz, 4H), 7.19 - 7.13 (m, 2H), 6.98 (t, J = 7.5 Hz, 1H), 6.92 (d, J = 8.7 Hz, 1H), 4.70 (d, J = 7.5 Hz, 1H), 4.34 (s, 1H), 3.24 (dd, J = 16.2, 8.0 Hz, 2H), 2.97 (dd, J = 16.0, 6.5 Hz, 2H), 2.54 (d, J = 13.1 Hz, 1H), 2.37 (d, J = 12.3 Hz, 1H), 1.36 (s, 3H); Purity ≥99.8% (RP-HPLC, Rₜ = 18.5 min). (No interchangeable protons are observed).

### 10) 3-(2-Methyl-4-oxo-5,6-dihydro-2H-2,6-methanobenzo[g][1,3,5]oxadiazocin-3(4H)-yl)-N-(pyridin-3-ylmethyl)benzamide (Compound 8f)

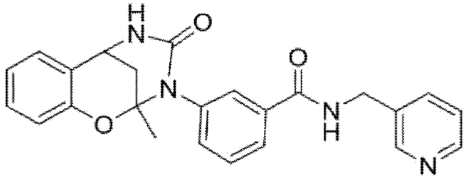

Compound 8f in off-white solid (0.05 g, 41.6%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 9.14 (t, J = 5.7 Hz, 1H), 8.56 (s, 1H), 8.47 (d, J = 4.5 Hz, 1H), 7.83 (d, J = 8.0 Hz, 1H), 7.71 (dd, J = 13.4, 6.4 Hz, 2H), 7.62 (s, 1H), 7.48 (t, J = 7.9 Hz, 1H), 7.37 (dd, J = 8.0, 5.4 Hz, 1H), 7.26 (dd, J = 15.6, 8.3 Hz, 2H), 7.01 - 6.90 (m, 2H), 4.56 - 4.42 (m, 2H), 4.35 (d, J = 4.3 Hz, 1H), 2.54 (dd, J = 13.5, 2.6 Hz, 1H), 2.37 (dd, J = 13.0, 2.6 Hz, 1H), 1.36 (s, 3H); Purity ≥99.9% (RP-HPLC, Rₜ = 9.84 min).

### 11) N-(Furan-2-ylmethyl)-3-(2-methyl-4-oxo-5,6-dihydro-2H-2,6-methanobenzo[g][1,3,5]oxadiazocin-3(4H)-yl)benzamide (Compound 8g)

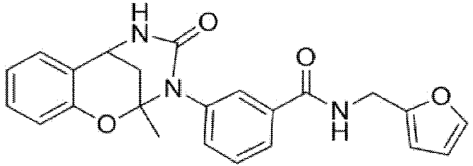

Compound 8g in off-white solid (0.05 g, 45.4%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 9.01 (t, J = 5.4 Hz, 1H), 7.82 (d, J = 7.5 Hz, 1H), 7.69 (d, J = 3.9 Hz, 1H), 7.63 (s, 1H), 7.58 (s, 1H), 7.46 (t, J = 7.3 Hz, 1H), 7.26 (dd, J = 16.4, 7.6 Hz, 3H), 7.02 - 6.90 (m, 2H), 6.42 - 6.38 (m, 1H), 6.28 (d, J = 2.8 Hz, 1H), 4.53 - 4.40 (m, 2H), 4.35 (s, 1H), 2.54 (dd, J = 12.0, 2.6 Hz, 1H), 2.37 (d, J = 12.3 Hz, 1H), 1.36 (s, 3H); Purity ≥99.2% (RP-HPLC, Rₜ = 15.9 min). (No interchangeable protons are observed).

### 12) N-Cyclopropyl-3-(2-methyl-4-oxo-5,6-dihydro-2H-2,6-methanobenzo[g][1,3,5]oxadiazocin-3(4H)-yl)benzamide (Compound 8h)

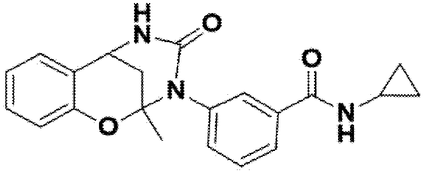

Compound 8h in off-white solid (0.02 g, 47.7%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 8.47 (d, J = 4.7 Hz, 1H), 7.75 (d, J = 7.2 Hz, 1H), 7.68 (d, J = 5.1 Hz, 1H), 7.58 (s, 1H), 7.44 (t, J = 7.5 Hz, 1H), 7.31 - 7.17 (m, 3H), 7.01 - 6.91 (m, 2H), 4.35 (d, J = 5.2 Hz, 1H), 2.89 - 2.78 (m, 1H), 2.57 - 2.52 (m, 1H), 2.37 (d, J = 11.1 Hz, 1H), 1.35 (s, 3H), 0.74 - 0.65 (m, 2H), 0.61 - 0.53 (m, 2H); Purity ≥99.5% (RP-HPLC, Rₜ = 14.0 min).

### 13) N-Benzyl-N-methyl-3-(2-methyl-4-oxo-5,6-dihydro-2H-2,6-methanobenzo[g][1,3,5]oxadiazocin-3(4H)-yl)benzamide (Compound 8i)

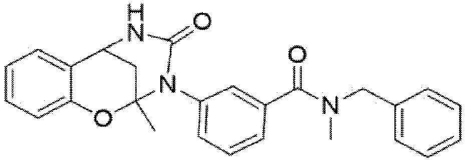

Compound 8i in off-white solid (0.03 g, 27.3%) was prepared from 3-(2-methyl-4-oxo-5,6-dihydro-2H-2,6-methanobenzo[g][1,3,5]oxadiazocin-3(4H)-yl)benzoic acid (Compound 7) according to the general process of acid-amine coupling (E).

¹H NMR (400 MHz, DMSO-d₆) δ 7.67 (d, J = 4.1 Hz, 1H), 7.36 (d, J = 18.1 Hz, 6H), 7.29 - 7.21 (m, 2H), 7.14 (bs, 2H), 6.98 (t, J = 7.5 Hz, 1H), 6.87 (bs, 1H), 4.67 (s, 1H), 4.49 (s, 1H), 4.33 (s, 1H), 2.86 (d, J = 20.4 Hz, 3H), 2.52 (s, 1H), 2.34 (d, J = 13.4 Hz, 2H), 1.38 (s, 2H); Purity ≥99.1% (RP-HPLC, Rₜ = 12.9 min).

### 14) 2,5-Dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 9)

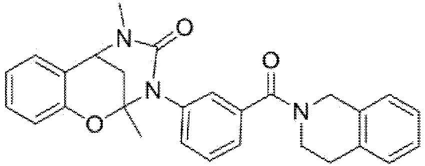

Methyl iodide (0.056 mL, 0.60 mmol) was added at room temperature to a solution in which 2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (0.10 g, 0.30 mmol) and Cs₂CO₃(0.195 g, 0.90 mmol) were dissolved in acetonitrile (5 mL), and the mixture was stirred at ambient temperature for 12 hours. The reaction mixture was diluted with ice-cold water (25 mL), and the aqueous phase was extracted with ethyl acetate (3 x 20 mL). The combined organic layers were dried on anhydrous sodium sulfate, filtered, and concentrated under reduced pressure. Residues were purified by silica gel column chromatography (5-10% MeOH dissolved in DCM) to obtain Chemical Formula 9 in off-white solid (0.03 g, 25.7%).

¹H NMR (400 MHz, CD₃OD) δ 7.48 (t, J = 7.7 Hz, 1H), 7.44 - 7.36 (m, 2H), 7.32 - 7.05 (m, 6H), 6.97 (dd, J = 17.8, 13.4 Hz, 3H), 4.76 (bs, 1H), 4.65 - 4.52 (bm, 1H), 4.47 (s, 1H), 3.57 (bs, 3H), 2.91 (s, 3H), 2.87 - 2.83 (s, 1H), 2.67 (d, J = 12.4 Hz, 1H), 2.40 (d, J = 10.8 Hz, 1H), 1.40 (s, 3H); Purity ≥98.1% (RP-HPLC, Rₜ = 19.5 min).

### Chiral separation of 3-(2-methyl-4-oxo-5,6-dihydro-2H-2,6-methanobenzo[g][1,3,5]oxadiazocin-3(4H)-yl)benzoic acid (Compound 7)

In order to separate an enantiomer of 3-(2-methyl-4-oxo-5,6-dihydro-2H-2,6-methanobenzo[g][1,3,5]oxadiazocin-3(4H)-yl)benzoic acid (Compound 7), (R,R)-Compound 7a (peak 1, Rₜ = 6.020) and (S,S)-Compound 7b (peak 2, Rₜ = 6.817) were separated using chiral SFC (column: ChiralPak-IE-3, 4.6 x 250 mm, 3 um; mobile phase: Hexane/EtOH/DEA/TFA (30/70/0.1/0.1); flow rate:1.0 mL/min; temperature: 25°C; UV: 235 nm).

### 15) 3-((2R,6R)-2-Methyl-4-oxo-5,6-dihydro-2H-2,6-methanobenzo[g][1,3,5]oxadiazocin-3(4H)-yl)benzoic acid (Compound 7a)

¹H NMR (400 MHz, DMSO-d₆) δ 13.04 (s, 1H), 7.88 (d, J = 7.9 Hz, 1H), 7.71 (d, J = 4.3 Hz, 1H), 7.66 (s, 1H), 7.50 (t, J = 7.8 Hz, 1H), 7.34 (d, J = 7.7 Hz, 1H), 7.27 (dd, J = 14.5, 7.4 Hz, 2H), 6.99 (t, J = 7.3 Hz, 1H), 6.93 (d, J = 8.0 Hz, 1H), 4.35 (s, 1H), 2.62 - 2.54 (m, 1H), 2.36 (d, J = 13.3 Hz, 1H), 1.37 (s, 3H); Chiral SFC assay: Purity ≥99.5%, Rₜ = 6.045 (column: ChiralPak-IE-3, 4.6 x 250mm, 3 *µ*m; mobile phase: Hexane/EtOH/DEA/TFA (30/70/0.1/0.1); Flow rate:1.0 mL/min; Temperature: 25°C; UV: 235 nm).

### 16) 3-((2S,6S)-2-Methyl-4-oxo-5,6-dihydro-2H-2,6-methanobenzo[g][1,3,5]oxadiazocin-3(4H)-yl)benzoic acid (Compound 7b)

¹H NMR (400 MHz, DMSO-d₆) δ 13.04 (s, 1H), 7.88 (d, J = 7.9 Hz, 1H), 7.71 (d, J = 4.3 Hz, 1H), 7.66 (s, 1H), 7.50 (t, J = 7.8 Hz, 1H), 7.34 (d, J = 7.7 Hz, 1H), 7.27 (dd, J = 14.5, 7.4 Hz, 2H), 6.99 (t, J = 7.3 Hz, 1H), 6.93 (d, J = 8.0 Hz, 1H), 4.35 (s, 1H), 2.62 - 2.54 (m, 1H), 2.36 (d, J = 13.3 Hz, 1H), 1.37 (s, 3H); Chiral SFC assay: Purity ≥99.9%, Rₜ = 6.859 (column: ChiralPak-IE-3, 4.6 x 250mm, 3 *µ*m; mobile phase: Hexane/EtOH/DEA/TFA (30/70/0.1/0.1); flow rate:1.0 mL/min; Temperature: 25°C; UV: 235 nm).

### 17) (2R,6R)-2-Methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 10a)

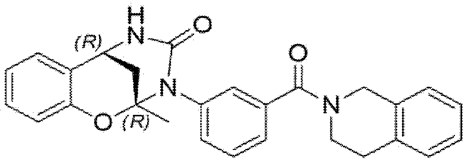

Compound 10a in off-white solid (0.04 g, 36.9%) was prepared from 3-((2R,6R)-2-methyl-4-oxo-5,6-dihydro-2H-2,6-methanobenzo[g][1,3,5]oxadiazocin-3(4H)-yl)benzoic acid (Compound 7a) according to the general process of acid-amine coupling (E).

SOR: [α]²³_{D} -61.05° (c 0.1, MeOH); ¹H NMR (400 MHz, CD₃OD) δ 7.51 (m, 2H), 7.37 - 7.04 (m, 7H), 7.01 - 6.77 (m, 3H), 4.62 (bs, 1H), 4.43 (bs, 1H), 3.96 (bs, 1H), 3.66 (bs, 1H), 2.91 (d, J = 50.3 Hz, 3H), 2.65 (d, J = 13.2 Hz, 1H), 2.43 (dd, J = 13.3, 3.0 Hz, 1H), 1.48 (s, 3H); Purity ≥98.9% (RP-HPLC, Rₜ = 18.1 min); Chiral SFC assay: Purity ≥99.5%, Rₜ = 9.86 (column: Chiralpak-IE-3, 4.6 x 150mm, 3 *µ*m; mobile phase: Hexane/EtOH/DEA/TFA (30/70/0.1/0.1); flow rate:0.8 mL/min; temperature: 25°C, UV:235 nm).

### 18) N-(4-Bromobenzyl)-3-((2R,6R)-2-methyl-4-oxo-5,6-dihydro-2H-2,6-methanobenzo[g][1,3,5]oxadiazocin-3(4H)-yl)benzamide (Compound 10b)

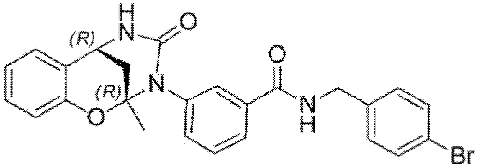

Compound 10b in light brown solid (0.01 g, 17.0%) was prepared from 3-((2R,6R)-2-methyl-4-oxo-5,6-dihydro-2H-2,6-methanobenzo[g][1,3,5]oxadiazocin-3(4H)-yl)benzoic acid (Compound 7a) according to the general process of acid-amine coupling (E).

¹H NMR (400 MHz, DMSO-d₆) δ 9.10 (t, J = 6.0 Hz, 1H), 7.84 (d, J = 8.5 Hz, 1H), 7.67 (d, J = 4.8 Hz, 1H), 7.62 (s, 1H), 7.52 (d, J = 3.8 Hz, 1H), 7.47 (t, J = 8.1 Hz, 1H), 7.26 (dd, J = 17.1, 7.3 Hz, 4H), 6.98 (t, J = 7.6 Hz, 1H), 6.93 (d, J = 8.2 Hz, 1H), 4.51 - 4.37 (m, 2H), 4.35 (s, 1H), 2.54 (dd, J = 13.4, 2.8 Hz, 1H), 2.37 (d, J = 13.7 Hz, 1H), 1.36 (s, 3H); Purity ≥98.8% (RP-HPLC, Rₜ = 19.2 min). (No interchangeable protons are observed).

### 19) (2S,6S)-2-Methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 10c)

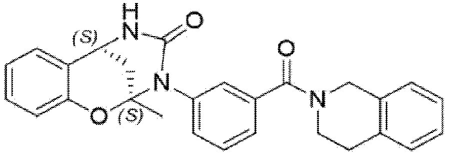

Compound 10c in off-white solid (0.04 g, 36.9%) was prepared from 3-((2S,6S)-2-methyl-4-oxo-5,6-dihydro-2H-2,6-methanobenzo[g][1,3,5]oxadiazocin-3(4H)-yl)benzoic acid (Compound 7b) according to the general process of acid-amine coupling (E).

SOR: [α]²³_{D} +66.44° (c 0.1,MeOH); ¹H NMR (400 MHz, CD₃OD) δ 7.51 (m, 2H), 7.37 - 7.04 (m, 7H), 7.01 - 6.77 (m, 3H), 4.62 (bs, 1H), 4.43 (bs, 1H), 3.96 (bs, 1H), 3.66 (bs, 1H), 2.91 (d, J = 50.3 Hz, 3H), 2.65 (d, J = 13.2 Hz, 1H), 2.43 (dd, J = 13.3, 3.0 Hz, 1H), 1.48 (s, 3H); Purity ≥98.9% (RP-HPLC, Rₜ = 18.2 min); Chiral SFC assay: Purity ≥99.5%, Rₜ = 12.25 (column: Chiralpak-IE-3, 4.6 x 150mm, 3 *µ*m; mobile phase: Hexane/EtOH/DEA/TFA (30/70/0.1/0.1); flow rate: 0.8 mL/min; temp: 25°C, UV:235 nm). (No interchangeable protons are observed).

### 20) N-(3,5-Dibromobenzyl)-3-((2S,6S)-2-methyl-4-oxo-5,6-dihydro-2H-2,6-methanobenzo[g][1,3,5]oxadiazocin-3(4H)-yl)benzamide (Compound 10d)

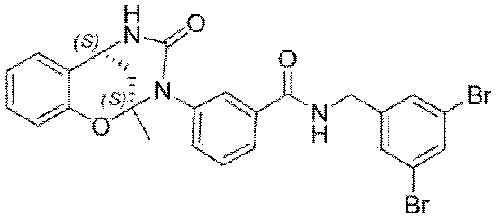

Compound 10d in off-white solid (0.04 g, 36.9%) and light brown solid (0.01 g, 14.6%) was prepared from 3-((2S,6S)-2-methyl-4-oxo-5,6-dihydro-2H-2,6-methanobenzo[g][1,3,5]oxadiazocin-3(4H)-yl)benzoic acid (Compound 7b) according to the general process of acid-amine coupling (E).

¹H NMR (400 MHz, DMSO-d₆) δ 9.13 (t, J = 5.0 Hz, 1H), 7.83 (d, J = 7.0 Hz, 1H), 7.73 (s, 1H), 7.69 (d, J = 4.5 Hz, 1H), 7.62 (s, 1H), 7.54 (s, 1H), 7.49 (t, J = 8.0 Hz, 1H), 7.26 (dd, J = 15.2, 7.5 Hz, 3H), 6.99 (d, J = 7.6 Hz, 1H), 6.97 - 6.91 (m, 1H), 4.48 - 4.42 (m, 2H), 4.35 (d, J = 4.6 Hz, 1H), 2.54 (d, J = 12.6 Hz, 1H), 2.37 (d, J = 11.8 Hz, 1H), 1.37 (s, 3H); Purity ≥99.2% (RP-HPLC, Rₜ = 21.5min). (No interchangeable protons are observed). 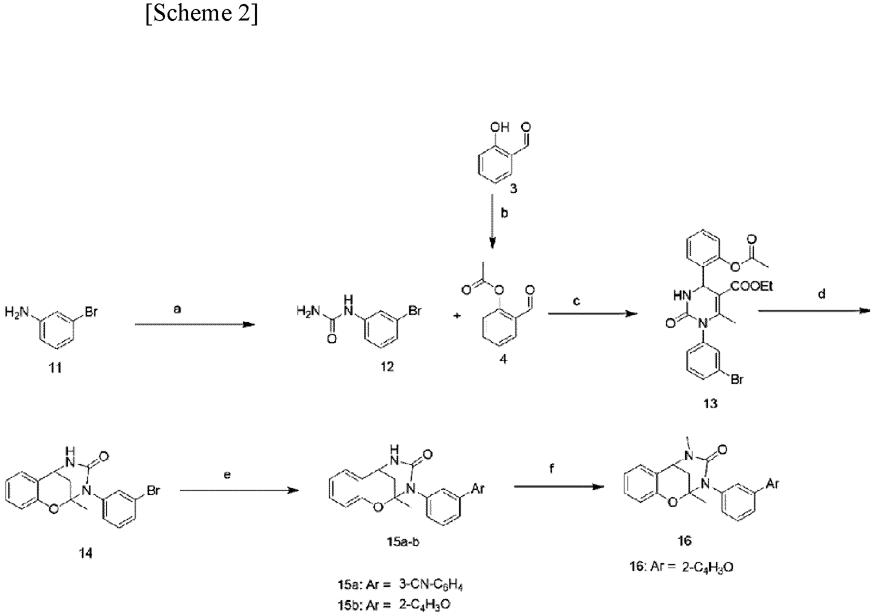 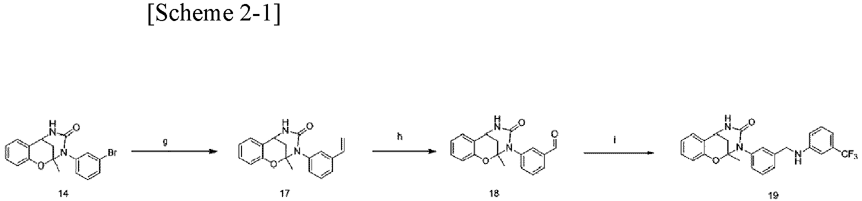
(a) KOCN, H₂O, AcOH, room temperature for 12 hours;
(b) Acetic anhydride, pyridine, room temperature for 12 hours;
(c) Ethyl acetoacetate, TMSCl, DMF, room temperature (rt), 12 hours;
(d) NaHCO₃, MeOH-H₂O, 40°C, 16 hours; LiOH, THF-H₂O, 40°C, 18 hours; 1 M HCl pH 1-2, 80°C, 6 hours;
(e) Boronic acid, Pd(dppf)Cl₂.DCM, CS₂CO₃, 1,4-dioxane:H₂O (10: 1) mixture, 100°C, 12 hours;
(f) MeI, CS₂CO₃, acetonitrile, room temperature for 12 hours;
(g) Potassium vinyltrifluoroborate, Pd(dppf)Cl₂.DCM, Et₃N, EtOH, 80°C for 2 hours;
(h) 2.5% OsO₄ dissolved in butanol, NaIO₄, acetone/H₂O, room temperature for 4 hours;
(i) 3-(Trifluoromethyl)aniline, AcOH, NaCNBH₃, DCM, room temperature for 12 hours.

The starting material 2-bromoaniline and KOCN were reacted to form a urea structure. By reacting the urea material thus obtained with acetic acid, aldehyde, and TMSCl, Compound 13 was synthesized through the key reaction, the Biginelli reaction. Subsequently, Compound 14 was obtained through cyclization using NaHCO₃, and Compounds 15a-b was obtained through Suzuki coupling using boronic acid and Pd(dppf)Cl₂.DCM. Compound 16 was synthesized by inducing methylation using MeI to introduce a methyl group at the secondary amine site of Compound 15b.

Additionally, Compound 17 was synthesized through olefination that Compound 14 was reacted with potassium vinyltrifluoroborate. Afterwards, an aldehyde structure was formed through oxidation using OsO₄, and then Compound 19 was synthesized through reductive amination in which the aldehyde and amine were reacted.

### 21) 1-(3-Bromophenyl)urea (Compound 12)

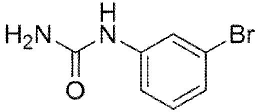

Compound 12 in white solid (8.00 g, 90.9%) was prepared from methyl 3-bromoaniline (Compound 11) according to the general process of converting amines to urea (A) to be used in the next step without additional purification.

¹H NMR (400 MHz, DMSO-d₆) d 8.98 (s, 1H), 7.84 (m, 1H), 7.23 (d, J = 7.8 Hz, 1H), 7.16 d, J = 8.0 Hz, 1H), 7.04 (d, J = 2.0 Hz, 1H), 6.05 (s, 2H).

### 22) Ethyl 4-(2-acetoxyphenyl)-1-(3-bromophenyl)-6-methyl-2-oxo-1,2,3,4-tetrahydropyrimidine-5-carboxylate (Compound 13)

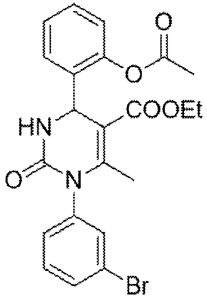

Compound 13 in white solid (2.30 g, 13.3%) was prepared from 2-formylphenyl acetate (Compound 4) and 1-(3-bromophenyl)urea (Compound 12) according to Biginelli reaction (C).

¹H NMR (400 MHz, CDCl₃) d 7.55 (ddd, J = 8.1, 1.8, 1.0 Hz, 1H), 7.44 (dd, J = 7.4, 1.9 Hz, 1H), 7.40 (s, 1H), 7.37 - 7.28 (m, 3H), 7.19 (d, J = 8.3 Hz, 1H), 7.08 (dd, J = 7.7, 1.4 Hz, 1H), 5.69 (s, 1H), 5.55 (s, 1H), 4.05 - 3.98 (m, 2H), 2.38 (s, 3H), 2.20 (s, 3H), 1.07 (t, J = 7.1 Hz, 3H).

### 23) 3-(3-Bromophenyl)-2-methyl-5,6-dihydro-2H-2,6-methanobenzo[g][1,3,5]oxadiazocin-4(3H)-one (Compound 14)

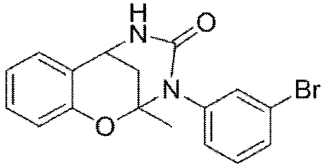

Compound 14 in pink solid (0.80 g, 45.9%) was prepared from ethyl 4-(2-acetoxyphenyl)-1-(3-bromophenyl)-6-methyl-2-oxo-1,2,3,4-tetrahydropyrimidine-5-carboxylate (Compound 13) according to the general process (D) to be used in the next step without additional purification.

¹H NMR (400 MHz, DMSO-d₆) d 7.81 (d, J = 6.0 Hz, 1H), 7.53 (d, J = 8.4 Hz, 1H), 7.40 (d, J = 10.6 Hz, 1H), 7.29 - 7.23 (m, 3H), 7.13 (d, J = 8.0 Hz, 1H), 7.0 - 7.06 (m, 2H), 4.34 (d, J = 4.0 Hz, 1H), 2.55 (d, J = 2.8 Hz, 1H), 2.35 (d, J = 11.6 Hz, 1H), 1.47 (s, 3H).

### 24) 3'-(2-Methyl-4-oxo-5,6-dihydro-2H-2,6-methanobenzo[g][1,3,5]oxadiazocin-3(4H)-yl)-[1,1'-biphenyl]-3-carbonitrile (Compound 15a)

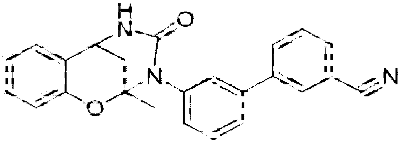

Compound 15a in off-white solid (0.07 g, 59.8%) was prepared from 3-(3-bromophenyl)-2-methyl-5,6-dihydro-2H-2,6-methanobenzo[g][1,3,5]oxadiazocin-4(3H)-one (Compound 14) according to the general process of Suzuki couplings F (Method B).

¹H NMR (400 MHz, CDCl₃) δ 7.86 (d, J = 1.2 Hz, 1H), 7.83 - 7.78 (m, 1H), 7.65 - 7.61 (m, 1H), 7.57 - 7.47 (m, 3H), 7.43 - 7.40 (m, 1H), 7.34 - 7.27 (m, 2H), 7.18 (dd, J = 7.8, 1.5 Hz, 1H), 7.02 - 6.95 (m, 2H), 5.53 (d, J = 4.0 Hz, 1H), 4.41 (d, J = 7.5 Hz, 1H), 2.68 (dd, J = 13.0, 2.9 Hz, 1H), 2.48 - 2.42 (m, 1H), 1.52 (s, 3H); Purity ≥98.9% (RP-HPLC, Rₜ = 20.1 min).

### 25) 3-(3-(Furan-2-yl)phenyl)-2-methyl-5,6-dihydro-2H-2,6-methanobenzo[g][1,3,5]oxadiazocin-4(3H)-one (Compound 15b)

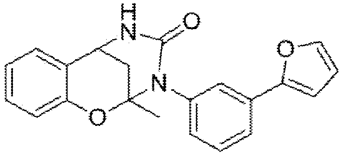

Compound 15b in off-white solid (0.06 g, 61.3%) was prepared from 3-(3-bromophenyl)-2-methyl-5,6-dihydro-2H-2,6-methanobenzo[g][1,3,5]oxadiazocin-4(3H)-one (Compound 14) according to the general process of Suzuki couplings F (Method B).

¹H NMR (400 MHz, CD₃OD) d 7.70 - 7.65 (m, 1H), 7.56 (dd, J = 1.8, 0.6 Hz, 1H), 7.48 (s, 1H), 7.41 (t, J = 7.8 Hz, 1H), 7.31 (dd, J = 12.0, 4.5 Hz, 2H), 7.07 (d, J = 8.2 Hz, 1H), 7.02 - 6.96 (m, 2H), 6.74 (d, J = 3.0 Hz, 1H), 6.51 (dd, J = 3.4, 1.8 Hz, 1H), 4.44 (t, J = 3.0 Hz, 1H), 2.65 (dd, J = 13.2, 3.0 Hz, 1H), 2.44 (dd, J = 13.2, 3.3 Hz, 1H), 1.48 (s, 3H); Purity ≥96.4% (RP-HPLC, Rₜ = 19.7 min).

### 26) 3-(3-(Furan-2-yl)phenyl)-2,5-dimethyl-5,6-dihydro-2H-2,6-methanobenzo[g][1,3,5]oxadiazocin-4(3H)-one (Compound 16)

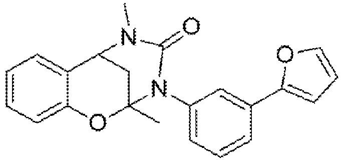

Iodomethane (0.036 g, 0.25 mmol) was added to a solution in which 3-(3-(furan-2-yl)phenyl)-2,5-dimethyl-5,6-dihydro-2H-2,6-methanobenzo[g][1,3,5]oxadiazocin-4(3H)-one (0.03 g, 0.08 mmol) and Cs₂CO₃ (84.6 g, 0.25 mmol) were dissolved in acetonitrile (10 mL), and the mixture was stirred at ambient temperature for 12 hours. The reaction mixture was diluted with water (10 mL), and the aqueous phase was extracted with EtOAc (3 x 20 mL). The combined organic layers were dried with anhydrous sodium sulfate, filtered, and concentrated under reduced pressure The residues were purified by silica gel column chromatography (25% EtOAc dissolved in hexane) to obtain Compound 16 in white solid (0.01 g, 32.2%).

¹H NMR (400 MHz, CD₃OD) d 7.69 - 7.65 (m, 1H), 7.56 (dd, J = 1.8, 0.6 Hz, 1H), 7.45 (s, 1H), 7.41 - 7.30 (m, 3H), 7.07 - 6.98 (m, 3H), 6.74 (d, J = 3.3 Hz, 1H), 6.51 (dd, J = 3.4, 1.8 Hz, 1H), 4.47 (t, J = 3.1 Hz, 1H), 3.05 (s, 3H), 2.72 (dd, J = 13.3, 3.0 Hz, 1H), 2.43 (dd, J = 13.3, 3.3 Hz, 1H), 1.46 (s, 3H); Purity ≥99.6% (P-HPLC, Rₜ = 21.2 min).

### 27) 2-Methyl-3-(3-vinylphenyl)-5,6-dihydro-2H-2,6-methanobenzo[g][1,3,5]oxadiazocin-4(3H)-one (Compound 17)

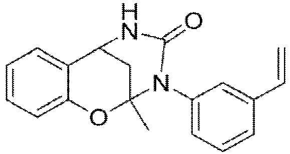

Et₃N (0.54 mL, 4.17 mmol) was added to a solution in which 3-(3-bromophenyl)-2-methyl-5,6-dihydro-2H-2,6-methanobenzo[g][1,3,5]oxadiazocin-4(3H)-one (0.50 g, 1.39 mmol) and potassium vinyltrifluoroborate (0.27 g, 2.08 mmol) were dissolved in EtOH (10 mL). The resulting reaction mixture was purged under argon for 10 minutes, and then Pd(dppf)Cl₂.DCM (0.113g, 0.13mmol) was added. The resulting mixture was heated in a sealed tube at 80°C for 2 hours. The reaction mixture was cooled to room temperature and diluted with water (20 mL), and then the aqueous phase was extracted with ethyl acetate (3 x 30 mL). The combined organic layers were dried with anhydrous sodium sulfate, filtered, and evaporated under reduced pressure. Residues were purified by silica gel column chromatography (50-100% EtOAc dissolved in hexane) to obtain Compound 18 in off-white solid (0.32 g, 70.5%).

¹H NMR (400 MHz, CD₃OD) δ 7.41 (d, J = 7.8 Hz, 1H), 7.35 (t, J = 7.8 Hz, 1H), 7.28 (ddd, J = 7.3, 4.2, 1.5 Hz, 2H), 7.22 (s, 1H), 7.06 (d, J = 8.1 Hz, 1H), 6.97 (dd, J = 12.1, 4.7 Hz, 2H), 6.73 (dd, J = 17.6, 10.9 Hz, 1H), 5.76 (d, J = 17.5 Hz, 1H), 5.26 (d, J = 11.0 Hz, 1H), 4.42 (t, J = 3.0 Hz, 1H), 2.63 (dd, J = 13.2, 3.0 Hz, 1H), 2.42 (dd, J = 13.2, 3.2 Hz, 1H), 1.45 (s, 3H).

### 29) 3-(2-Methyl-4-oxo-5,6-dihydro-2H-2,6-methanobenzo[g][1,3,5]oxadiazocin-3(4H)-yl)benzaldehyde (Compound 18)

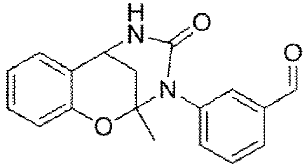

2.5% osmium tetroxide (0.1 mL, 0.0097 mmol) was added to a solution in which 2-methyl-3-(3-vinylphenyl)-5,6-dihydro-2H-2,6-methanobenzo[g][1,3,5]oxadiazocin-4(3H)-one (0.30 g, 0.97 mmol) is dissolved in 30 mL of acetone:H₂O (7:3) mixture, the mixture was then stirred at room temperature for 2 hours, and NaIO₄ (0.31 g, 1.46 mmol) was added to the stirred solution. The reaction mixture was diluted with water (30 mL), and the aqueous phase was extracted with EtOAc (3 x 40 mL). The combined organic layers were dried with anhydrous sodium sulfate, filtered, and concentrated under reduced pressure The residues were purified by silica gel column chromatography (80-100 % ETOAc dissolved in hexane) to obtain Compound 16 in white solid (0.18 g, 59.8%).

¹H NMR (400 MHz, CDCl₃) δ 10.01 (s, 1H), 7.87 (dt, J = 7.5, 1.3 Hz, 1H), 7.74 (t, J = 1.6 Hz, 1H), 7.57 (t, J = 7.7 Hz, 1H), 7.52 - 7.48 (m, 1H), 7.31 (dd, J = 7.2, 1.4 Hz, 1H), 7.17 (dd, J = 7.4, 1.5 Hz, 1H), 7.03 - 6.98 (m, 2H), 5.73 (s, 1H), 4.40 (d, J = 4.3 Hz, 1H), 2.67 (dd, J = 13.0, 3.0 Hz, 1H), 2.44 (ddd, J = 13.0, 3.2, 2.0 Hz, 1H), 1.48 (s, 3H).

### 28) (2-Methyl-3-(3-(((3-(trifluoromethyl)phenyl)amino)methyl)phenyl)-5,6-dihydro-2H-2,6-methanobenzo[g][1,3,5]oxadiazocin-4(3H)-one (Compound 19)

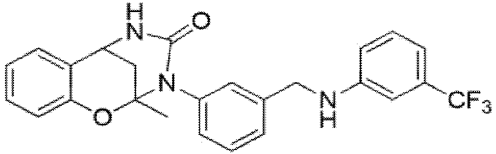

3-(Trifluoromethyl)aniline (0.06 g, 0.36 mmol) and acetic acid (0.1 mL) were added to a solution in which 3-(2-methyl-4-oxo-5,6-dihydro-2H-2,6-methanobenzo[g][1,3,5]oxadiazocin-3(4H)-yl)benzaldehyde (0.10 g, 0.30 mmo) is dissolved in DCM (30 mL), the mixture was stirred at room temperature for 2 hours, and then sodium cyanoborohydride (0.03 g, 2.0 eq) was added to the stirred solution. The reaction mixture was stirred at room temperature for 12 hours. The reaction mixture was quenched with an aqueous sodium bicarbonate solution (30 mL), and the aqueous phase was extracted with DCM (3 x 40 mL). The combined organic layers were dried with anhydrous sodium sulfate, filtered, and evaporated under reduced pressure. Residues were purified by silica gel column chromatography (0 - 8% MeOH in DCM) to obtain Compounds 19 in white solid (0.05 g, 40.4%).

¹H NMR (400 MHz, DMSO-d₆) δ 7.56 (d, J = 4.5 Hz, 1H), 7.35 - 7.14 (m, 5H), 7.04 (s, 1H), 6.94 (t, J = 8.2 Hz, 2H), 6.80 (bt, J = 7.8 Hz, 3H), 4.34 (d, J = 5.6 Hz, 2H), 4.30 (s, 1H), 2.47 (d, J = 2.4 Hz, 1H), 2.30 (d, J = 15.1 Hz, 1H), 1.26 (s, 3H). purity ≥97.0% (RP-HPLC, Rₜ = 18.8 min). (No interchangeable protons are observed). 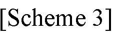 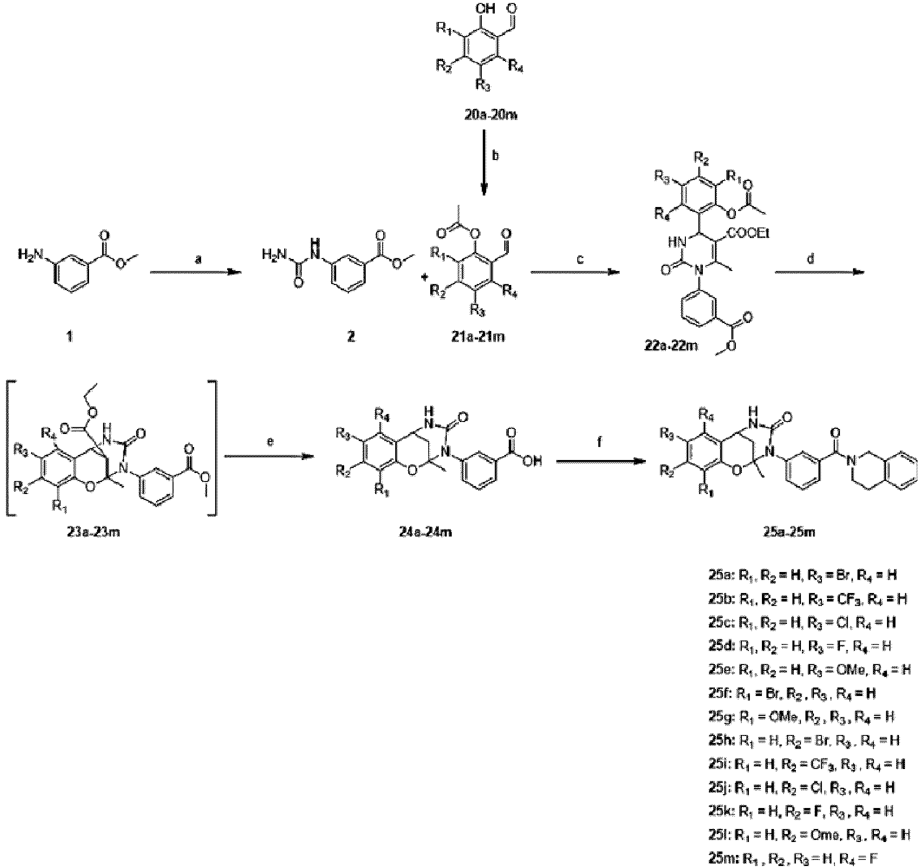
(a) KOCN, H₂O, AcOH, room temperature (rt), 12 hours;
(b) Acetic anhydride, pyridine, room temperature for 12 hours;
(c) Ethyl acetoacetate, TMSCl, DMF, room temperature (rt), 12 hours;
(d) NaHCO₃, MeOH-H₂O, 40°C, 16 hours;
(e) LiOH, THF-H₂O, 40°C, 18 hours; 4M HCl pH1-2, 80°C, 6 hours;
(f) 1,2,3,4-Tetrahydroisoquinoline, HATU, DIPEA, DMF, room temperature for 2 hours.

In order to introduce various derivatives such as Br, Cl, CF₃, F, and OMe at the R₁~R₄ positions, 25a-25m were synthesized through the reactions utilized in Scheme 1 and 2.

### 29) 4-Bromo-2-formylphenyl acetate (Compound 21a)

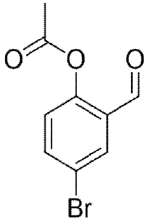

Compound 21a in yellow powder (10.5 g, 66.0%) was prepared from 5-bromo-2-hydroxybenzaldehyde (compound 20a) according to the general process of acylation (B) to be used in the next step without additional purification.

¹H NMR (400 MHz, DMSO-d₆) d 10.05 (s, 1H). 7.88-7.80 (d, J = 8.0 Hz, 1H), 7.74-7.65 (m, 2H), 2.35-2.32 (s, 3H).

### 30) 2-Formyl-4-(trifluoromethyl)phenyl acetate (Compound 21b)

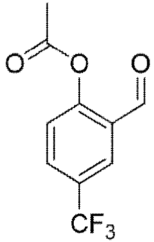

Compound 21b in off-white powder (2.85 g, 70.0%) was prepared from 2-hydroxy-5-(trifluoromethyl)benzaldehyde (Compound 20b) according to the general process of acylation (B) to be used in the next step without additional purification.

¹H NMR (400 MHz, DMSO-d₆) δ 10.18 (s, 1H), 8.25 (d, J = 4.0 Hz, 1H), 8.12 (dd, J = 8.0, 8.0 Hz, 1H), 7.59 (d, J = 8.0 Hz, 1H), 2.24 (s, 3H).

### 31) 4-Chloro-2-formylphenyl acetate (Compound 21c)

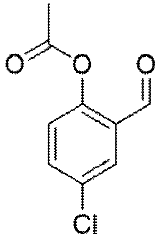

Compound 21c in off-white powder (2.30 g, 64.0%) was prepared from 5-chloro-2-hydroxybenzaldehyde (Compound 20c) according to the general process of acylation (B).

¹H NMR (400 MHz, DMSO-d₆) δ 10.08 (s, 1H), 7.90 (d, J = 4 Hz, 1H), 7.81 - 7.75 (m, 1H), 7.36 (s, 1H), 2.24 (s, 3H).

### 32) 4-Fluoro-2-formylphenyl acetate (Compound 21d)

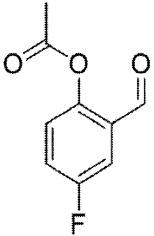

Compound 21d in off-white powder (2.00 g, 74.0%) was prepared from 5-fluoro-2-hydroxybenzaldehyde (Compound 20d) according to the general process of acylation (B) to be used in the next step without additional purification.

¹H NMR (400 MHz, DMSO-d₆) d 10.05 (s, 1H), 8.01 (d, J = 8.0 Hz, 1H), 7.39 - 7.27 (m, 2H), 2.37 (s, 3H).

### 33) 2-Formyl-4-methoxyphenyl acetate (Compound 21e)

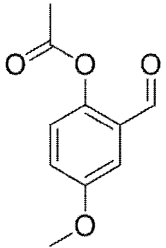

Compound 21e in off-white powder (2.00 g, 72.0%) was prepared from 2-hydroxy-5-methoxybenzaldehyde (Compound 20e) according to the general process of acylation (B).

¹H NMR (400 MHz, DMSO-d₆) δ 9.94 (s, 1H), 7.86 (d, J = 8.7 Hz, 1H), 7.04 (d, J = 2.5 Hz, 1H), 6.87 (s, 1H), 3.87 (s, 3H), 2.35 (s, 3H).

### 34) 2-Bromo-6-formylphenyl acetate (Compound 21f)

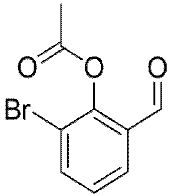

Compound 21f in yellow powder (3.50 g, 66.0%) was prepared from 3-bromo-2-hydroxybenzaldehyde (Compound 20f) according to the general process of acylation (B) to be used in the next step without additional purification.

¹H NMR (400 MHz, DMSO-d₆) d 10.83 (s, 1H), 8.86 - 8.81 (m, 8.0 Hz, 1H), 8.72 - 8.68 (m, 1H), 8.29-8.19 (m,1H) 2.33 (s, 3H).

### 35) 2-Formyl-6-methoxyphenyl acetate (Compound 21g)

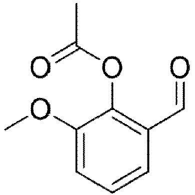

Compound 21g in off-white powder (3.00 g, 78.0%) was prepared from 2-hydroxy-3-methoxybenzaldehyde (Compound 20g) according to the general process of acylation (B) to be used in the next step without additional purification.

¹H NMR (400 MHz, DMSO-d₆) δ 9.96 (s, 1H), 7.88 (d, J = 8.0 Hz, 1H), 7.05 (d, J = 4.0 Hz, 1H), 6.89 (s, 1H), 3.86 (s, 3H), 2.34 (s, 3H).

### 36) 5-Bromo-2-formylphenyl acetate (Compound 21h)

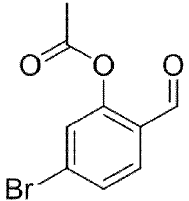

Compound 21h in off-white powder (3.40 g, 66.5%) was prepared from 4-bromo-2-hydroxybenzaldehyde (Compound 20h) according to the general process of acylation (B).

¹H NMR (400 MHz, DMSO-d₆) d 10.12-10.03 (s, 1H). 7.88-7.80 (d, J=8.0 Hz, 1H), 7.73-7.64 (m, 2H), 2.34-2.31 (S, 3H).

### 37) 2-Formyl-5-(trifluoromethyl)phenyl acetate (Compound 21i)

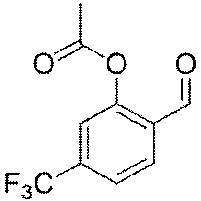

Compound 21i in off-white powder (2.85 g, 70.0%) was prepared from 2-hydroxy-4-(trifluoromethyl)benzaldehyde (Compound 20i) according to the general process of acylation (B) to be used in the next step without additional purification.

¹H NMR (400 MHz, DMSO-d₆) d 10.12-10.03 (s, 1H). 7.88-7.80 (d, J=8.0, 1H), 7.73-7.64 (m, 2H), 2.34-2.31 (s, 3H).

### 38) 5-Chloro-2-formylphenyl acetate (Compound 21j)

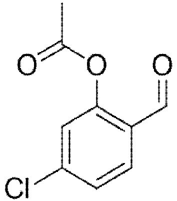

Compound 21j in off-white powder (2.20 g, 57.0%) was prepared from 4-chloro-2-hydroxybenzaldehyde (Compound 20j) according to the general process of acylation (B) to be used in the next step without additional purification.

¹H NMR (400 MHz, DMSO-d₆) δ 9.94 (s, 1H), 7.86 (dd, J = 8.0, 8.0 Hz, 1H), 7.05 - 6.97 (m, 1H), 6.88 (d, J = 2.4 Hz, 1H), 3.87 (s, 3H).

### 39) 5-Fluoro-2-formylphenyl acetate (Compound 21k)

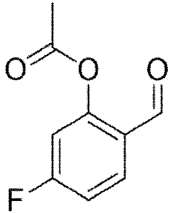

Compound 21k in yellow powder (2.50 g, 64.0%) was prepared from 4-fluoro-2-hydroxybenzaldehyde (compound 20k) according to the general process of acylation (B) to be used in the next step without additional purification.

¹H NMR (400 MHz, DMSO-d₆) δ 10.0 (s, 1H), 8.03 (dd, J = 8.0, 8.0 Hz, 1H), 7.45 - 7.24 (m, 2H), 2.35 (s, 3H).

### 40) 2-Formyl-5-methoxyphenyl acetate (Compound 21l)

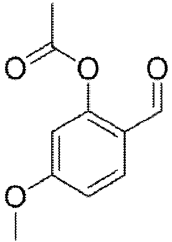

Compound 21l in yellow powder (3.00 g, 47.0%) was prepared from 2-hydroxy-4-methoxybenzaldehyde (compound 20l) according to the general process of acylation (B) to be used in the next step without additional purification.

¹H NMR (400 MHz, DMSO-d₆) δ 9.94 (s, 1H), 7.86 (d, J = 8.0 Hz, 1H), 7.02 (dd, J = 4.0, 4.0 Hz, 1H), 6.88 (d, J = 8.0 Hz, 1H), 3.87 (s, 3H), 2.35 (s, 3H).

### 41) 3-Fluoro-2-formylphenyl acetate (Compound 21m)

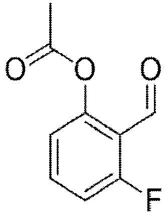

Compound 21m in off-white powder (0.55 g, 14.0%) was prepared from 2-fluoro-6-hydroxybenzaldehyde (Compound 20m) according to the general process of acylation (B), which is then used in the next step without additional purification of ethyl4-(2-acetoxy-5-bromophenyl)-1-(3-methoxycarbonyl)phenyl)-6-methyl-2-oxo-1,2,3,4-tetrahydropyrimidine-5-carboxylate.

¹H NMR (400 MHz, DMSO-d₆) δ 10.22 (s, 1H), 7.78 (m, 1H), 7.37 - 7.30 (m, 1H), 7.15 (d, J = 8.0 Hz, 1H), 2.34 (s, 3H).

### 42) Ethyl 4-(2-acetoxy-5-bromophenyl)-1-(3-methoxycarbonyl)phenyl)-6-methyl-2-oxo-1,2,3,4-tetrahydropyrimidine-5-carboxylate (Compound 22a)

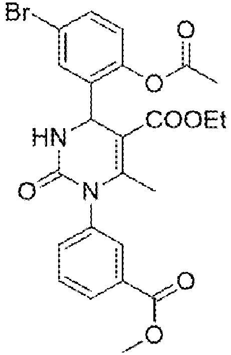

Compound 22a in yellow crystal (0.80 g, 11.4%) was prepared from 4-bromo-2-formylphenyl acetate (Compound 21a) according to the general Biginelli reaction (C).

¹H NMR (400 MHz, DMSO-d₆) δ 8.20 (d, J = 3.6 Hz, 1H), 8.00 (d, J = 7.5 Hz, 1H), 7.80 (s, 1H), 7.65 (t, J = 7.8 Hz, 1H), 7.60 - 7.53 (m, 3H), 7.21 (d, J = 8.9 Hz, 1H), 5.54 (d, J = 3.5 Hz, 1H), 4.02 - 3.96 (m, 2H), 3.88 (s, 3H), 2.33 (s, 3H), 2.07 (s, 3H), 1.05 (t, J = 7.1 Hz, 3H).

### 43) Ethyl 4-(2-acetoxy-5-(trifluoromethyl)phenyl)-1-(3-(methoxycarbonyl)phenyl)-6-methyl-2-oxo-1,2,3,4-tetrahydropyrimidine-5-carboxylate (Compound 22b)

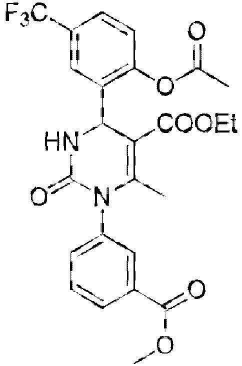

Compound 22b in white needle-like shape (0.65 g, 12.0%) was prepared from 2-formyl-4-(trifluoromethyl)phenyl acetate (Compound 21b) according to the general Biginelli reaction (C).

¹H NMR (400 MHz, DMSO-d₆) δ 8.22 (s, 1H), 8.00 (d, J = 8.0 Hz, 1H), 7.78 (s, 1H), 7.67-7.57 (m, 2H), 7.46 -7.42 (m, 2H), 7.29 - 7.25 (m, 1H), 5.55 (d, J = 8.0 Hz, 1H), 4.37 - 4.30 (m, 2H), 4.06 - 3.95 (m, 2H), 2.33 (s, 2H), 2.06 (s, 3H), 1.34 (t, J = 8.0 Hz, 3H), 1.05 (t, J = 8.0 Hz, 2H).

### 44) Ethyl 4-(2-acetoxy-5-chlorophenyl)-1-(3-(ethoxycarbonyl)phenyl)-6-methyl-2-oxo-1,2,3,4-tetrahydropyrimidine-5-carboxylate (Compound 22c)

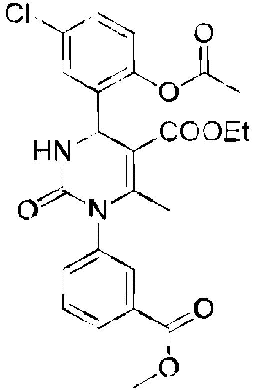

Compound 22c in yellow crystal (5.00 g, 28.7%) was prepared from 4-chloro-2-formylphenyl acetate (Compound 21c) according to the general Biginelli reaction (C).

¹H NMR (400 MHz, DMSO-d₆) δ 8.22 (d, J = 4.0 Hz, 1H), 8.00 (d, J = 8.0 Hz, 1H), 7.78 (s, 1H), 7.65 (t, J = 8.0 Hz, 1H), 7.58 (d, J = 8.0 Hz, 1H), 7.45 (dd, J = 8.0, 8.0 Hz, 2H), 7.30 - 7.25 (m, 1H), 5.55 (d, J = 4.0 Hz, 1H), 4.38 - 4.29 (m, 2H), 4.05 - 3.94 (m, 2H), 2.33 (s, 2H), 2.06 (d, J = 8.0 Hz, 3H), 1.34 (t, J = 8.0 Hz, 3H), 1.05 (t, J = 8.0 Hz, 2H).

### 45) Ethyl 4-(2-acetoxy-5-fluorophenyl)-1-(3-(methoxycarbonyl)phenyl)-6-methyl-2-oxo-1,2,3,4-tetrahydropyrimidine-5-carboxylate (Compound 22d)

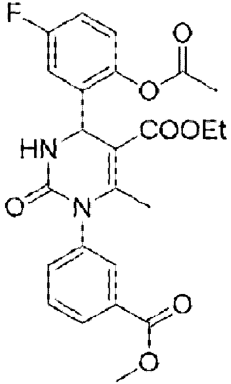

Compound 22d in yellow crystal (0.50 g, 12.2%) was prepared from 4-fluoro-2-formylphenyl acetate (Compound 21d) according to the general Biginelli reaction (C).

¹H NMR (400 MHz, DMSO-d₆) δ 8.16 (d, J = 4.0 Hz, 1H), 7.96 (d, J = 8.0 Hz, 1H), 7.79 (s, 1H), 7.60 (dd, J = 12.0, 8.0 Hz, 2H), 7.28 - 7.16 (m, 3H), 5.52 (s, 1H), 4.00 - 3.89 (m, 2H), 3.85 (s, 3H), 2.30 (s, 3H), 2.04 (s, 3H), 1.00 (t, J = 8.0 Hz, 3H).

### 46) Ethyl 4-(2-acetoxy-5-methoxyphenyl)-1-(3-(methoxycarbonyl)phenyl)-6-methyl-2-oxo-1,2,3,4-tetrahydropyrimidine-5-carboxylate (Compound 22e)

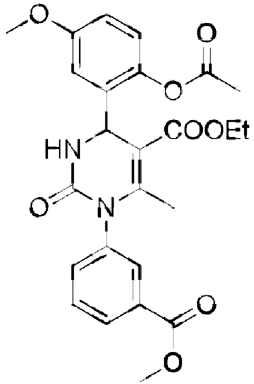

Compound 22e in yellow crystal (0.48 g, 12.0%) was prepared from 2-formyl-4-methoxyphenyl acetate (Compound 21e) according to the general Biginelli reaction (C).

¹H NMR (400 MHz, DMSO-d₆) δ 7.75 (d, J = 4.7 Hz, 1H), 7.51- 7.47 (m, 2H), 7.40 (d, J = 12 Hz, 1H), 7.26 - 7.18 (m, 6H), 6.93 (t, J = 8 Hz, 1H), 4.75 (s, 3H), 4.41 (s, 1H), 3.55 (s, 2H), 3.17 - 3.11 (m, 1H), 2.86 (s, 2H), 2.60 (dd, J = 4.0, 4.0 Hz, 1H), 2.42 (dd, J = 4.0, 4.0 Hz, 1H), 1.46 (s, 2H), 1.28 - 1.23 (m, 3H).

### 47) Ethyl 4-(2-acetoxy-5-bromophenyl)-1-(3-(methoxycarbonyl)phenyl)-6-methyl-2-oxo-1,2,3,4-tetrahydropyrimidine-5-carboxylate (Compound 22f)

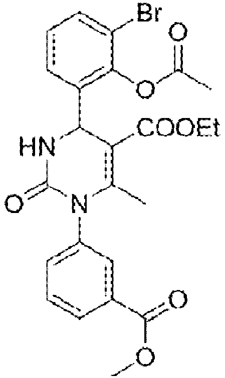

Compound 22f in yellow crystal (1.30 g, 21.5%) was prepared from 2-bromo-6-formylphenyl acetate (compound 21f) according to the general Biginelli reaction (C).

¹H NMR (400 MHz, DMSO-d₆) δ 8.06 (s, 1H), 8.01 - 7.93 (m, 2H), 7.81 (s, 1H), 7.69 (dd, J = 4.0, 4.0 Hz, 1H), 7.64 - 7.57 (m, 2H), 7.51 (d, J = 8.0 Hz, 1H), 7.31 (t, J = 8.0 Hz, 1H), 5.49 (d, J = 4.0 Hz, 1H), 3.88 (s, 3H), 2.89 (s, 2H), 2.73 (s, 2H), 2.07 (s, 3H), 1.05 (t, J = 8.0 Hz, 3H).

### 48) Ethyl 4-(2-acetoxy-3-methoxyphenyl)-1-(3-(methoxycarbonyl)phenyl)-6-methyl-2-oxo-1,2,3,4-tetrahydropyrimidine-5-carboxylate (Compound 22g)

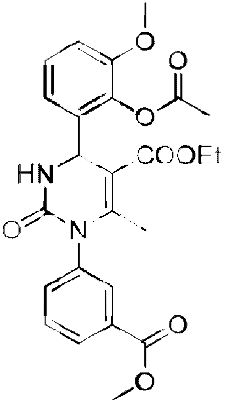

Compound 22g in yellow crystal (1.00 g, 13.0%) was prepared from 2-formyl-6-methoxyphenyl acetate (Compound 21g) according to the general Biginelli reaction (C).

¹H NMR (400 MHz, DMSO-d₆) δ 8.04 - 7.95 (m, 2H), 7.81 (s, 1H), 7.66 - 7.55 (m, 2H), 7.29 (t, J = 8.0 Hz, 1H), 7.08 (dd, J = 8.0, 8.0 Hz, 2H), 5.50 (d, J = 4.0 Hz, 1H), 3.88 (s, 2H), 3.78 (s, 3H), 2.89 (s, 2H), 2.73 (s, 2H), 2.31 (s, 3H), 2.05 (s, 2H), 1.07 (t, J = 8.0 Hz, 3H).

### 49) Ethyl 4-(2-acetoxy-4-bromophenyl)-1-(3-(methoxycarbonyl)phenyl)-6-methyl-2-oxo-1,2,3,4-tetrahydropyrimidine-5-carboxylate (Compound 22h)

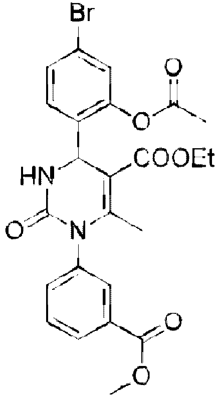

Compound 22h in yellow crystal (2.20 g, 21.5%) was prepared from 5-bromo-2-formylphenyl acetate (Compound 21h) according to the general Biginelli reaction (C).

¹H NMR (400 MHz, DMSO-d₆) δ 8.25-8.20 (d, J= 4.0, 1H), 8.01 - 7.98 (d, J= 8.0 Hz, 1H),7.8 (s, 1H) 7.69 - 7.61 (m, 1H), 7.61-7.53 (m, 3H), 7.23 - 7.17 (m, 1H), 5.56 - 5.52 (m, 1H), 4.06-3.95 (m, 3H), 4.0 - 3.85 (s, 3H), 2.33 (s, 3H), 2.0 (s, 1H), 1.120 - 1.17 (m, 1H), 1.04 (m, 3H).

### 50) Ethyl 4-(2-acetoxy-6-(trifluoromethyl)phenyl)-1-(3-(methoxycarbonyl)phenyl)-6-methyl-2-oxo-1,2,3,4-tetrahydropyrimidine-5-carboxylate (Compound 22i)

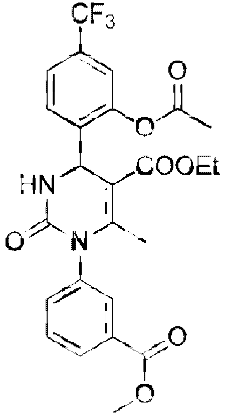

Compound 22i in yellow semi solid (3.00 g-crude) was prepared from 2-formyl-5-(trifluoromethyl)phenyl acetate (Compound 21i) according to the general Biginelli reaction (C). The residue was used in the next step without additional purification.

### 51) Ethyl 4-(2-acetoxy-4-chlorophenyl)-1-(3-(methoxycarbonyl)phenyl)-6-methyl-2-oxo-1,2,3,4-tetrahydropyrimidine-5-carboxylate (Compound 22j)

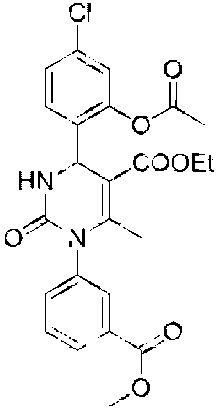

Compound 22j in brown semi solid (1.50 g-crude) was prepared from 5-chloro-2-formylphenyl acetate (Compound 21j) according to the general Biginelli reaction (C). The residue was used in the next step without additional purification.

### 52) Ethyl 4-(2-acetoxy-4-fluorophenyl)-1-(3-(methoxycarbonyl)phenyl)-6-methyl-2-oxo-1,2,3,4-tetrahydropyrimidine-5-carboxylate (Compound 22k)

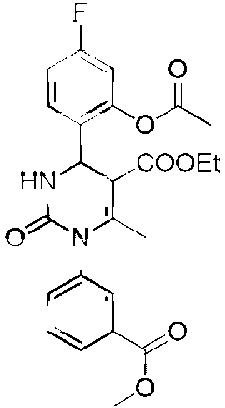

Compound 22k in yellow crystal (0.50 g, 8.00%) was prepared from 5-fluoro-2-formylphenyl acetate (Compound 21k) according to the general Biginelli reaction (C).

¹H NMR (400 MHz, DMSO-d₆) δ 8.16 (d, J = 4.0 Hz, 1H), 8.01 - 7.98 (m, 1H), 7.96 (s, 1H), 7.83 (s, 1H), 7.66 - 7.57 (m, 2H), 7.21 - 7.15 (m, 2H), 5.55 (d, J = 4.0 Hz, 1H), 3.88 (s, 3H), 3.32 (s, 2H), 2.34 (s, 3H), 2.07 (s, 3H), 1.03 (t, J = 8.0 Hz, 3H).

### 53) Ethyl 4-(2-acetoxy-4-methoxyphenyl)-1-(3-(methoxycarbonyl)phenyl)-6-methyl-2-oxo-1,2,3,4-tetrahydropyrimidine-5-carboxylate (Compound 22l)

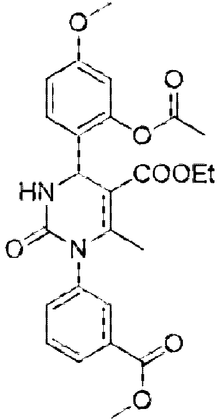

Compound 22l in yellow crystal (1.90 g, 25.0%) was prepared from 2-formyl-5-methoxyphenyl acetate (Compound 21l) according to the general Biginelli reaction (C).

¹H NMR (400 MHz, DMSO-d₆) δ 8.00 - 7.92 (m, 2H), 7.78 (s, 1H), 7.63 - 7.53 (m, 2H), 7.37 (d, J = 8.0 Hz, 1H), 6.87 (dd, J = 4.0, 4.0 Hz, 1H), 6.75 (d, J = 4.0 Hz, 1H), 5.44 (d, J = 4.0 Hz, 1H), 3.85 (s, 3H), 3.73 (s, 3H), 2.86 (s, 1H), 2.70 (s, 2H), 2.30 (s, 3H), 2.02 (s, 2H), 1.03 (t, J = 8.0 Hz, 3H).

### 54) Ethyl 4-(2-acetoxy-6-fluorophenyl)-1-(3-(methoxycarbonyl)phenyl)-6-methyl-2-oxo-1,2,3,4-tetrahydropyrimidine-5-carboxylate (Compound 22m)

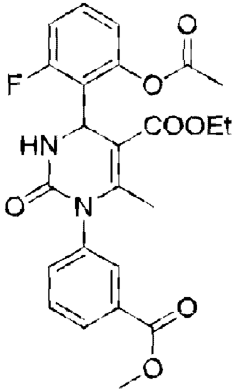

Compound 22m in yellow crystal (0.50 g, 8.00%) was prepared from 3-fluoro-2-formylphenyl acetate (compound 21m) according to the general Biginelli reaction (C).

¹H NMR (400 MHz, DMSO-d₆) δ 8.03 - 7.92 (m, 3H), 7.81 (s, 1H), 7.64 (t, J = 7.8 Hz, 1H), 7.42 - 7.33 (m, 2H), 7.20 - 7.13 (m, 1H), 7.06 (d, J = 8.0 Hz, 1H), 2.89 (s, 4H), 2.73 (s, 4H), 2.33 (s, 2H), 1.99 (s, 2H), 1.02 (t, J = 8.0 Hz, 2H).

### 55) 3-(8-Bromo-2-methyl-4-oxo-5,6-dihydro-2H-2,6-methanobenzo[g][1,3,5]oxadiazocin-3(4H)-yl)benzoic acid (Compound 24a)

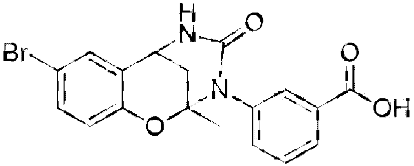

Compound 24a in purple solid (0.55 g, 42.0%) was prepared from ethyl 4-(2-acetoxy-5-bromophenyl)-1-(3-methoxycarbonyl)phenyl)-6-methyl-2-oxo-1,2,3,4-tetrahydropyrimidine-5-carboxylate (Compound 22a) according to the general process (D) to be used in the next step without additional purification.

¹H NMR (400 MHz, DMSO-d₆) δ 13.02 (s, 1H), 7.89 (d, J = 7.7 Hz, 1H), 7.69 - 7.61 (m, 2H), 7.51 (t, J = 7.8 Hz, 1H), 7.47 - 7.41 (m, 2H), 7.33 (d, J = 6.4 Hz, 1H), 6.92 (d, J = 7.6 Hz, 1H), 4.40 (d, J = 2.5 Hz, 1H), 2.59 (dd, J = 13.3, 2.8 Hz, 1H), 2.36 (d, J = 13.0 Hz, 1H), 1.37 (s, 3H).

### 56) 3-(2-Methyl-4-oxo-8-(trifluoromethyl)-5,6-dihydro-2H-2,6-methanobenzo[g][1,3,5]oxadiazocin-3(4H)-yl)benzoic acid (Compound 24b)

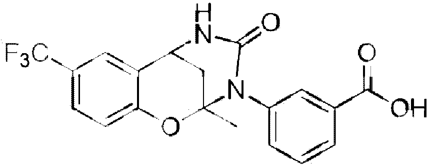

Compound 24b in reddish-brown solid (0.39 g, 9.00%) was prepared from ethyl 4-(2-acetoxy-5-(trifluoromethyl)phenyl)-1-(3-(methoxycarbonyl)phenyl)-6-methyl-2-oxo-1,2,3,4-tetrahydropyrimidine-5-carboxylate (Compound 22b) according to the general process (D) to be used in the next step without additional purification.

¹H NMR (400 MHz, DMSO-d₆)δ 12.86 (s, 1H), 7.89 (m, 3H), 7.77 (d, J = 4.0 Hz, 1H), 7.65 (s, 1H), 7.52 (t, J = 8.0 Hz, 1H), 7.36 - 7.32 (m, 1H), 7.02 (d, J = 8.0 Hz, 1H), 4.48 (s, 1H), 2.63 (d, J = 12.0 Hz, 1H), 2.39 (d, J = 12.0 Hz, 1H), 1.40 (s, 3H).

### 57) 3-(8-Chloro-2-methyl-4-oxo-5,6-dihydro-2H-2,6-methanobenzo[g][1,3,5]oxadiazocin-3(4H)-yl)benzoic acid (Compound 24c)

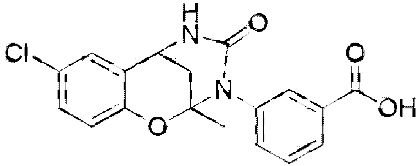

Compound 24c in white solid (2.37 g, 45.0%) was prepared from ethyl 4-(2-acetoxy-5-chlorophenyl)-1-(3-(ethoxycarbonyl)phenyl)-6-methyl-2-oxo-1,2,3,4-tetrahydropyrimidine-5-carboxylate (Compound 22c) according to the general process (D) to be used in the next step without additional purification.

¹H NMR (400 MHz, DMSO-d₆) δ 13.07 (s, 1H), 7.89 (d, J = 8.0 Hz, 1H), 7.68 (d, J = 4.0 Hz, 1H), 7.64 (s, 1H), 7.51 (t, J = 8.0 Hz, 1H), 7.37 - 7.28 (m, 3H), 6.98 (d, J = 8.0 Hz, 1H), 4.39 (d, J = 4.0 Hz, 1H), 2.62 - 2.58 (m, 4 Hz, 1H), 2.36 (d, J = 8.0 Hz, 1H), 1.37 (s, 3H).

### 58) 3-(8-Fluoro-2-methyl-4-oxo-5,6-dihydro-2H-2,6-methanobenzo[g][1,3,5]oxadiazocin-3(4H)-yl)benzoic acid (Compound 24d)

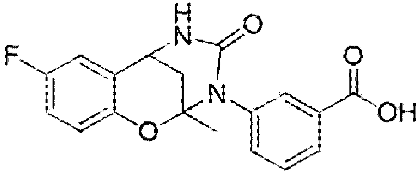

Compound 24d in ashgray solid (0.15 g, 40%) was prepared from ethyl 4-(2-acetoxy-5-fluorophenyl)-1-(3-(methoxycarbonyl)phenyl)-6-methyl-2-oxo-1,2,3,4-tetrahydropyrimidine-5-carboxylate (Compound 22d) according to the general process (D) to be used in the next step without additional purification.

¹H NMR (400 MHz, DMSO-d₆) δ 13.03 (s, 1H), 7.86 (d, J = 8.0 Hz, 1H), 7.75 - 7.56 (m, 2H), 7.48 (t, J = 8.0, 8.0 Hz, 1H), 7.31 (d, J = 7.8 Hz, 1H), 7.11 (dd, J = 8.0, 8.0 Hz, 1H), 7.04 (dd, J = 8.0, 8.0 Hz, 1H), 6.94 (dd, J = 8.0, 8.0 Hz, 1H), 4.34 (s, 1H), 2.55 (dd, J = 4.0, 4.0 Hz, 1H), 2.32 (d, J = 8.0 Hz, 1H), 1.33 (s, 3H).

### 59) 3-(8-Methoxy-2-methyl-4-oxo-5,6-dihydro-2H-2,6-methanobenzo[g][1,3,5]oxadiazocin-3(4H)-yl)benzoic acid (Compound 24e)

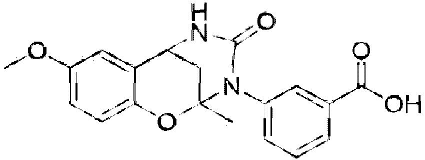

Compound 24e in white solid (0.40 g, 54.0%) was prepared from ethyl 4-(2-acetoxy-5-methoxyphenyl)-1-(3-(methoxycarbonyl)phenyl)-6-methyl-2-oxo-1,2,3,4-tetrahydropyrimidine-5-carboxylate (Compound 22e) according to the general process (D) to be used in the next step without additional purification.

¹H NMR (400 MHz, DMSO-d₆) δ 13.03 (s, 1H), 7.88 (d, J = 8.0 Hz, 1H), 7.67 - 7.59 (m, 2H), 7.50 (t, J = 8.0 Hz, 1H), 7.33 (d, J = 8.0 Hz, 1H), 6.85 (d, J = 12 Hz, 3H), 4.30 (d, J = 4.0 Hz, 1H), 3.73 (s, 3H), 2.54 (dd, J = 8.0, 8.0 Hz, 1H), 2.33 (d, J = 8.0 Hz, 1H), 1.34 (s, 3H).

### 60) 3-(10-Bromo-2-methyl-4-oxo-5,6-dihydro-2H-2,6-methanobenzo[g][1,3,5]oxadiazocin-3(4H)-yl)benzoic acid (Compound 24f)

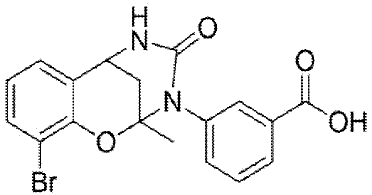

Compound 24f in purple white solid (0.50 g, 41.0%) was prepared from ethyl 4-(2-acetoxy-5-bromophenyl)-1-(3-(methoxycarbonyl)phenyl)-6-methyl-2-oxo-1,2,3,4-tetrahydropyrimidine-5-carboxylate (Compound 22f) according to the general process (D) to be used in the next step without additional purification.

¹H NMR (400 MHz, DMSO-d₆) δ 13.02 (s, 1H), 7.89 (d, J = 8.0 Hz, 1H), 7.78 (d, J = 8.0 Hz, 1H), 7.71 (s, 1H), 7.6-7.48 (m, 2H), 7.37 (d, J = 8.0 Hz,1H), 7.26 (d, 1H), 7.0-6.9 (m, 1H), 4.42-4.40 (s, 1H), 2.65-2.59 (d, J = 12.0 Hz 1H), 2.42 (d, J = 12.0 Hz 1H), 1.42 (s, 3H).

### 61) 3-(10-Methoxy-2-methyl-4-oxo-5,6-dihydro-2H-2,6-methanobenzo[g][1,3,5]oxadiazocin-3(4H)-yl)benzoic acid (Compound 24g)

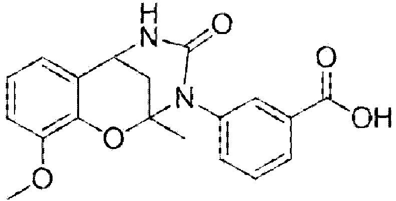

Compound 24g in white solid (0.27 g, 37.0%) was prepared from ethyl 4-(2-acetoxy-3-methoxyphenyl)-1-(3-(methoxycarbonyl)phenyl)-6-methyl-2-oxo-1,2,3,4-tetrahydropyrimidine-5-carboxylate (Compound 22g) according to the general process (D) to be used in the next step without additional purification.

¹H NMR (400 MHz, DMSO-d₆) δ 12.98 (s, 1H), 7.87 (d, J = 8.0 Hz, 1H), 7.67 (s, 2H), 7.49 (t, J = 8.0 Hz, 1H), 7.34 (d, J = 8.0 Hz, 1H), 7.00 - 6.89 (m, 2H), 6.82 (d, J = 8.0 Hz, 1H), 4.32 (d, J = 4.0 Hz, 1H), 3.81 (s, 3H), 2.55 (d, J = 8.0 Hz, 1H), 2.35 (d, J = 12.0 Hz, 1H), 1.38 (s, 3H).

### 62) 3-(9-Bromo-2-methyl-4-oxo-5,6-dihydro-2H-2,6-methanobenzo[g][1,3,5]oxadiazocin-3(4H)-yl)benzoic acid (Compound 24h)

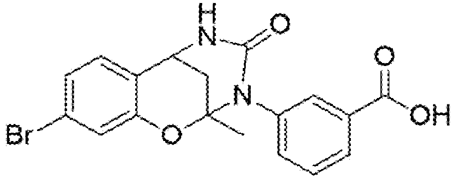

Compound 24h in white solid (2.3 g, 41.0%) was prepared from ethyl 4-(2-acetoxy-4-bromophenyl)-1-(3-(methoxycarbonyl)phenyl)-6-methyl-2-oxo-1,2,3,4-tetrahydropyrimidine-5-carboxylate (Compound 22h) according to the general process (D) to be used in the next step without additional purification.

¹H NMR (400 MHz, DMSO-d₆) δ 13.01 (s, 1H), 7.91-7.88 (d, J = 8.0 Hz, 1H), 7.80 - 7.77 (d, J = 8.0 Hz, 1H), 7.71 (s, 1H), 7.59 - 7.47 (m, 2H), 7.39 - 7.35 (m, 1H), 7.29 - 7.24 (m, 1H), 7.0-6.9 (t, J= 8.0 Hz, 1H), 4.42-4.40 (s, 1H), 2.65-2.59 (d, J = 12.0 Hz, 1H), 2.42 (d, J = 12.0 Hz, 1H), 1.41 (s, 3H).

### 63) 3-(2-Methyl-4-oxo-9-(trifluoromethyl)-5,6-dihydro-2H-2,6-methanobenzo[g][1,3,5]oxadiazocin-3(4H)-yl)benzoic acid (Compound 24i)

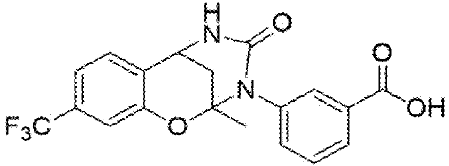

Compound 24i in white solid (0.78 g, 20.0%) was prepared from ethyl 4-(2-acetoxy-6-(trifluoromethyl)phenyl)-1-(3-(methoxycarbonyl)phenyl)-6-methyl-2-oxo-1,2,3,4-tetrahydropyrimidine-5-carboxylate (Compound 22i) according to the general process (D) to be used in the next step without additional purification.

¹H NMR (400 MHz, DMSO-d₆) δ 13.05 (s, 1H), 7.89 (d, J = 8.0 Hz, 1H), 7.82 (d, J = 4.0 Hz, 1H), 7.65 (s, 1H), 7.54 - 7.46 (m, 2H), 7.36 (t, J = 8.0 Hz, 2H), 7.27 (s, 1H), 4.48 (s, 1H), 2.65 (d, J = 12.0 Hz, 1H), 2.41 (d, J = 12.0 Hz, 1H), 1.40 (s, 3H).

### 64) 3-(9-Chloro-2-methyl-4-oxo-5,6-dihydro-2H-2,6-methanobenzo[g][1,3,5]oxadiazocin-3(4H)-yl)benzoic acid (Compound 24j)

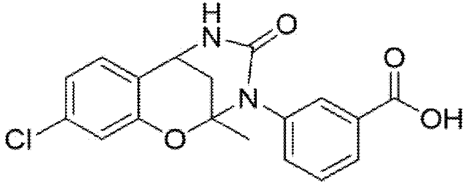

Compound 24j in white solid (0.06 g, 4.00%) was prepared from ethyl 4-(2-acetoxy-4-chlorophenyl)-1-(3-(methoxycarbonyl)phenyl)-6-methyl-2-oxo-1,2,3,4-tetrahydropyrimidine-5-carboxylate (Compound 22j) according to the general process (D) to be used in the next step without additional purification.

¹H NMR (400 MHz, DMSO-d₆) δ 13.06 (s, 1H), 7.92 - 7.86 (m, 1H), 7.74 (dd, J = 8.0, 8.0 Hz, 1H), 7.65 (d, J = 8.0 Hz, 1H), 7.61 (dd, J = 8.0, 8.0 Hz, 1H), 7.55 - 7.48 (m, 1H), 7.35 (d, J = 8.0 Hz, 1H), 7.27 (d, J = 8.0 Hz, 1H), 7.08 - 7.02 (m, 1H), 4.39 (s, 1H), 2.59 (d, J = 12.0 Hz, 1H), 2.36 (d, J = 12.0 Hz, 1H), 1.37 (s, 2H), 1.24 (s, 1H).

### 65) 3-(9-Fluoro-2-methyl-4-oxo-5,6-dihydro-2H-2,6-methanobenzo[g][1,3,5]oxadiazocin-3(4H)-yl)benzoic acid (Compound 24k)

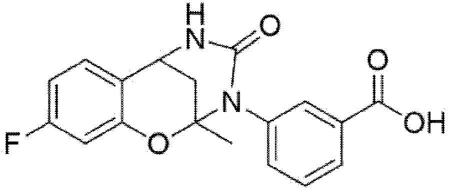

Compound 24k in white powder (0.20 g, 69.0%) was prepared from ethyl 4-(2-acetoxy-4-fluorophenyl)-1-(3-(methoxycarbonyl)phenyl)-6-methyl-2-oxo-1,2,3,4-tetrahydropyrimidine-5-carboxylate (Compound 22k) according to the general process (D) to be used in the next step without additional purification.

¹H NMR (400 MHz, DMSO-d₆) δ 13.04 (s, 1H), 7.89 (d, J = 7.8 Hz, 1H), 7.72 - 7.64 (m, 2H), 7.51 (t, J = 8.0 Hz, 1H), 7.35 (d, J = 8.0 Hz, 1H), 7.28 (dd, J = 8.0, 8.0 Hz, 1H), 6.85 - 6.81 (m, 2H), 4.38 (d, J = 8.0 Hz, 1H), 2.59 (dd, J = 8.0, 8.0 Hz, 1H), 2.36 (d, J = 12.0 Hz, 1H), 1.37 (s, 3H).

### 66) 3-(9-Methoxy-2-methyl-4-oxo-5,6-dihydro-2H-2,6-methanobenzo[g][1,3,5]oxadiazocin-3(4H)-yl)benzoic acid (Compound 24l)

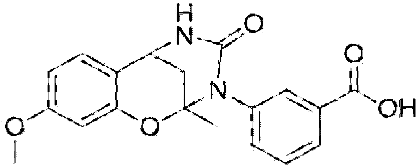

Compound 24l in white powder (0.28 g, 15.0 %) was prepared from ethyl 4-(2-acetoxy-4-methoxyphenyl)-1-(3-(methoxycarbonyl)phenyl)-6-methyl-2-oxo-1,2,3,4-tetrahydropyrimidine-5-carboxylate (Compound 22l) according to the general process (D) to be used in the next step without additional purification.

¹H NMR (400 MHz, DMSO-d₆) δ 12.99 (s, 1H), 7.86 (d, J = 8.0 Hz, 1H), 7.70 - 7.56 (m, 2H), 7.53 - 7.41 (m, 1H), 7.34 (d, J = 8.0 Hz, 1H), 7.12 (d, J = 8.0 Hz, 1H), 6.55 (d, J = 8.0 Hz, 1H), 6.47 (s, 1H), 4.27 (s, 1H), 3.72 (s, 3H), 2.52 (d, J = 12.0 Hz, 1H), 2.29 (d, J = 12.0 Hz, 1H), 1.33 (s, 3H).

### 67) 3-(7-Fluoro-2-methyl-4-oxo-5,6-dihydro-2H-2,6-methanobenzo[g][1,3,5]oxadiazocin-3(4H)-yl)benzoic acid (Compound 24m)

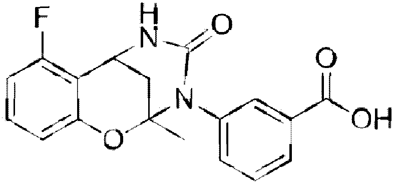

Compound 24m in white powder (1.00 g, 45.0 %) was prepared from ethyl 4-(2-acetoxy-6-fluorophenyl)-1-(3-(methoxycarbonyl)phenyl)-6-methyl-2-oxo-1,2,3,4-tetrahydropyrimidine-5-carboxylate (Compound 22m) according to the general process (D) to be used in the next step without additional purification.

¹H NMR (400 MHz, DMSO-d₆) δ 13.02 (s, 1H), 7.99 (d, J = 8.0 Hz, 1H), 7.89 (d, J = 8.0 Hz, 1H), 7.66 (s, 1H), 7.51 (t, J = 8.0 Hz, 1H), 7.36 - 7.28 (m, 2H), 6.87 - 6.79 (m, 2H), 4.65 (d, J = 8.0 Hz, 1H), 2.59 (d, J = 8.0 Hz, 1H), 2.33 (d, J = 12.0 Hz, 1H), 1.38 (s, 3H).

### 68) 8-Bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a)

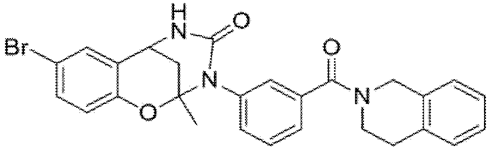

Compound 25a in brown crystal (1.80 g, 66.5%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.66 (d, J = 3.6 Hz, 1H), 7.48 (t, J = 7.8 Hz, 1H), 7.42 (bs, 3H), 7.19 (bs, 5H), 6.90 (bs, 2H), 4.76 (bs, 1H), 4.65 - 4.53 (m, 1H), 4.38 (s, 1H), 3.62 (d, J = 4.3 Hz, 2H), 2.85 (s, 2H), 2.57 (d, J = 11.6 Hz, 1H), 2.35 (d, J = 13.7 Hz, 1H), 1.41 (s, 3H); Purity ≥99.0% (RP-HPLC, Rₜ = 16.0 min).

### 69) 2-Methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b)

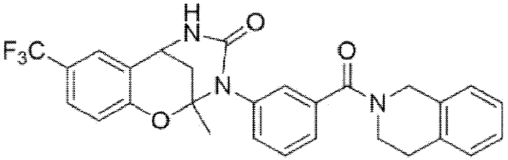

Compound 25b in off-white powder (0.05 g, 31.5%) was prepared from 3-(2-methyl-4-oxo-8-(trifluoromethyl)-5,6-dihydro-2H-2,6-methanobenzo[g][1,3,5]oxadiazocin-3(4H)-yl)benzoic acid (Compound 24b) according to the general process of acid-amine coupling (E).

¹H NMR (400 MHz, DMSO-d₆) δ 7.71 (d, J = 8.0 Hz, 1H), 7.49 (d, J = 8.0 Hz, 1H), 7.40 (t, J = 12.0 Hz, 3H), 7.19 (s, 7H), 4.71 (s, 2H), 4.43 (s, 1H), 3.72 (s, 2H), 2.87 (d, J = 8.0 Hz, 2H), 2.59 (s, 1H), 2.40 (d, J = 12.3 Hz, 1H), 1.44 (s, 3H); Purity ≥97.4% (RP-HPLC, Rₜ = 15.4 min).

### 70) 8-Chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25c)

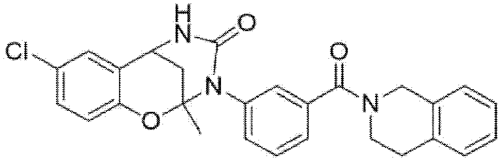

Compound 25c in purple solid (0.02 g, 21.8%) was prepared from 3-(8-chloro-2-methyl-4-oxo-5,6-dihydro-2H-2,6-methanobenzo[g][1,3,5]oxadiazocin-3(4H)-yl)benzoic acid (Compound 24c) according to the general process of acid-amine coupling (E).

¹H NMR (400 MHz, DMSO-d₆) δ 7.66 (d, J = 4.0 Hz, 1H), 7.48 (t, J = 8.0 Hz, 1H), 7.41 (d, J = 8.0 Hz, 1H), 7.32 - 7.11 (m, 7H), 6.95 (s, 2H), 4.76 (s, 2H), 4.39 (s, 1H), 3.66 - 3.50 (m, 2H), 2.85 (s, 2H), 2.57 (d, J = 8.0 Hz, 1H), 2.35 (d, J = 11.7 Hz, 1H), 1.41 (s, 3H); purity ≥97.3% (RP-HPLC, Rₜ = 15.5 min).

### 71) 8-Fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25d)

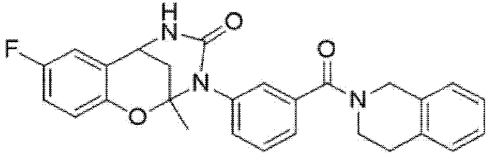

Compound 25d in off-white powder (0.01 g, 16.0%) was prepared from 3-(8-fluoro-2-methyl-4-oxo-5,6-dihydro-2H-2,6-methanobenzo[g][1,3,5]oxadiazocin-3(4H)-yl)benzoic acid (Compound 24d) according to the general process of acid-amine coupling (E).

¹H NMR (400 MHz, DMSO-d₆) δ 7.62 (s, 1H), 7.44 (d, J = 7.8 Hz, 1H), 7.39 (d, J = 4.0 Hz, 1H), 7.27 - 6.98 (m, 7H), 6.92 (s, 2H), 4.73 (s, 1H), 4.33 (s, 1H), 3.54 (s, 2H), 2.83 (s, 3H), 2.53 (d, J = 12.0 Hz, 1H), 2.32 (d, J = 12.0 Hz, 1H), 1.38 (s, 3H); Purity ≥99.4% (RP-HPLC, Rₜ = 14.5 min).

### 72) 8-Methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e)

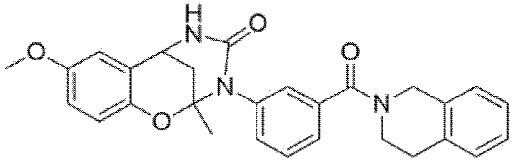

Compound 25e in off-white powder (0.02 g, 8.00%) was prepared from 3-(8-methoxy-2-methyl-4-oxo-5,6-dihydro-2H-2,6-methanobenzo[g][1,3,5]oxadiazocin-3(4H)-yl)benzoic acid (Compound 24e) according to the general process of acid-amine coupling (E).

¹H NMR (400 MHz, DMSO-d₆) δ 7.66 (d, J = 4.0 Hz, 1H), 7.47 (t, J = 8.0 Hz, 1H), 7.39 (d, J = 8.0 Hz, 1H), 7.20 (s, 6H), 6.91 (d, J = 8.0 Hz, 2H), 6.84 - 6.80 (m, 1H), 4.75 (s, 2H), 4.31 (d, J = 4.0 Hz, 1H), 3.56 (s, 3H), 2.89 (s, 2H), 2.53 (d, J = 12.0 Hz, 1H), 2.35 (d, J = 12.0 Hz, 1H), 2.08 (s, 2H), 1.42 (s, 3H); Purity ≥99.8% (RP-HPLC, Rₜ = 14.0 min).

### 73) 10-Bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f)

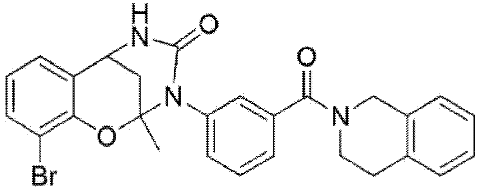

Compound 25f in off-white powder (1.20 g, 60.5%) was prepared from 3-(10-bromo-2-methyl-4-oxo-5,6-dihydro-2H-2,6-methanobenzo[g][1,3,5]oxadiazocin-3(4H)-yl)benzoic acid (Compound 24f) according to the general process of acid-amine coupling (E).

¹H NMR (400 MHz, DMSO-d₆) δ 7.78-7.73 (m, 1H), 7.54-7.45 (m, 2H), 7.43-7.37( m, 1H) , 7.28-7.10 (m, 6H), 6.96-6.90 (t, J= 8.0Hz 1H), 5.75 (s, 1H) 4.85-4.56 (m, 2H), 3.69-3.51 (m, 2H), 2.85 (s, 2H), 2.49 (d, J=8.0 Hz, 1H), 2.46-2.34 (d, J = 8.0 Hz, 1H), 1.47-1.44 (s, 1H), 1.28-1.22 (m, 3H); Purity ≥99.3% (RP-HPLC, Rₜ = 15.0 min).

### 74) 10-Methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25g)

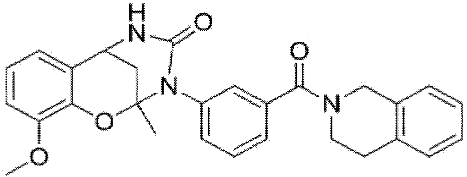

Compound 25g in off-white powder (0.12 g, 80.0%) was prepared from 3-(10-methoxy-2-methyl-4-oxo-5,6-dihydro-2H-2,6-methanobenzo[g][1,3,5]oxadiazocin-3(4H)-yl)benzoic acid (Compound 24g) according to the general process of acid-amine coupling (E).

¹H NMR (400 MHz, DMSO-d₆) δ 7.66 (d, J = 4.0 Hz, 1H), 7.47 (t, J = 8.0 Hz, 1H), 7.39 (d, J = 8.0 Hz, 1H), 7.20 (s, 6H), 6.90 (s, 2H), 6.85 - 6.79 (m, 1H), 4.75 (s, 1H), 4.31 (s, 1H), 3.56 (s, 3H), 2.89 (s, 3H), 2.55 (s, 1H), 2.35 (d, J = 12.0 Hz, 1H), 2.08 (s, 2H), 1.42 (s, 3H); Purity ≥97.5% (RP-HPLC, Rₜ = 12.3 min).

### 75) 9-Bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h)

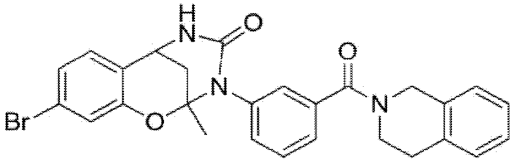

Compound 25h in off-white powder (0.80 g, 58.5%) was prepared from 3-(9-bromo-2-methyl-4-oxo-5,6-dihydro-2H-2,6-methanobenzo[g][1,3,5]oxadiazocin-3(4H)-yl)benzoic acid (Compound 24h) according to the general process of acid-amine coupling (E).

¹H NMR (400 MHz, DMSO-d₆) δ 7.75-7.66 (d, J = 8.0 Hz, 1H), 7.55-7.46 (t, J = 8.0 Hz, 1H), 7.44-7.38 (d, J = 8.0 Hz, 1H), 7.25-6.9 (m, 9H), 4.76 (s, 2H), 4.35 (s, 1H), 3.64( s, 2H), 2.85 (d, J = 4.0 Hz, 2H), 2.57 (d, J = 12.0 Hz, 1H), 2.36 (d, 12.0 Hz, 1H), 1.51(s, 3H); HRMS (ESI): [M+H]⁺ C₂₇H₂₄BrN₃O₃ calcd 518.1078, found 518.1082; Purity ≥98.5% (RP-HPLC, Rₜ = 16.1 min).

### 76) 2-Methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one (Compound 25i)

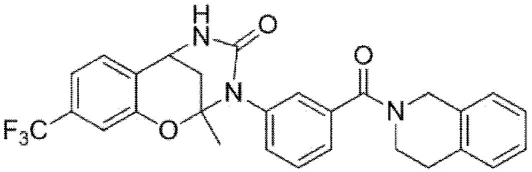

Compound 25i in off-white powder (0.06 g, 32.7%,) was prepared from 3-(2-methyl-4-oxo-9-(trifluoromethyl)-5,6-dihydro-2H-2,6-methanobenzo[g][1,3,5]oxadiazocin-3(4H)-yl)benzoic acid (Compound 24i) according to the general process of acid-amine coupling (E).

¹H NMR (400 MHz, DMSO-d₆) δ 7.79 (d, J = 8.0 Hz, 1H), 7.53 - 7.38 (m, 4H), 7.34 (d, J = 8.0 Hz, 1H), 7.21 (d, J = 8.0 Hz, 6H), 4.76 (s, 2H), 4.48 (s, 1H), 3.57 (s, 2H), 2.87 (s, 2H), 2.61 (s, 1H), 2.39 (s, 1H), 1.44 (s, 3H); Purity ≥98.6% (RP-HPLC, Rₜ = 16.6 min).

### 77) 9-Chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25j)

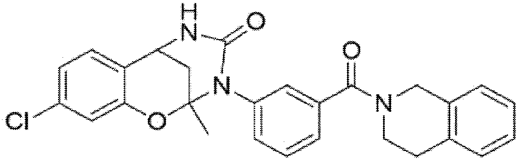

Compound 25j in off-white powder (0.04 g, 21.0%) was prepared from 3-(9-chloro-2-methyl-4-oxo-5,6-dihydro-2H-2,6-methanobenzo[g][1,3,5]oxadiazocin-3(4H)-yl)benzoic acid (Compound 24j) according to the general process of acid-amine coupling (E).

¹H NMR (400 MHz, DMSO-d₆) δ 7.73 - 7.71 (m, 1H), 7.50 (t, J = 8.0 Hz, 1H), 7.44 - 7.40 (m, 1H), 7.28 - 7.15 (m, 7H), 7.04 (d, J = 8.0 Hz, 2H), 4.76 (s, 1H), 4.38 (s, 1H), 3.58 (s, 2H), 2.87 (s, 3H), 2.58 (d, J = 12.0 Hz, 1H), 2.35 (d, J = 12.0 Hz, 1H), 1.42 (s, 3H); Purity ≥97.0% (RP-HPLC, Rₜ = 18.5 min).

### 78) 9-Fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25k)

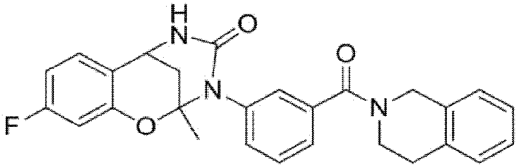

Compound 25k in off-white powder (0.23 g, 57.0%) was prepared from 3-(9-fluoro-2-methyl-4-oxo-5,6-dihydro-2H-2,6-methanobenzo[g][1,3,5]oxadiazocin-3(4H)-yl)benzoic acid (Compound 24k) according to the general process of acid-amine coupling (E).

¹H NMR (400 MHz, DMSO-d₆) δ 7.68 (d, J = 8.0 Hz, 1H), 7.49 (t, J = 8.0 Hz, 1H), 7.41 (d, J = 8.0 Hz, 1H), 7.29 - 7.14 (m, 7H), 6.86 - 6.73 (m, 2H), 4.76 (s, 1H), 4.37 (d, J = 4.0 Hz, 1H), 3.58 (s, 2H), 2.86 (s, 3H), 2.56 (d, J = 12.0 Hz, 1H), 2.35 (d, J = 12.0 Hz, 1H), 1.42 (s, 3H); Purity ≥99.6% (RP-HPLC, Rₜ = 14.8 min).

### 79) 9-Methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25l)

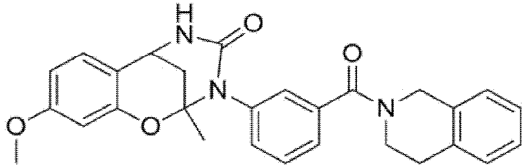

Compound 25l in off-white powder (0.040 g, 21.0%) was prepared from 3-(9-methoxy-2-methyl-4-oxo-5,6-dihydro-2H-2,6-methanobenzo[g][1,3,5]oxadiazocin-3(4H)-yl)benzoic acid (Compound 24l) according to the general process of acid-amine coupling (E).

¹H NMR (400 MHz, DMSO-d₆) δ 7.60 (d, J = 4 Hz, 1H), 7.46 (t, J = 8.0 Hz, 1H), 7.39 (s, 1H), 7.26 - 7.08 (m, 6H), 6.53 (dd, J = 4.0, 2.0 Hz, 1H), 6.42 (s, 1H), 4.73 (s, 3H), 4.25 (s, 1H), 3.65 (s, 3H), 3.54 (s, 1H), 2.83 (s, 2H), 2.51 (s, 1H), 2.28 (d, J = 12.0 Hz, 1H), 1.37 (s, 3H), 1.06 - 0.98 (m, 1H); Purity ≥99.1% (RP-HPLC, Rt = 9.7 min).

### 80) 7-Fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25m)

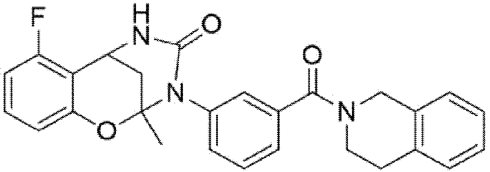

Compound 25m in off-white powder (0.20 g, 50.0%) was prepared from 3-(7-fluoro-2-methyl-4-oxo-5,6-dihydro-2H-2,6-methanobenzo[g][1,3,5]oxadiazocin-3(4H)-yl)benzoic acid (Compound 24m) according to the general process of acid-amine coupling (E).

¹H NMR (400 MHz, DMSO-d₆) δ 7.69 (d, J = 4.0 Hz, 1H), 7.49 (t, J = 8.0 Hz, 1H), 7.41 (d, J = 8.0 Hz, 1H), 7.30 - 7.07 (m, 7H), 6.84 - 6.80 (m, 2H), 4.75 (s, 1H), 4.37 (s, 1H), 3.57 (s, 2H), 2.86 (s, 2H), 2.57 (d, J = 8.0 Hz, 1H), 2.35 (d, J = 12.0, 1H), 1.42 (s, 3H), 1.24 (d, J = 8 Hz, 1H); Purity ≥95.8% (RP-HPLC, Rₜ = 14.5 min). 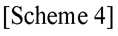 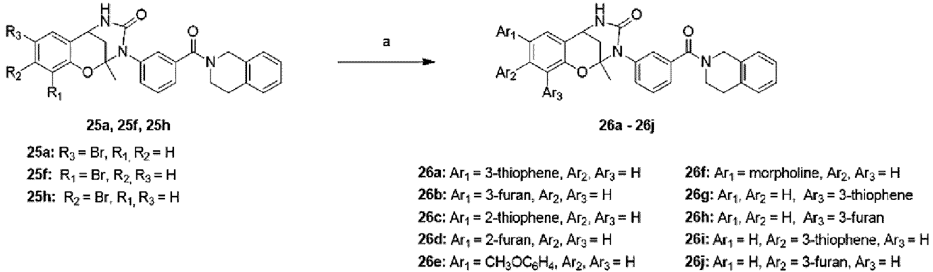
① For Compounds 26a-e and i-l, respectively, Boronic acid and Xphos-Pd G2, CS₂CO₃, DMF:H₂O (10:1) mixture, 80°C, 3 hours;
② for Compound 26f, Xphos-Pd G2, Kt-tBuOH, dioxane, 100°C, 12 hours;
③ for Compounds 26g-h, respectively, Xphos, CS₂CO₃, pd(dba)₂, dioxane, 100°C, 12 hours.

In order to introduce derivatives of various heterocyclic or pyridine groups at the Br positions of R₁~R₃, a total of 12 types of Compounds 26a-26l were synthesized through Suzuki coupling and Buchwald Hartwig coupling.

### 81) 2-Methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a)

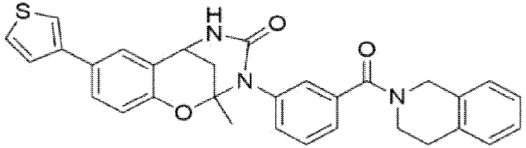

Compound 26a in off-white solid (0.04 g, 40.0%) was prepared from 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) according to the general process of Suzuki couplings F (Method A).

¹H NMR (400 MHz, DMSO-d₆) δ 7.70 (dd, J = 2.8, 1.2 Hz, 1H), 7.67 - 7.62 (m, 2H), 7.57 (s, 2H), 7.48 (dd, J = 14.0, 6.3 Hz, 2H), 7.41 (d, J = 7.3 Hz, 1H), 7.21 (d, J = 13.4 Hz, 6H), 6.92 (s, 1H), 4.76 (s, 2H), 4.40 (s, 1H), 3.58 (s, 2H), 2.86 (s, 2H), 2.59 (dd, J = 13.5, 2.6 Hz, 1H), 2.40 (d, J = 14.1 Hz, 1H), 1.43 (s, 3H); ¹³C NMR (100 MHz, CD₃OD) δ 156.68, 150.03, 141.43, 128.92, 128.41, 127.34, 125.83, 125.56, 118.87, 117.09, 85.59, 44.33, 33.91, 25.76; LCMS m/z: calcd for C₃₁H₂₈N₃O₃S [M + H]⁺ 522.18, found 522.15; Purity ≥98.6% (RP-HPLC, Rₜ = 17.6 min).

### 82) 8-(Furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b)

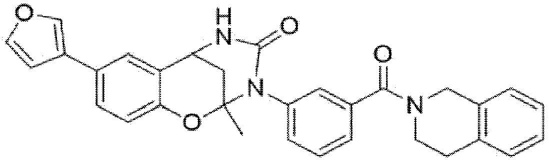

Compound 26b in off-white solid (0.04 g, 48.9%) was prepared from 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) according to the general process of Suzuki couplings F (Method A).

¹H NMR (400 MHz, DMSO-d₆) δ 8.04 (s, 1H), 7.73 (t, J = 1.6 Hz, 1H), 7.67 (d, J = 3.8 Hz, 1H), 7.53 - 7.36 (m, 4H), 7.21 (d, J = 9.9 Hz, 6H), 6.91 (s, 1H), 6.85 (s, 1H), 4.75 (s, 2H), 4.37 (s, 1H), 3.58 (s, 2H), 2.86 (s, 2H), 2.58 (d, J = 10.8 Hz, 1H), 2.38 (d, J = 12.3 Hz, 1H), 1.42 (s, 3H); ¹³C NMR (100 MHz, CD₃OD) δ 156.66, 149.89, 143.55, 138.20, 137.92, 136.17, 128.89, 128.32, 126.84, 126.17, 125.94, 125.77, 125.28, 117.11, 108.17, 85.52, 44.31, 33.94, 25.82; LCMS m/z: calcd for C₃₁H₂₈N₃O₄ [M + H]⁺ 506.20, found 506.15; Purity ≥98.3% (RP-HPLC, Rₜ = 16.3 min).

### 83) 2-Methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c)

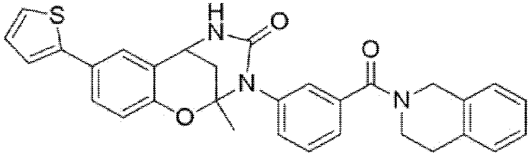

Compound 26c in off-white solid (0.02 g, 25.4%) was prepared from 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) according to the general process of Suzuki couplings F (Method A),

¹H NMR (400 MHz, DMSO-d₆) δ 7.71 (d, J = 4.0 Hz, 1H), 7.50 - 7.46 (m, 3H), 7.42 (s, 1H), 7.37 (d, J = 4.0 Hz, 1H), 7.14 (dd, J = 8.0, 8.0 Hz, 7H), 6.95 (s, 2H), 4.42 (s, 1H), 4.42 (s, 2H), 3.57 (s, 2H), 3.17 (d, J = 8.0 Hz, 1H), 2.73 (s, 1H), 2.59 (d, J = 12.0 Hz, 1H), 2.39 (d, J = 12.0 Hz, 1H), 1.42 (d, J = 8.0 Hz, 3H); Purity ≥93.4% (RP-HPLC, Rₜ = 18.0 min).

### 84) (8-(Furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d)

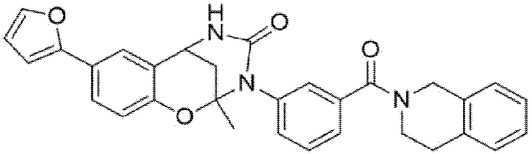

Compound 26d in off-white solid (0.02 g, 25.8%) was prepared from 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) according to the general process of Suzuki couplings F (Method A),

¹H NMR (400 MHz, DMSO-d₆) δ 7.70 (d, J = 8.0 Hz, 2H), 7.57 (s, 2H), 7.46 (t, J = 8.0 Hz, 1H), 7.39 (s, 1H), 7.16 (s, 6H), 6.93 (s, 1H), 6.75 (d, J = 4.0 Hz, 1H), 6.57 - 6.52 (m, 1H), 4.73 (s, 2H), 4.38 (s, 2H), 3.54 (s, 2H), 2.86 (s, 1H), 2.56 (d, J = 12.0 Hz, 1H), 2.36 (d, J = 12.0 Hz, 1H), 1.39 (d, J = 8.0 Hz, 3H); Purity ≥94.7% (RP-HPLC, Rₜ = 16.9 min).

### 85) 8-(4-Methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e)

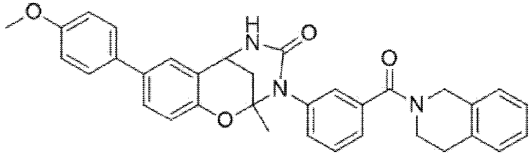

Compound 26e in gray powder (0.02 g, 27.8%) was prepared from 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) according to the general process of Suzuki couplings F (Method A),

¹H NMR (400 MHz, DMSO-d₆) δ 7.67 (d, J = 4.0 Hz, 1H), 7.55 (d, J = 8.0 Hz, 2H), 7.49 (t, J = 8.0 Hz, 3H), 7.43 (s, 1H), 7.19 (s, 6H), 7.03 (d, J = 8.0 Hz, 2H), 6.97 (s, 1H), 4.76 (s, 2H), 4.42 (s, 1H), 3.79 (s, 3H), 3.58 (s, 2H), 2.86 (s, 2H), 2.59 (d, J = 12.0 Hz, 1H), 2.40 (d, J = 12.0 Hz, 1H), 1.42 (d, J = 8.0 Hz, 3H); Purity ≥94.8% (RP-HPLC, Rₜ = 18.4 min).

### 86) 2-Methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26f)

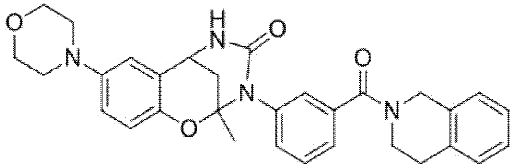

Xphos-Pd G2 (0.017 g, 0.02 mmol) was added to a degassing solution in which 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25a) (0.1 g, 0.19 mmol), morpholine (0.025 g, 0.28 mmol), and sodium t-butoxide (0.055 g, 0.57 mmol) were dissolved in dioxane (20 mL). The resulting mixture was heated in a sealed tube at 100°C for 10 hours. The reaction mixture was cooled to room temperature and diluted with water (20 mL), and the aqueous phase was extracted with ethyl acetate (3 x 30 mL). The combined organic layers were dried with anhydrous sodium sulfate, filtered, and evaporated under reduced pressure. Residues were purified by silica gel column chromatography (5-10% MeOH dissolved in DCM) to prepare Compound 26f in off-white solid (0.03 g, 35.0%).

¹H NMR (400 MHz, DMSO-d₆) δ 7.67 (d, J = 4.0 Hz, 1H), 7.55 (d, J = 8.0 Hz, 2H), 7.49 (t, J = 8.0 Hz, 3H), 7.43 (s, 1H), 7.19 (s, 7H), 7.03 (d, J = 8.7 Hz, 2H), 6.97 (s, 1H), 4.76 (s, 2H), 4.42 (s, 1H), 3.79 (s, 3H), 3.58 (s, 2H), 2.86 (s, 2H), 2.59 (d, J = 12.0 Hz, 1H), 2.40 (d, J = 12.0 Hz, 1H), 1.42 (d, J = 8.0 Hz, 3H); Purity ≥99.4% (RP-HPLC, Rₜ = 9.78 min).

### 87) 2-Methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one (Compound 26g)

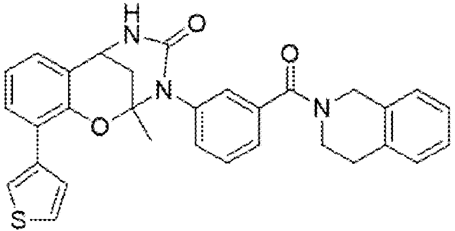

Compound 26g in off-white solid (0.02 g, 21.6%) was prepared from 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f) according to the general process of Suzuki couplings F (Method A),

¹H NMR (400 MHz, DMSO-d₆) δ 7.62 (s, 1H), 7.53 - 7.33 (m, 4H), 7.22 (d, J = 8.0 Hz, 5H), 7.16 - 6.90 (m, 5H), 4.84 (s, 2H), 4.57 (d, J = 8.0 Hz, 1H), 4.36 (s, 1H), 3.83 (d, J = 8.0 Hz, 1H), 3.48 (s, 1H), 2.89 (s, 1H), 2.64 (dd, J = 8.0, 8.0 Hz, 1H), 2.49 (d, J = 12.0 Hz, 1H), 1.50 (s, 3H); HRMS (ESI): [M+H]⁺ C₃₁H₂₇N₃O₃S calcd 522.6430, found 522.1892; Purity ≥95.4% (RP-HPLC, Rₜ = 16.7 min).

### 88) 10-(Furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26h)

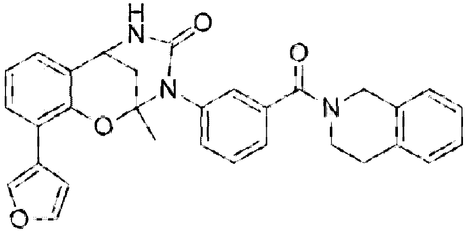

Compound 26h in off-white solid (0.045 g, 48.9%) was prepared from 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25f) according to the general process of Suzuki couplings F (Method A).

¹H NMR (400 MHz, CD₃OD) δ 7.87 (d, J = 8.0 Hz 1H), 7.48 (d, J = 8.0 Hz, 4H), 7.32 - 6.97 (m, 8H), 6.85 (s, 1H), 6.54 (s, 1H), 4.53 (d, J = 12.0 Hz, 3H), 3.84 (d, J = 8.0 Hz, 1H), 3.55 (s, 1H), 3.34 (s, 1H), 2.89 (s, 1H), 2.67 (d, J = 12.0 Hz, 1H), 2.50 (d, J = 12.0 Hz, 1H), 1.56 (s, 3H); Purity ≥97.1% (RP-HPLC, Rₜ = 15.4 min).

### 89) 2-Methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one (Compound 26i)

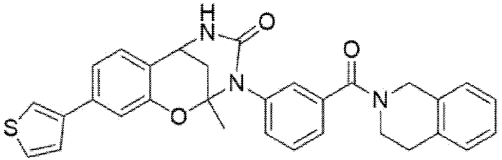

Compound 26i in off-white solid (0.08 g, 41.6%) was prepared from 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h) according to the general process of Suzuki couplings F (Method A),

¹H NMR (400 MHz, DMSO-d₆) δ 7.83 (s, 1H), 7.69 (d, J = 4.0 Hz, 1H), 7.63 - 7.59 (m, 1H), 7.50 (dd, J = 8.0, 8.0 Hz, 2H), 7.42 (s, 1H), 7.33 (d, J = 8.0 Hz, 1H), 7.29 - 7.21 (m, 4H), 7.18 (d, J = 8.0 Hz, 4H), 4.75 (s, 2H), 4.36 (s, 1H), 3.57 (s, 2H), 2.87 (s, 2H), 2.58 (d, J = 12.0 Hz, 1H), 2.38 (d, J = 12.0 Hz, 1H), 1.44 (s, 3H); HRMS (ESI): [M+H]⁺ C₃₁H₂₇N₃O₃S calcd 522.1851, found 522.1857; Purity ≥98.5% (RP-HPLC, Rt = 17.1 min).

### 90) 9-(Furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26j)

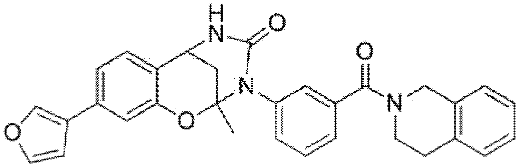

Compound 26j in off-white solid (0.03 g, 38.9%) was prepared from 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25h) according to the general process of Suzuki couplings F (Method A),

¹H NMR (400 MHz, DMSO-d₆) δ 8.15 (s, 1H), 7.71 (s, 1H), 7.68 (d, J = 8.0 Hz, 1H), 7.49 (t, J = 8.0 Hz, 1H), 7.42 (s, 1H), 7.20 (d, J = 25.1 Hz, 9H), 6.92 (s, 1H), 4.73 (s, 2H), 4.35 (d, J = 8.0 Hz, 1H), 3.57 (s, 2H), 2.86 (s, 2H), 2.56 (s, 1H), 2.36 (d, J = 12.0 Hz, 1H), 1.43 (s, 3H); HRMS (ESI): [M+H]⁺ C₃₁H₂₇N₃O₄ calcd 506.2080, found 506.2073; Purity ≥98.1% (RP-HPLC, Rₜ = 15.8 min). 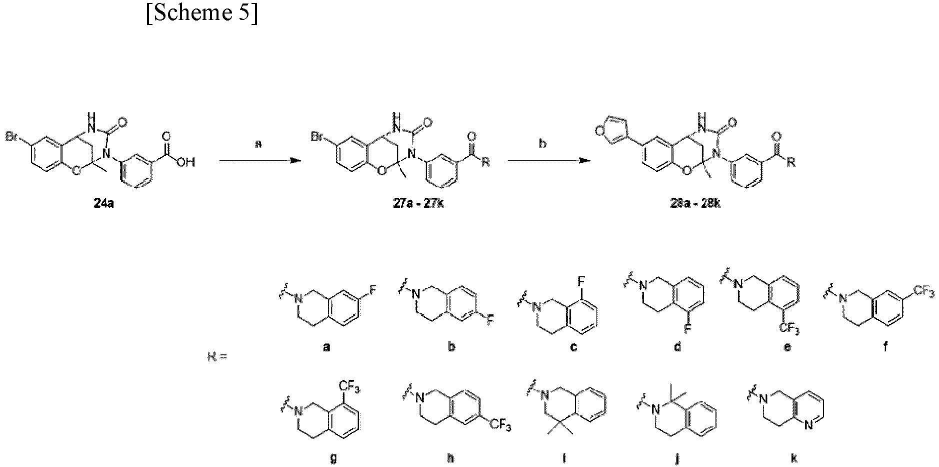
(a) Amine, HATU, DIPEA, DMF, room temperature for 12 hours;
(b) 3-Furanboronic acid, Xphos-Pd G2, CS₂CO₃, DMF:H₂O (10:1) mixture, 80°C, 3 hours.

Amide coupling was performed using each amine and HATU to introduce various structures such as F, CF₃, and dimethyl at the tetrahydroisoquinoline position of Compound 25a. Thereby, 11 types of Compounds 27a-27k were synthesized. Subsequently, 11 types of Compounds 28a-28k were synthesized through Suzuki coupling using 3-furanboronic acid and Xphos-PD G2 catalyst to substitute Br with 3-furan.

### 91) 8-Bromo-3-(3-(7-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27a)

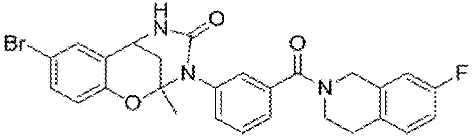

Compound 27a in off-white powder (0.35 g, 51.7%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.66 (d, J = 3.9 Hz, 1H), 7.49 (t, J = 7.8 Hz, 1H), 7.42 (s, 3H), 7.15 (ddd, J = 62.4, 15.7, 8.2 Hz, 5H), 6.89 (s, 1H), 4.76 (bs, 1H), 4.67 - 4.51 (bs, 1H), 4.39 (s, 1H), 3.67 - 3.47 (m, 2H), 2.86 (bs, 2H), 2.57 (d, J = 13.4 Hz, 1H), 2.35 (d, J = 12.1 Hz, 1H), 1.41 (s, 3H); Purity ≥99.8% (RP-HPLC, Rₜ = 16.4 min).

### 92) 8-Bromo-3-(3-(6-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27b)

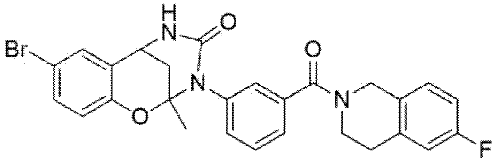

Compound 27b of light brown crystal (0.14 g, 52.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.95 (s, 1H), 7.65 (d, J = 4.0 Hz, 1H), 7.48 (t, J = 8.0 Hz, 1H), 7.42 (s, 3H), 7.17 (s, 2H), 7.06 (d, J = 8.0 Hz, 2H), 6.89 (s, 1H), 4.73 (s, 2H), 4.39 (d, J = 4.0 Hz, 1H), 3.57 (s, 2H), 2.57 (d, J = 12.0 Hz, 1H), 2.35 (d, J = 12.0 Hz, 1H), 1.41 (s, 3H), 1.30 - 1.19 (m, 2H); Purity ≥95.6% (RP-HPLC, Rₜ = 15.7 min).

### 93) 8-Bromo-3-(3-(8-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27c)

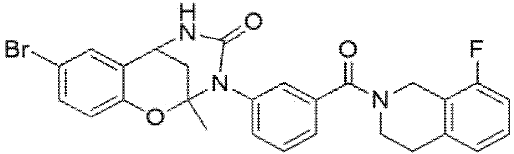

Compound 27c in light brown crystal (0.13 g, 48.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.95 (s, 1H), 7.65 (s, 1H), 7.49 (t, J = 7.8 Hz, 1H), 7.42 (s, 3H), 7.29 - 7.23 (m, 1H), 7.17 (s, 2H), 7.06 (d, J = 8.0 Hz, 2H), 4.79 - 4.65 (m, 2H), 4.38 (s, 1H), 3.58 (s, 2H), 2.89 (s, 2H), 2.73 (s, 2H), 2.69 (s, 1H), 2.56 (s, 1H), 2.34 (s, 1H); Purity ≥99.9% (RP-HPLC, Rₜ = 16.5 min).

### 94) 8-Bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27d)

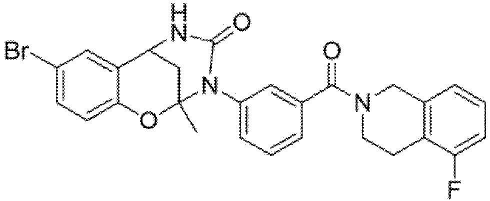

Compound 27d in light brown crystal (0.14 g, 56.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.95 (s, 1H), 7.65 (d, J = 3.4 Hz, 1H), 7.48 (t, J = 7.8 Hz, 1H), 7.42 (s, 3H), 7.18 (d, J = 7.8 Hz, 2H), 7.06 (d, J = 10.3 Hz, 2H), 6.89 (s, 1H), 4.73 (s, 1H), 4.38 (s, 1H), 3.57 (s, 1H), 2.73 (s, 2H), 2.55 (s, 1H), 2.34 (d, J = 12.5 Hz, 1H), 1.41 (s, 3H), 1.28 - 1.22 (m, 2H).

### 95) 8-Bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27e)

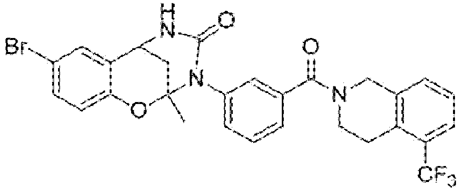

Compound 27e in off-white powder (0.23 g, 57.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 8.65 (s, 1H), 8.55 (d, J = 8.0 Hz, 1H), 8.36 (s, 1H), 8.30 (d, J = 4.0 Hz, 1H), 8.26 (d, J = 8.0 Hz, 1H), 8.01 (d, J = 8.0 Hz, 1H), 7.88 (d, J = 8.0 Hz, 1H), 7.73 (d, J = 4.0 Hz, 1H), 7.64 (t, J = 8.0 Hz, 1H), 7.46 (d, J = 8.0 Hz, 1H), 7.30 - 7.27 (m, 1H), 7.21 - 7.15 (m, 1H), 4.33 (s, 1H), 3.44 (dd, J = 4.0, 4.0 Hz, 3H), 2.89 (s, 4H), 2.69 (s, 1H), 1.06 (t, J = 8.0 Hz, 3H); Purity ≥97.0% (RP-HPLC, Rₜ = 18.5 min).

### 96) 8-Bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27f)

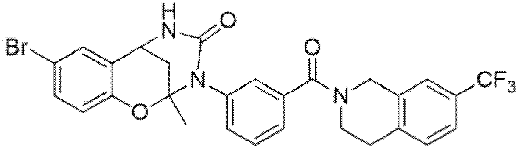

Compound 27f in off-white powder (0.70 g, 58.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.95 (s, 2H), 7.64 - 7.38 (m, 6H), 7.25 - 7.05 (m, 2H), 6.88 (s, 1H), 4.84 (s, 1H), 4.38 (s, 1H), 3.62 (s, 1H), 2.93 (s, 3H), 2.69 (s, 1H), 2.57 (d, J = 12.0 Hz, 1H), 2.35 (d, J = 12.0 Hz, 1H), 1.41 (s, 3H).

### 97) 8-Bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27g)

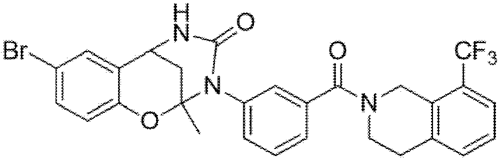

Compound 27g in off-white powder (0.63 g, 43.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.63 (d, J = 4.0 Hz, 1H), 7.53 (d, J = 8.0 Hz, 1H), 7.49 - 7.30 (m, 5H), 7.19 (d, J = 8.0 Hz, 2H), 6.90 (s, 2H), 4.94 (s, 1H), 4.38 (s, 1H), 3.61 (s, 1H), 2.97 (s, 4H), 2.57 (d, J = 12.0 Hz, 1H), 2.35 (d, J = 12.0 Hz, 1H), 1.40 (s, 3H).

### 98) 8-Bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27h)

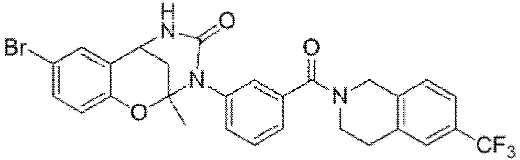

Compound 27h in light brown crystal (0.11 g, 75.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 7.95 (s, 1H), 7.66 - 7.56 (m, 3H), 7.49 (t, J = 8.0 Hz, 2H), 7.42 (s, 2H), 7.18 (s, 2H), 6.88 (s, 1H), 5.75 (s, 1H), 4.83 (s, 1H), 4.38 (s, 1H), 3.62 (s, 1H), 2.73 (s, 2H), 2.69 (s, 1H), 2.57 (d, J = 8.0 Hz, 1H), 2.35 (d, J = 12.0 Hz, 1H), 1.41 (s, 3H); Purity ≥96.1% (RP-HPLC, Rₜ = 18.7 min).

### 99) 8-Bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27i)

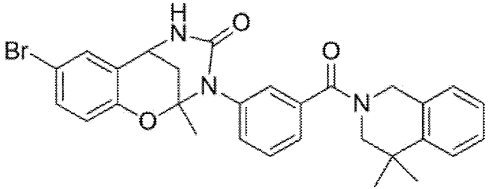

Compound 27i in brown powder (0.088 g, 67%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 8.04 (s, 1H), 7.73 (s, 1H), 7.67 (d, J = 4.0 Hz, 1H), 7.52 - 7.34 (m, 4H), 7.31 - 7.08 (m, 4H), 6.84 (s, 1H), 4.83 (s, 1H), 4.36 (s, 1H), 2.58 (d, J = 12.0 Hz, 1H), 2.37 (d, J = 12.0 Hz, 1H), 1.43 (s, 3H), 1.36 - 1.02 (m, 9H).

### 100) 8-Bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27j)

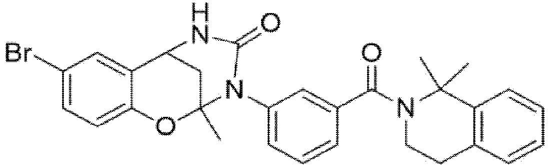

Compound 27j in light brown powder (0.06 g, 61.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 8.04 (s, 1H), 7.74 (s, 1H), 7.68 (d, J = 4.0 Hz, 1H), 7.52 - 7.37 (m, 3H), 7.29 - 7.10 (m, 4H), 6.92 (d, J = 8.0 Hz, 1H), 6.85 (s, 1H), 4.38 (d, J = 4.0 Hz, 1H), 3.41 (d, J = 5.3 Hz, 2H), 2.84 (s, 2H), 2.60 (d, J = 12.0 Hz, 1H), 2.38 (d, J = 12.0 Hz, 1H), 1.86 (s, 5H), 1.43 (s, 3H), 1.19 (s, 1H).

### 101) 8-Bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 27k)

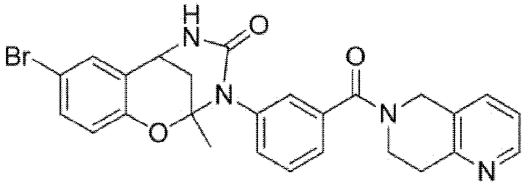

Compound 27k in light brown powder (0.15 g, 57.0%) was prepared from 3-(8-bromo-2-methyl-4-oxo-5,6-dihydro-2H-2,6-methanobenzo[g][1,3,5]oxadiazocin-3(4H)-yl)benzoic acid (Compound 24a) according to the general process of acid-amine coupling (E).

¹H NMR (400 MHz, DMSO-d₆) δ 8.39 (s, 1H), 7.66 (d, J = 8.0 Hz, 1H), 7.58 - 7.48 (m, 2H), 7.45 (s, 1H), 7.41 - 7.20 (m, 4H), 6.88 (s, 2H), 4.43 (s, 1H), 3.71 (s, 2H), 2.66 (d, J = 12.0 Hz, 1H), 2.43 (d, J = 12.0 Hz, 1H), 1.48 (s, 3H), 1.35 (d, J = 8.0 Hz, 4H).

### 102) 3-(3-(7-Fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28a)

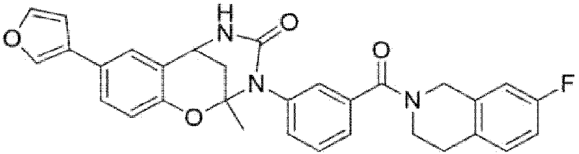

Compound 28a in off-white solid (0.04 g, 38.7%) was prepared from 8-bromo-3-(3-(7-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27a) according to the general process of Suzuki coupling F (Method A).

¹H NMR (400 MHz, DMSO-d₆) δ 8.05 (s, 1H), 7.74 (t, J = 4.0 Hz, 1H), 7.69 (d, J = 4.0 Hz, 1H), 7.52 - 7.36 (m, 4H), 7.21 (m, 4H), 7.05 (d, J = 8.0 Hz, 1H), 6.93 (s, 1H), 6.85 (s, 1H), 4.76 (s, 2H), 4.37 (s, 1), 3.57 (s, 2H), 2.83 (s, 2H), 2.58 (d, J = 12.0 Hz, 1H), 2.38 (d, J = 12.0 Hz, 1H), 1.42 (s, 3H); Purity ≥99.0% (RP-HPLC, Rₜ = 16.6 min).

### 103) 3-(3-(6-Fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b)

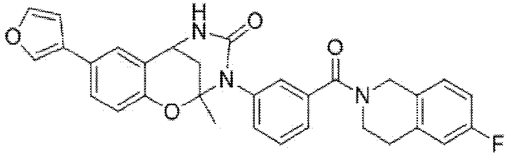

Compound 28b in off-white solid (0.05 g, 47.0%) was prepared from 8-bromo-3-(3-(6-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27b) according to the general process of Suzuki coupling F (Method A).

¹H NMR (400 MHz, DMSO-d₆) δ 8.05 (s, 1H), 7.73 (s, 1H), 7.68 (d, J = 4.0 Hz, 1H), 7.52 - 7.37 (m, 4H), 7.20 (s, 2H), 7.05 (d, J = 8.0 Hz, 2H), 6.85 (s, 1H), 4.73 (s, 3H), 4.38 (s, 1H), 3.57 (s, 2H), 2.87 (s, 3H), 2.58 (d, J = 8 Hz, 1H), 2.38 (d, J = 12.0 Hz, 1H), 1.42 (s, 3H); Purity ≥99.9% (RP-HPLC, Rₜ = 16.9 min).

### 104) 3-(3-(8-Fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c)

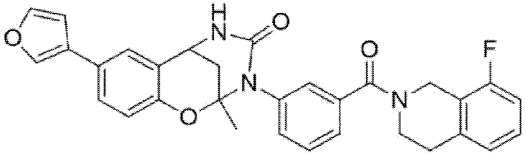

Compound 28c in yellow crystal (0.035 g, 36.0%) was prepared from 8-bromo-3-(3-(8-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27c) according to the general process of Suzuki coupling F (Method A).

¹H NMR (400 MHz, DMSO-d₆) δ 8.05 (s, 1H), 7.73 (s, 1H), 7.69 (d, J = 4.0 Hz, 1H), 7.50 - 7.43 (m, 4H), 7.29 - 7.20 (m, 3H), 7.06 (d, J = 8.0 Hz, 1H), 6.85 (s, 1H), 4.75 (s, 3H), 4.38 (s, 1H), 3.58 (s, 2H), 2.90 (s, 3H), 2.58 (d, J = 12.0 Hz, 1H), 2.39 (d, J = 12.0 Hz, 1H), 1.42 (s, 3H); Purity ≥97.0% (RP-HPLC, Rₜ = 17.1 min).

### 105) 3-(3-(5-Fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d)

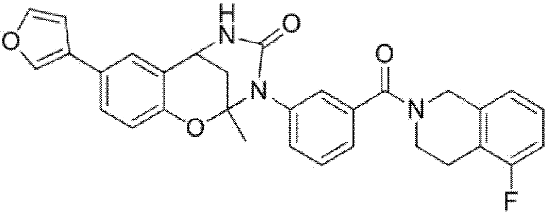

Compound 28d in yellow crystal (25.0 mg, 17.0%) was prepared from 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27d) according to the general process of Suzuki coupling F (Method A).

¹H NMR (400 MHz, DMSO-d₆) δ 8.05 (s, 1H), 7.72 (s, 1H), 7.70 (d, J = 4.0 Hz, 1H), 7.58 - 7.42 (m, 4H), 7.25 (m, 3H), 7.09 (d, J = 8.0 Hz, 1H), 6.82 (s, 1H), 4.76 (s, 3H), 4.40 (s, 1H), 3.58 (s, 2H), 2.91 (s, 3H), 2.56 (d, J = 12.0 Hz, 1H), 2.37 (d, J = 12.0 Hz, 1H), 1.41 (s, 3H); Purity ≥96.5% (RP-HPLC, Rₜ = 16.8 min).

### 106) 8-(Furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28e)

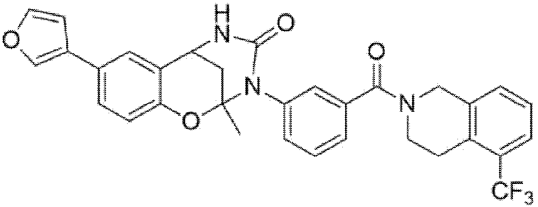

Compound 28e in off-white solid (0.03 g, 32.0%) was prepared from 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27e) according to the general process of Suzuki coupling F (Method A).

¹H NMR (400 MHz, DMSO-d₆) δ 8.04 (s, 1H), 7.73 (s, 1H), 7.67 (d, J = 4.0 Hz, 1H), 7.61 (d, J = 8.0 Hz, 2H), 7.47 (m, 6H), 7.24 - 7.18 (m, 2H), 6.84 (s, 1H), 4.82 (s, 2H), 4.38 (s, 2H), 2.99 (s, 3H), 2.58 (d, J = 4.0 Hz, 1H), 2.39 (d, J = 4.0 Hz, 1H), 1.43 (s, 3H); Purity ≥99.6% (RP-HPLC, Rₜ = 18.7 min).

### 107) 8-(Furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28f)

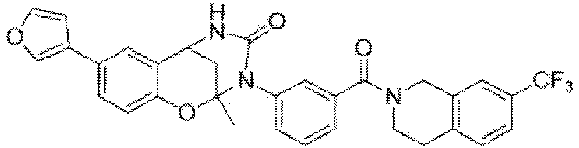

Compound 28f in off-white solid (0.03 g, 32.0%) was prepared from 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27f) according to the general process of Suzuki coupling F (Method A).

¹H NMR (400 MHz, DMSO-d₆) δ 8.05 (s, 1H), 7.73 (s, 1H), 7.66 (d, J = 4.0 Hz, 1H), 7.57 - 7.30 (m, 6H), 7.22 (d, J = 8.0 Hz, 2H), 6.93 (s, 1H), 6.85 (s, 1H), 5.75 (s, 1H), 4.95 (s, 1H), 4.38 (s, 2H), 3.63 (s, 1H), 2.98 (s, 3H), 2.58 (d, J = 8.0 Hz, 1H), 2.39 (d, J = 12.0 Hz, 1H), 1.42 (s, 3H). Purity ≥98.7% (RP-HPLC, Rₜ = 18.7 min).

### 108) 8-(Furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28g)

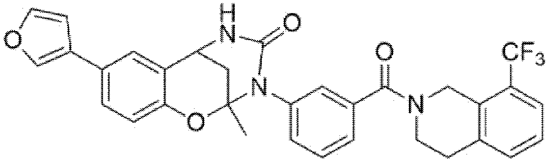

Compound 28g in off-white solid (0.03 g, 32.0%) was prepared from 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27g) according to the general process of Suzuki coupling F (Method A).

¹H NMR (400 MHz, DMSO-d₆) δ 8.04 (s, 1H), 7.72 (s, 1H), 7.67 (d, J = 4.0 Hz, 1H), 7.58 - 7.29 (m, 6H), 7.21 (d, J = 8.0 Hz, 2H), 6.93 (s, 1H), 6.85 (s, 1H), 5.76 (s, 1H), 4.94 (s, 1H), 4.37 (s, 2H), 3.62 (s, 1H), 2.99 (s, 3H), 2.57 (d, J = 8.0 Hz, 1H), 2.40 (d, J = 12 .0 Hz, 1H), 1.41 (s, 3H); Purity ≥99.8% (RP-HPLC, Rₜ = 18.6 min).

### 109) 8-(Furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 28h)

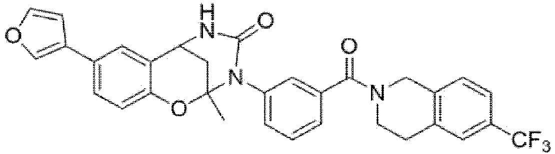

Compound 28h in off-white solid (0.036 g, 66.0%) was prepared from 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one (Compound 27h) according to the general process of Suzuki coupling F (Method A).

¹H NMR (400 MHz, DMSO-d₆) δ 8.04 (s, 1H), 7.73 (s, 1H), 7.67 (d, J = 4.0 Hz, 1H), 7.58 (s, 1H), 7.54 - 7.44 (m, 4H), 7.42 (d, J = 8.0 Hz, 2H), 7.24 - 7.16 (m, 2H), 6.92 (s, 1H), 6.84 (s, 1H), 5.75 (s, 1H), 4.82 (s, 1H), 4.38 (s, 1H), 3.62 (s, 2H), 2.96 (s, 2H), 2.58 (d, J = 12.0 Hz, 1H), 2.38 (d, J = 12.0 Hz, 1H), 1.43 (s, 3H); purity ≥99.9% (RP-HPLC, Rₜ = 18.8 min).

### 110) 3-(3-(4,4-Dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28i)

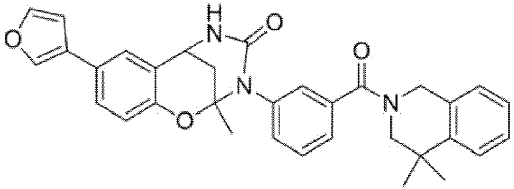

Compound 28i in off-white solid (0.04 g, 41.0%) was prepared from 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27i) according to the general process of Suzuki coupling F (Method A).

¹H NMR (400 MHz, DMSO-d₆) δ 8.04 (s, 1H), 7.73 (s, 1H), 7.68 (d, J = 4.0 Hz, 1H), 7.51 - 7.35 (m, 5H), 7.30 - 7.07 (m, 6H), 6.84 (s, 1H), 4.83 (s, 1H), 4.36 (s, 1H), 2.57 (d, J = 12.0 Hz, 1H), 2.37 (d, J = 12.0 Hz, 1H), 1.42 (s, 3H), 1.35 - 1.01 (m, 9H); HRMS (ESI): [M+H]⁺ C₃₃H₃₁N₃O₄ calcd 534.2393, found 534.2383; Purity ≥96.2% (RP-HPLC, Rₜ = 18.3 min).

### 111) 3-(3-(1,1-Dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28j)

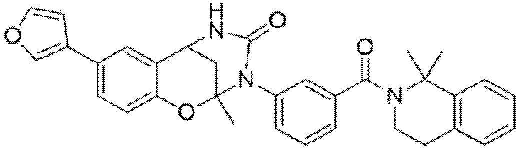

Compound 28j in yellow solid (0.03 g, 31.0%) was prepared from 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one (Compound 27j) according to the general process of Suzuki coupling F (Method A).

¹H NMR (400 MHz, DMSO-d₆) δ 8.04 (s, 1H), 7.73 (s, 1H), 7.68 (d, J = 4.0 Hz, 1H), 7.52 - 7.37 (m, 5H), 7.29 - 7.10 (m, 5H), 6.92 (d, J = 8.0 Hz, 1H), 6.85 (s, 1H), 4.38 (d, J = 4.0 Hz, 1H), 3.41 (d, J = 5.3 Hz, 2H), 2.84 (s, 2H), 2.60 (d, J = 12.0 Hz, 1H), 2.38 (d, J = 12.0 Hz, 1H), 1.85 (s, 5H), 1.43 (s, 3H), 1.18 (s, 1H); HRMS (ESI): [M+H]⁺ C₃₃H₃₁N₃O₄ calcd 534.2393, found 534.2394; Purity ≥94.6% (RP-HPLC, Rₜ = 19.5 min).

### 112) 8-(Furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 28k)

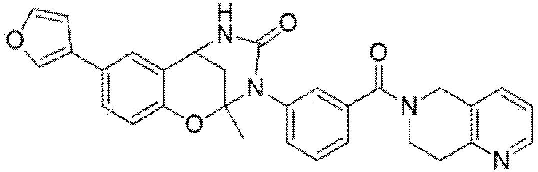

Compound 28k in off-white solid (0.015 g, 16.0%) was prepared from 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one (Compound 27k) according to the general process of Suzuki coupling F (Method A).

¹H NMR (400 MHz, DMSO-d₆) δ 8.40 (s, 1H), 8.05 (s, 1H), 7.75 - 7.66 (m, 2H), 7.56 - 7.37 (m, 5H), 7.22 (d, J = 8.0 Hz, 3H), 6.86 (s, 2H), 4.79 (s, 1H), 4.37 (s, 1H), 3.69 (s, 1H), 2.97 (s, 3H), 2.58 (d, J = 12.0 Hz, 1H), 2.38 (d, J = 12.0 Hz, 1H), 1.42 (s, 2H), 1.07 (s, 2H); Purity ≥96.6% (RP-HPLC, Rₜ = 5.43 min).

### <Experimental Example 1> NOX2 inhibitory activity of synthetic derivatives

### 1) Evaluation of NOX2 inhibitory ability of synthetic derivatives

The NOX2 inhibitory ability was tested as shown in the following for the synthetic derivatives prepared above, the results of which are shown in Table 1 below.

NOX activity was evaluated by measuring the reactive oxygen species (ROS) produced by NOX. The probes that were used to measure ROS were water soluble tetrazolium-1 (WST-1, superoxide-specific colorimetric probe) and Amplex Red/horseradish peroxidase (AR/HRP, hydrogen peroxide-specific fluorometric probe), and the concentration of the probes used was WST-1. For WST-1, the change in the absorbance at 450 nm was measured, and, for AR, the change in fluorescence signals was measured at 560 nm and 585 nm, respectively for excitation wavelength (Ex) and emission wavelength (Em). The optical signal was measured using the SpectraMax M3 microplate reader from Molecular Devices at 37°C for 60 minutes.

Used for the NOX2 activity assay was human leukemia 60 cells (HL-60), which are widely, universally used to assess NOX2 activity. 1×10⁵ cells/mL of HL-60 were treated with 1.5 µM all-trans-retinoic acid (ATRA) for 4 days to induce differentiation. Differentiated HL-60 (diff. HL-60) was centrifuged at 200 g for 5 minutes and washed twice with HBSS. Differentiation was induced to reach 3×10⁴ cells/well on a 96-well plate (diff). HL-60 was seeded. The test material was treated to cells and left in a 37°C incubator for 60 minutes. As a control group, the same concentration of DMSO, which is the vehicle of the test material, was used. 200 ng/mL phorbol 12-myristate 13-acetate (PMA) was used as the NOX2 activator, and the production amount of ROS induced by PMA was measured for 60 minutes. 10 µM diphenyleneiodonium (DPI), which is well known to inhibit NOX, was used as a positive control group to evaluate whether the NOX2 activity tester properly operates.

**TABLE 1**

| Compound No. | Inhibitory ability* |
|---|---|
| 8a | ++ |
| 7 | + |
| 15a | + |
| 15b | + |
| 16 | + |
| 10a | + |
| 10c | ++ |
| 10b | + |
| 10d | + |
| 8c | + |
| 8b | + |
| 8d | ++ |
| 8e | + |
| 8f | + |
| 8g | + |
| 8h | + |
| 9 | + |
| 25f | + |
| 19 | + |
| 8i | + |
| 25a | + |
| 26f | ++ |
| **26a** | +++ |
| **26b** | +++ |
| 25h | ++ |
| 26i | ++ |
| 26j | ++ |
| **25b** | + |
| 26h | ++ |
| 26g | ++ |
| 27a | ++ |
| 28a | ++ |
| 25i | ++ |
| 25c | ++ |
| **26c** | + |
| **26d** | + |
| **26e** | + |
| 25k | + |
| 25m | ++ |
| 25l | + |
| 25d | ++ |
| **25e** | + |
| 25g | + |
| 25j | ++ |
| 27b | ++ |
| 27c | + |
| **28b** | ++ |
| **28c** | + |
| **28d** | + |
| 27e | ++ |
| 28e | ++ |
| 28f | + |
| 28g | + |
| 27h | ++ |
| 28h | + |
| 28i | ++ |
| **28j** | + |
| 28k | + |
| [Standard] | |
| >10 uM : + | |
| 1 < inhibitory ability < 10 uM: ++ | |
| < 1 uM: +++ | |

Referring to Table 1, the inhibitory ability was measured using WST-1. Specifically, among synthetic derivatives other than 2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 8a) and stereoisomers, a total of 11 types were determined as materials with excellent inhibitory ability, which are 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one (Compound 26a), 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26b), 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one (Compound 25b), 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one (Compound 26c), 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26d), 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 26e), 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one (Compound 25e), 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28b), 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28c), 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28d), and 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one (Compound 28j).

In addition, experiments for concentration dependence on the NOX2 inhibitory ability of Compound 26a and Compound 26b were performed, which is shown in FIG. 1.

As shown in FIG. 1, both Compound 26a and Compound 26b inhibited NOX2 activity in a concentration-dependent manner and showed up to 70-80% inhibitory ability. For Compound 26a, IC₅₀ was calculated to be 0.87 and 0.53 µM in the WST-1 assay and Amplex Red assay, respectively, and for Compound 26b to be 0.14 µM via both assays.

### 2) Testing of NOX1, 3, 4, 5 inhibitory abilities of effective compounds

Compound 8a and 11 types of synthetic derivatives with NOX2 inhibitory ability identified were tested for inhibitory effects on other isoforms, the results of which are shown in Table 2.

The cells for the assay were obtained as follows.

CHO-NOX1 cells and TREx-NOX3 cells were used to measure the activity of NOX1 and NOX3, respectively. For CHO-NOX1, used were cells in which NOX1 catalytic subunits and regulatory subunits (p22phox, NOXO1, NOXA1) of NOX1 were expressed in Chinese hamster ovary cells (CHO). For TREx-NOX3, cells, in which NOX3 catalytic subunits and regulatory subunits (p22phox, NOXO1, NOXA1) are expressed in the tetracycline-regulated expression (T-REx^{™}) cell line, were used.

HEK-NOX4 cells were used to measure NOX4 activity.

HEK-NOX5 cells were used to measure NOX5 activity. For HEK-NOX5, cells in which NOX5 is expressed in HEK293 cells were used.

NOX3 activity was evaluated as follows: TREx-NOX3 was seeded in a 96-well plate at 3×10⁴ cells/well. After culture overnight in a 37°C incubator, 1 µg/mL tetracycline was treated for 24 hours to induce the expression of NOX3. After washing two times with HBSS, the test material was treated to the cells and left for 60 minutes. The same concentration of DMSO, which is the vehicle of the test material, was used as the control group, and 10 µM DPI was used as the positive control group. 200 ng/mL of phorbol 12-myristate 13-acetate (PMA) was used as the NOX2 activator, and the ROS produced by cells was measured for 60 minutes.

NOX4 activity was evaluated as follows: HEK-NOX4 and the control group HEK were seeded in a 96-well plate at 3×10⁴ cells/well. Culture was carried out overnight in a 37°C incubator and then washed twice with HBSS. The test material was pre-treated for 60 minutes. The same concentration of DMSO, which is the vehicle of the test material, was used as the control group, and 10 µM DPI was used as the positive control group. ROS derived from HEK-NOX4 or HEK was measured for 60 minutes.

NOX5 activities were evaluated as follows: HEK-NOX5 was seeded in a 96-well plate at 3×10⁴ cells/well. Culture was carried out overnight in a 37°C incubator, followed by washing twice with HBSS. The test material was pre-treated for 60 minutes. The same concentration of DMSO, which is the vehicle of the test material, was used as the control group, and 10 µM DPI was used as the positive control group. NOX5 was activated with 5 µM ionomycin, and the ROS produced was measured for 60 minutes.

**TABLE 2**

| Compound No. | IC₅₀ | | | |
|---|---|---|---|---|
| | NOX1* | NOX3* | NOX4^{#} | NOX5* |
| 8a | 30< | 30< | 30< | 30< |
| 26a | 30< | 30< | 30< | 10< <30 |
| 26b | 30< | 30< | 30< | 10< <30 |
| 25b | 30< | 30< | 30< | 30< |
| 26c | 30< | 30< | ^{≈} 30 | 30< |
| 26d | 30< | 30< | 30< | 30< |
| 26e | 30< | 30< | 30< | 30< |
| 25e | 30< | 30< | 30< | 30< |
| 28b | 30< | 30< | ^{≈} 30 | 30< |
| 28c | 30< | 30< | ^{≈} 30 | 30< |
| 28d | 30< | 30< | ^{≈} 30 | 30< |
| 28j | 30< | 30< | 30< | 30< |
| * Activity was measured using WST-1 | | | | |
| # Activity was measured using Amplex Red | | | | |

As shown in Table 2 above, most of the effective substances did not show inhibitory ability against other isoforms other than NOX2 up to 30 µM, and the effective concentration was more than 50 times higher than that of NOX2 even if weak inhibitory effect was derived.

### 3) Non-specific free radical scavenging ability of effective compounds

The scavenging activity that the effective compound in 10 or 30 µM scavenges the superoxide produced by xanthine/xanthine oxidase was measured by WST-1, the results of which are shown in Table 2. As a result, both Compound 8a and 11 types of effective compounds did not exhibit significant peroxide scavenging ability up to 30 µM.

While a specific part of the present disclosure has been described in detail above, it is clear for those skilled in the art that this specific description is merely preferred example embodiments, and the scope of the present disclosure is not limited thereby. Accordingly, the substantial scope of the present disclosure is defined by the appended claims and their equivalents.

## Claims

1. A compound selected from a compound represented by the following Chemical Formula 1, an isomer thereof, or a pharmaceutically acceptable salt thereof: 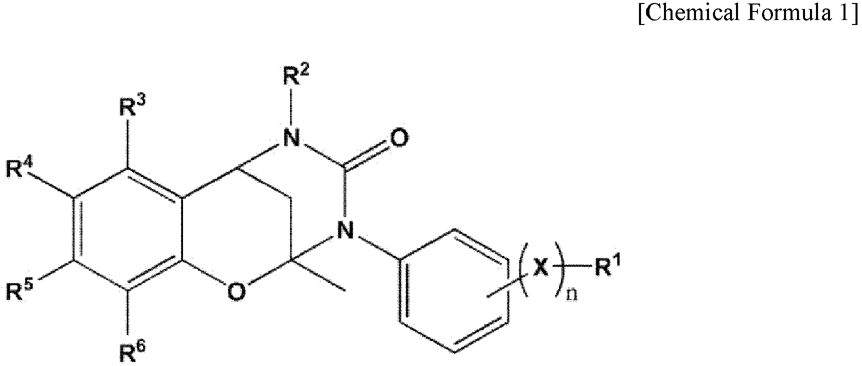 wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ are the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound,
provided that the case where, in the compound, X is carbonyl, n is 1, R¹ is tetrahydroisoquinoline, and R² to R⁶ are all hydrogen is excluded.

2. The compound of claim 1, wherein the 5-ring or 6-ring heterocyclic compound is selected from the group consisting of thiophenyl, furanyl, morpholino, pyridinyl, pyranyl, oxazinyl, thiazinyl, pyrimidinyl, and piperazinyl.

3. The compound of claim 1 or claim 2, wherein the compound is a compound represented by the following Chemical Formula 1-1: 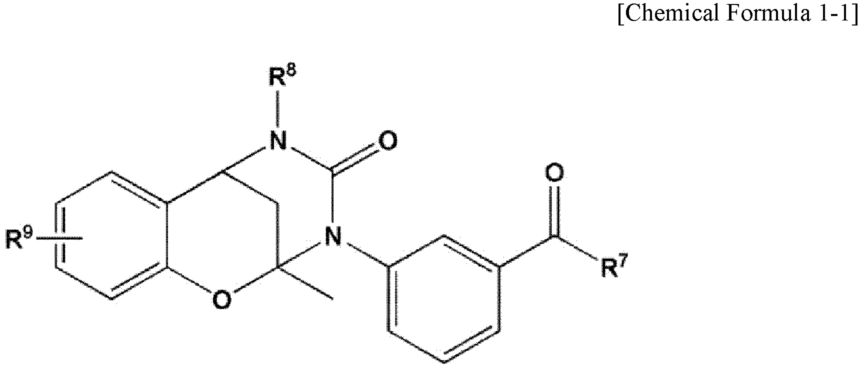 wherein, in the Chemical Formula 1-1,
R⁷ is tetrahydroisoquinoline which is substituted or unsubstituted with one or more substituents selected from halo, di(C1~C4)alkyl, difluoromethyl, or trifluoromethyl,
R⁸ is hydrogen or (C1~C2)alkyl, and
R⁹ is the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

4. The compound of claim 1 or claim 2, wherein the compound is selected from the group consisting of 3-(2-methyl-4-oxo-5,6-dihydro-2H-2,6-methanobenzo[g][1,3,5]oxadiazocin-3(4H)-yl)benzoic acid); 3-(3-(6,7-dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 3-(2-methyl-4-oxo-5,6-dihydro-2H-2,6-methanobenzo[g][1,3,5]oxadiazocin-3(4H)-yl)-N-(4-(trifluoromethyl)benzyl)benzamide; 3-(2-methyl-4-oxo-5,6-dihydro-2H-2,6-methanobenzo[g][1,3,5]oxadiazocin-3(4H)-yl)-N-(4-(trifluoromethyl)phenethyl)benzamide; N-(2,3-dihydro-1H-inden-2-yl)-3-(2-methyl-4-oxo-5,6-dihydro-2H-2,6-methanobenzo[g][1,3,5]oxadiazocin-3(4H)-yl)benzamide; 3-(2-methyl-4-oxo-5,6-dihydro-2H-2,6-methanobenzo[g][1,3,5]oxadiazocin-3(4H)-yl)-N-(pyridin-3-ylmethyl)benzamide; N-(furan-2-ylmethyl)-3-(2-methyl-4-oxo-5,6-dihydro-2H-2,6-methanobenzo[g][1,3,5]oxadiazocin-3(4H)-yl)benzamide; N-cyclopropyl-3-(2-methyl-4-oxo-5,6-dihydro-2H-2,6-methanobenzo[g][1,3,5]oxadiazocin-3(4H)-yl)benzamide; N-benzyl-N-methyl-3-(2-methyl-4-oxo-5,6-dihydro-2H-2,6-methanobenzo[g][1,3,5]oxadiazocin-3(4H)-yl)benzamide; 2,5-dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; N-(4-bromobenzyl)-3-((2R,6R)-2-methyl-4-oxo-5,6-dihydro-2H-2,6-methanobenzo[g][1,3,5]oxadiazocin-3(4H)-yl)benzamide; N-(3,5-dibromobenzyl)-3-((2S,6S)-2-methyl-4-oxo-5,6-dihydro-2H-2,6-methanobenzo[g][1,3,5]oxadiazocin-3(4H)-yl)benzamide); 3'-(2-methyl-4-oxo-5,6-dihydro-2H-2,6-methanobenzo[g][1,3,5]oxadiazocin-3(4H)-yl)-[1,1'-biphenyl]-3-carbonitrile; 3-(3-(furan-2-yl)phenyl)-2-methyl-5,6-dihydro-2H-2,6-methanobenzo[g][1,3,5]oxadiazocin-4(3H)-one; 3-(3-(furan-2-yl)phenyl)-2,5-dimethyl-5,6-dihydro-2H-2,6-methanobenzo[g][1,3,5]oxadiazocin-4(3H)-one; 2-methyl-3-(3-(((3-(trifluoromethyl)phenyl)amino)methyl)phenyl)-5,6-dihydro-2H-2,6-methanobenzo[g][1,3,5]oxadiazocin-4(3H)-one; 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one; 8-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-methoxy-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 10-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one; 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-methoxy-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 7-fluoro-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one; 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one; 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one; 10-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one; 9-(furan-3-yl)-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 8-bromo-3-(3-(7-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(6-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(8-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one; 3-(3-(7-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 8-(furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 3-(3-(4,4-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; and 8-(furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one.

5. The compound of claim 1 or claim 2, wherein the compound selectively inhibits NADPH oxidase 2 (NOX2).

6. A pharmaceutical composition for treating or preventing an NADPH oxidase 2 (NOX2)-related disease, comprising a compound selected from a compound represented by the following Chemical Formula 1, an isomer thereof, or a pharmaceutically acceptable salt thereof: 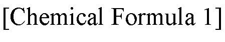 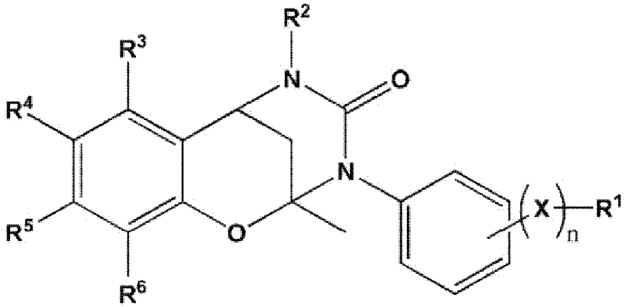 wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ are the same or different and each independently selected from hydrogen, (C1~C10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound,
provided that the case where, in the compound, X is carbonyl, n is 1, R¹ is tetrahydroisoquinoline, and R² to R⁶ are all hydrogen is excluded.

7. The pharmaceutical composition of claim 6, wherein the compound is a compound represented by the following Chemical Formula 1-1: 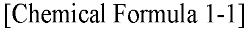 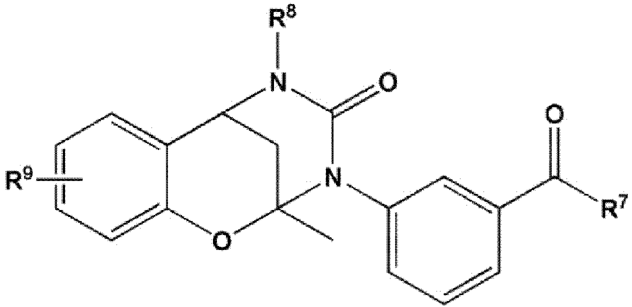 wherein, in the Chemical Formula 1-1,
R⁷ is tetrahydroisoquinoline which is substituted or unsubstituted with one or more substituents selected from halo, di(C1~C4)alkyl, difluoromethyl, or trifluoromethyl,
R⁸ is hydrogen or (C1~C2)alkyl, and
R⁹ is the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

8. The pharmaceutical composition of claim 6, wherein the compound is selected from the group consisting of 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one; 8-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one; 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one; 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3, 5]oxadiazocin-4(3H)-one; 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; and 3-(3-(5-fluoro-1,2,3,4-tetrahydroisoquinoline-2-carbonyl)phenyl)-8-(furan-3 -yl)-2-methyl-5,6-dihydro-2H-2,6-methanobenzo[g][1,3, 5]oxadiazocin-4(3H)-one.

9. The pharmaceutical composition of claim 6, wherein the NADPH oxidase 2 (NOX2)-related disease is selected from the group consisting of neurodegenerative diseases.

10. The pharmaceutical composition of claim 9, wherein the neurodegenerative disease is one selected from the group consisting of microglia-mediated neuroinflammatory disease, stroke, apoplexy, memory loss, memory impairment, dementia, forgetfulness, Parkinson's disease, Alzheimer's disease, Pick disease, Creutzfeldt-Jakob disease, Huntington's disease, and Lou Gehrig's disease.

11. A pharmaceutical composition for treating or preventing an inflammatory disease or a fibrotic disease, comprising a compound selected from a compound represented by the following Chemical Formula 1, an isomer thereof, or a pharmaceutically acceptable salt thereof: 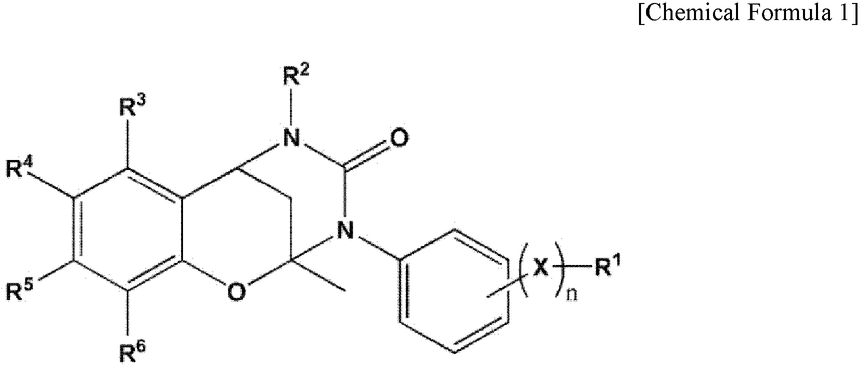 wherein, in the Chemical Formula 1,
X is selected from carbonyl or methylene, n is an integer of 0 or 1,
R¹ is selected from substituted or unsubstituted tetrahydroisoquinoline, benzylamino, phenethylamino, indenylamino, pyridinylmethylamino, furanylmethylamino, (C3~C6)cycloalkylmethylamino, benzylmethylamino, cyanophenyl, furanyl, or tetrahydronaphthylidinyl, the substitution is carried out with one or more substituents selected from (C1~C10)alkyl, (C1~C10)alkoxy, halo, di(C1~C4)alkyl, difluoromethyl, trifluoromethyl, hydroxy, cyano, or nitro,
R² is hydrogen or (C1~C4)alkyl, and
R³ to R⁶ are the same or different and each independently selected from hydrogen, (C1~Cl10)alkyl, (C1~C10)alkoxy, halo, difluoromethyl, trifluoromethyl, hydroxy, cyano, nitro, (C1~C4)alkoxyphenyl, or a 5-ring or 6-ring heteroaryl compound.

12. The pharmaceutical composition of claim 11, wherein the compound is a compound represented by the following Chemical Formula 1-1: 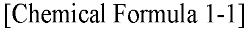 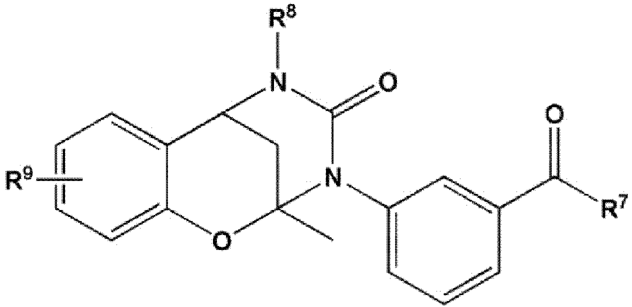 wherein, in the Chemical Formula 1-1,
R⁷ is tetrahydroisoquinoline which is substituted or unsubstituted with one or more substituents selected from halo, di(C1~C4)alkyl, difluoromethyl, or trifluoromethyl,
R⁸ is hydrogen or (C1~C2)alkyl, and
R⁹ is the same or different and each independently selected from hydrogen, (C1~C4)alkyl, (C1~C4)alkoxy, halo, difluoromethyl, trifluoromethyl, (C1~C4)alkoxyphenyl, thiophenyl, furanyl, or morpholino.

13. The pharmaceutical composition of claim 11, wherein the compound is selected from the group consisting of 3-(2-methyl-4-oxo-5,6-dihydro-2H-2,6-methanobenzo[g][1,3,5]oxadiazocin-3(4H)-yl)benzoic acid; 2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 3-(3-(6,7-dimethoxy-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 3-(2-methyl-4-oxo-5,6-dihydro-2H-2,6-methanobenzo[g][1,3,5]oxadiazocin-3(4H)-yl)-N-(4-(trifluoromethyl)benzyl)benzamide; 3-(2-methyl-4-oxo-5,6-dihydro-2H-2,6-methanobenzo[g][1,3,5]oxadiazocin-3(4H)-yl)-N-(4-(trifluoromethyl)phenethyl)benzamide; N-(2,3 -dihydro-1H-inden-2-yl)-3-(2-methyl-4-oxo-5,6-dihydro-2H-2,6-methanobenzo[g][1,3,5]oxadiazocin-3(4H)-yl)benzamide; 3-(2-methyl-4-oxo-5,6-dihydro-2H-2,6-methanobenzo[g][1,3,5]oxadiazocin-3(4H)-yl)-N-(pyridin-3-ylmethyl)benzamide; N-(furan-2-ylmethyl)-3-(2-methyl-4-oxo-5,6-dihydro-2H-2,6-methanobenzo[g][1,3,5]oxadiazocin-3(4H)-yl)benzamide; N-cyclopropyl-3-(2-methyl-4-oxo-5,6-dihydro-2H-2,6-methanobenzo[g][1,3,5]oxadiazocin-3(4H)-yl)benzamide; N-benzyl-N-methyl-3-(2-methyl-4-oxo-5,6-dihydro-2H-2,6-methanobenzo[g][1,3,5]oxadiazocin-3(4H)-yl)benzamide; 2,5-dimethyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; (2R,6R)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; N-(4-bromobenzyl)-3-((2R,6R)-2-methyl-4-oxo-5,6-dihydro-2H-2,6-methanobenzo[g][1,3,5]oxadiazocin-3(4H)-yl)benzamide; (2S,6S)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; N-(3,5-dibromobenzyl)-3-((2S,6S)-2-methyl-4-oxo-5,6-dihydro-2H-2,6-methanobenzo[g][1,3,5]oxadiazocin-3(4H)-yl)benzamide; 3'-(2-methyl-4-oxo-5,6-dihydro-2H-2,6-methanobenzo[g][1,3,5]oxadiazocin-3(4H)-yl)-[1,1'-biphenyl]-3-carbonitrile; 3-(3-(furan-2-yl)phenyl)-2-methyl-5,6-dihydro-2H-2,6-methanobenzo[g][1,3,5]oxadiazocin-4(3H)-one; 3-(3-(furan-2-yl)phenyl)-2,5-dimethyl-5,6-dihydro-2H-2,6-methanobenzo[g][1,3,5]oxadiazocin-4(3H)-one; 2-methyl-3-(3-(((3-(trifluoromethyl)phenyl)amino)methyl)phenyl)-5,6-dihydro-2H-2,6-methanobenzo[g][1,3,5]oxadiazocin-4(3H)-one; 8-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(trifluoromethyl)-5,6-dihydro-2H-2,6-methanobenzo[g][1,3,5]oxadiazocin-4(3H)-one; 8-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-methoxy-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 10-bromo-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 10-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-bromo-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 2-methyl-3-(3-(1,2,3,4-tetrahydroisoquinoline-2-carbonyl)phenyl)-9-(trifluoromethyl)-5,6-dihydro-2H-2,6-methanobenzo[g][1,3,5]oxadiazocin-4(3H)-one; 9-chloro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-fluoro-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 9-methoxy-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 7-fluoro-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 2-methyl-3-(3-(1,2,3,4-tetrahydroisoquinoline-2-carbonyl)phenyl)-8-(thiophen-3-yl)-5,6-dihydro-2H-2,6-methanobenzo[g][1,3,5]oxadiazocin-4(3H)-one; 8-(furan-3-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-8-(thiophen-2-yl)-5,6-dihydro-2H-2,6-methanobenzo[g][1,3,5]oxadiazocin-4(3H)-one; 8-(furan-2-yl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 8-(4-methoxyphenyl)-2-methyl-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-8-morpholino-3-(3-(1,2,3,4-tetrahydroisoquinoline-2-carbonyl)phenyl)-5,6-dihydro-2H-2,6-methanobenzo[g][1,3,5]oxadiazocin-4(3H)-one; 2-methyl-3-(3-(1,2,3,4-tetrahydroisoquinoline-2-carbonyl)phenyl)-10-(thiophen-3-yl)-5,6-dihydro-2H-2,6-methanobenzo[g][1,3,5]oxadiazocin-4(3H)-one; 10-(furan-3-yl)-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 2-methyl-3-(3-(1,2,3,4-tetrahydroisoquinoline-2-carbonyl)phenyl)-9-(thiophen-3-yl)-5,6-dihydro-2H-2,6-methanobenzo[g][1,3,5]oxadiazocin-4(3H)-one; 9-(furan-3-yl)-2-methyl-3-(3-(1,2,3,4-tetrahydroisoquinoline-2-carbonyl)phenyl)-5,6-dihydro-2H-2,6-methanobenzo[g][1,3, 5]oxadiazocin-4(3H)-one; 8-bromo-3-(3-(7-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(6-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(8-fluoro-1,2,3,4-tetrahydroisoquinoline-2-carbonyl)phenyl)-2-methyl-5,6-dihydro-2H-2,6-methanobenzo[g][1,3, 5]oxadiazocin-4(3H)-one; 8-bromo-3-(3-(5-fluoro-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-bromo-3-(3-(4,4-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-3-(3-(1,1-dimethyl-1,2,3,4-tetrahydroisoquinoline-2-carbonyl)phenyl)-2-methyl-5,6-dihydro-2H-2,6-methanobenzo[g][1,3,5]oxadiazocin-4(3H)-one; 8-bromo-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one; 3-(3-(7-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(6-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(8-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(5-fluoro-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 8-(furan-3-yl)-2-methyl-3-(3-(5-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(7-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(8-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 8-(furan-3-yl)-2-methyl-3-(3-(6-(trifluoromethyl)-1,2,3,4-tetrahydroisoquinoline-2-carbonyl)phenyl)-5,6-dihydro-2H-2,6-methanobenzo[g][1,3,5]oxadiazocin-4(3H)-one; 3-(3-(4,4-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; 3-(3-(1,1-dimethyl-1,2,3,4-tetrahydroisoquinoline-2-carbonyl)phenyl)-8-(furan-3-yl)-2-methyl-5,6-dihydro-2H-2,6-methanobenzo[g][1,3,5]oxadiazocin-4(3H)-one; and 8-(furan-3-yl)-2-methyl-3-(3-(5,6,7,8-tetrahydro-1,6-naphthyridine-6-carbonyl)phenyl)-5,6-dihydro-2H-2,6-methanobenzo[g][1,3,5]oxadiazocin-4(3H)-one.

14. The pharmaceutical composition of claim 11, wherein the inflammatory disease is selected from the group consisting of osteoarthritis, rheumatoid arthritis, dermatitis, allergies, atopy, asthma, psoriasis, conjunctivitis, rhinitis, otitis media, pharyngolaryngitis, tonsillitis, pneumonia, gastric ulcer, gastritis, Crohn's disease, inflammatory bowel disease, lupus, hepatitis, cystitis, interstitial cystitis, nephritis, Sjogren's syndrome, multiple sclerosis, Hashimoto thyroiditis, polymyositis, scleroderma, Addison disease, vitiligo, pernicious anemia, cystic fibrosis, graft-versus-host disease, transplant rejection disease, autoimmune diabetes, diabetic retinopathy, ischemia-reperfusion injury, post-angioplasty restenosis, chronic obstructive pulmonary disease (COPD), Graves' disease, and acute and chronic inflammatory diseases.

15. The pharmaceutical composition of claim 11, wherein the fibrotic disease is selected from the group consisting of liver fibrosis, pulmonary fibrosis, renal fibrosis, cardiac fibrosis, dermatofibrosis, and corneal fibrosis.
